# EUROPEAN PATENT APPLICATION

(11) **EP 4 431 510 A1**
(43) Date of publication of application: **18.09.2024**
(21) Application number: 22891994.0
(22) Date of filing: 09.11.2022
(51) Int. Cl.: C07D 487/04, A61K 31/4985, A61P 37/02, A61P 29/00

(54) **PYRAZOLO FUSED RING COMPOUND AND USE THEREOF**

(30) Priority: 12.11.2021 CN 202111342790; 01.11.2022 CN 202211358879
(71) Applicant: Soter Biopharma Pte. Ltd., Singapore 179803 (SG)
(72) Inventor: WEI, Changqing, Shanghai 200131 (CN); GUO, Qiang, Shanghai 200131 (CN); WANG, Cong, Shanghai 200131 (CN); YUE, Bao, Shanghai 200131 (CN); QIAN, Wenyuan, Shanghai 200131 (CN); LI, Jian, Shanghai 200131 (CN); CHEN, Shuhui, Shanghai 200131 (CN)
(74) Representative: Maiwald GmbH
(86) International application number: PCT/CN2022/130770
(87) International publication number: WO 2023/083200

(57) **Abstract**

A pyrazolo fused ring compound and the use thereof. Specifically disclosed are a compound represented by formula (IV) and a pharmaceutically acceptable salt thereof.

## Description

### CROSS-REFERENCE OF RELECVANT APPLICATIONS

The present application claims priorities of Chinese Patent Application No. CN202111342790.8 filed on November 12, 2021, and Chinese Patent Application No. CN202211358879.8 filed on November 01, 2022, the contents of which are incorporated herein by reference in their entireties.

### TECHNICAL FIELD

The present disclosure relates to a series of pyrazolo fused ring compounds and use thereof, specifically relates to a compound represented by formula (IV) and a pharmaceutically acceptable salt thereof.

### BACKGROUND

Janus kinases (JAKs) are cytoplasmic tyrosine kinases that transmit cytokine signals from membrane receptors to STAT transcription factors. The JAK family consists of four members, JAK1, JAK2, JAK3 and TYK2. The JAK-STAT pathway transduces extracellular signals from a variety of cytokines, growth factors, and hormones to the nucleus and is responsible for the expression of thousands of protein-coding genes. The JAK-STAT pathway involves several steps in converting extracellular signals into transcriptional responses: 1) when the cytokine receptors on the cell surface bind to their respective cytokine ligands, the configuration changes, causing dimerization of receptor molecules, which makes JAK kinases coupled with receptors approach each other and activate through interactive tyrosine phosphorylation. 2) after activation of JAK, the tyrosine residues on the catalytic receptor are phosphorylated, and then these phosphorylated tyrosine sites form a "docking site" with the surrounding amino acid sequences, and at the same time, STAT protein containing SH2 domain is recruited to this "docking site". 3) finally, the kinase JAK catalyzes the phosphorylation modification of the STAT protein bound to the receptor. The activated STAT protein leaves the receptor and forms a dimer which is then transferred to the nucleus to regulate the transcription of specific genes. JAK-STAT intracellular signaling serves interferons, most interleukins, and a variety of cytokines and endocrine factors, such as EPO, TPO, GH, OSM, LIF, CNTF, GM-CSF and PRL (Vainchenker W.E T al. (2008).

Different JAK family members selectively bind to different cytokine receptors, conferring signaling specificity and thereby exerting different physiological effects. This selective mode of action allows JAK inhibitors to be applied to treatment of diseases with relative specificity. TYK2 is a member of the JAK kinase family and is a key mediator of signaling of inflammatory cytokines such as IL-12, IL-23, and type I interferon. These cytokines are related to the pathogenesis of various inflammatory and autoimmune diseases such as psoriasis, inflammatory bowel disease (IBD), and systemic lupus erythematosus (SLE). Therefore, the development of inhibitors with high inhibitory activity against TYK2 and certain inhibitory activity against JAK2 and JAK1 can inhibit the formation of intracellular heterodimers of TYK2 with JAK2 and JAK1 to the greatest extent, blocking the signal transduction pathway, thereby blocking signal transduction of inflammatory cytokines IL-12, IL-23 and type I interferon to treat specific diseases.

### CONTENT OF THE PRESENT INVENTION

The present disclosure provides a compound represented by formula (IV) or a pharmaceutically acceptable salt thereof, 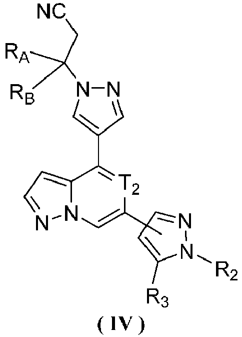 wherein
R_{A} is selected from H, and R_{B} is selected from 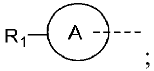
alternatively, R_{A} and R_{B} and the carbon atom to which they are both attached form 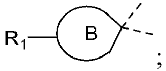
ring A is selected from C₅₋₈ cycloalkyl;
ring B is selected from C₅₋₁₂ cycloalkyl or 5- to 12-membered heterocycloalkyl;
R₁ is selected from H, C₁₋₃ alkyl-SO₂-, CN and -CH₂CN, and the C₁₋₃ alkyl is optionally substituted with 1, 2 or 3 halogens;
R₂ is selected from H and C₁₋₃ alkyl, and the C₁₋₃ alkyl is optionally substituted with 1, 2 or 3 halogens;
R₃ is selected from H, NH₂, halogen, OH, CN and C₁₋₃ alkyl, and the C₁₋₃ alkyl is optionally substituted with 1, 2 or 3 halogens;
T₂ is selected from CH and N.

In some embodiments of the present disclosure, ring A is selected from

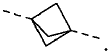

In some embodiments of the present disclosure, ring B is selected from 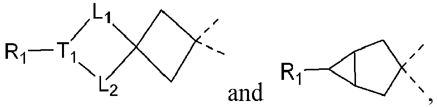
wherein, L₁ is selected from -(CH₂)ₘ-, and the -(CH₂)ₘ- is optionally substituted with 1, 2 or 3 halogens;
L₂ is selected from -(CH₂)ₙ-, and the -(CH₂)ₙ- is optionally substituted with 1, 2 or 3 halogens;
m and n are each independently selected from 1, 2 and 3;
T₁ is selected from CH and N;
R₁ is as defined in formula (IV).

In some embodiments of the present disclosure, the compound represented by formula (IV) or the pharmaceutically acceptable salt thereof is selected from a compound represented by formulas (IV-1) and (IV-2), 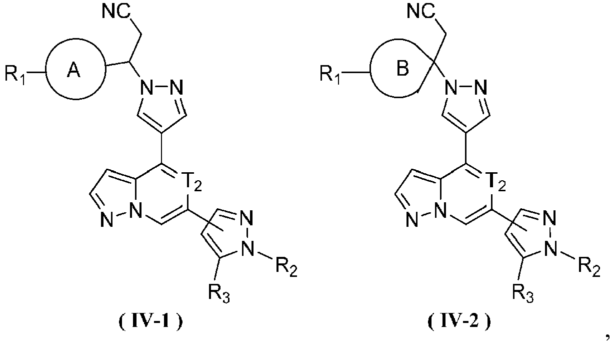 wherein, R₁, R₂, R₃, T₂, ring A and ring B are as defined in formula (IV).

The present disclosure further provides a compound represented by formula (I) or a pharmaceutically acceptable salt thereof, 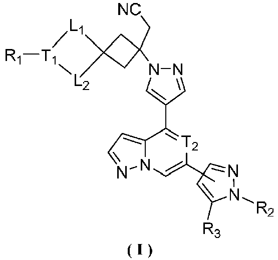 wherein
R₁ is selected from H, C₁₋₃ alkyl-SO₂-, CN and -CH₂CN, and the C₁₋₃ alkyl is optionally substituted with 1, 2 or 3 halogens;
R₂ is selected from H and C₁₋₃ alkyl, and the C₁₋₃ alkyl is optionally substituted with 1, 2 or 3 halogens;
R₃ is selected from H, NH₂, halogen, OH, CN and C₁₋₃ alkyl, and the C₁₋₃ alkyl is optionally substituted with 1, 2 or 3 halogens;
L₁ is selected from -(CH₂)ₘ-, and the -(CH₂)ₘ- is optionally substituted with 1, 2 or 3 halogens;
L₂ is selected from -(CH₂)ₙ-, and the -(CH₂)ₙ- is optionally substituted with 1, 2 or 3 halogens;
m and n are each independently selected from 1, 2 and 3;
T₁ is selected from CH and N;
T₂ is selected from CH and N.

In some embodiments of the present disclosure, the above-mentioned R₁ is selected from H, C₁₋₃ alkyl-SO₂-, CN and -CH₂CN, and the C₁₋₃ alkyl is optionally substituted with 1, 2 or 3 F, and other variables are as defined in the present disclosure.

In some embodiments of the present disclosure, the above-mentioned R₁ is selected from H, CF₃SO₂-, CN and -CH₂CN, and other variables are as defined in the present disclosure.

In some embodiments of the present disclosure, the above-mentioned R₂ is selected from H and CH₃, and other variables are as defined in the present disclosure.

In some embodiments of the present disclosure, the above-mentioned R₃ is selected from H and NH₂, and other variables are as defined in the present disclosure.

In some embodiments of the present disclosure, the above-mentioned L₁ is selected from -CH₂- and -CH₂CH₂-, and the -CH₂- and - CH₂CH₂- are optionally substituted with 1, 2 or 3 F, and other variables are as defined in the present disclosure.

In some embodiments of the present disclosure, the above-mentioned Li is selected from -CH₂- and -CH₂CH₂-, and other variables are as defined in the present disclosure.

In some embodiments of the present disclosure, the above-mentioned L₂ is selected from -CH₂- and -CH₂CH₂-, and the -CH₂- and -CH₂CH₂- are optionally substituted with 1, 2 or 3 F, and other variables are as defined in the present disclosure.

In some embodiments of the present disclosure, the above-mentioned L₂ is selected from -CH₂- and -CH₂CH₂-, and other variables are as defined in the present disclosure.

In some embodiments of the present disclosure, the above-mentioned moiety 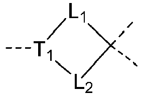 is selected from acridinyl, cyclobutyl, tetrahydropyrrolyl, piperidinyl and cyclohexyl, and other variables are as defined in the present disclosure.

In some embodiments of the present disclosure, the above-mentioned moiety 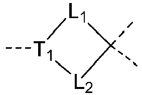 is selected from 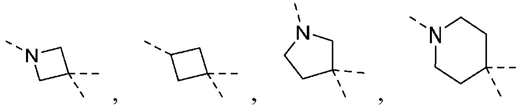 and 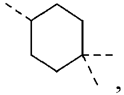 other variables are as defined in the present disclosure.

In some embodiments of the present disclosure, the above-mentioned compound or the pharmaceutically acceptable salt thereof is selected from: 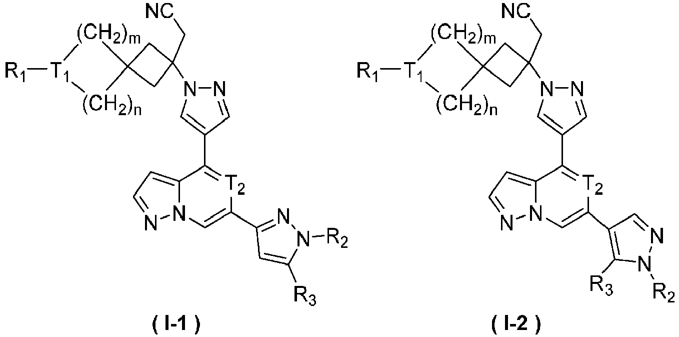 wherein R₁, R₂, R₃, T₁, T₂, m and n are as defined in the present disclosure.

The present disclosure further provides a compound represented by formula (I) or a pharmaceutically acceptable salt thereof, 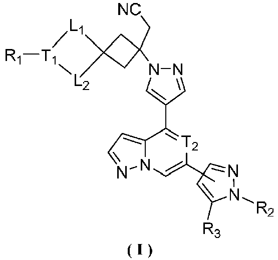 wherein
R₁ is selected from H, C₁₋₃ alkyl-SO₂-, CN and -CH₂CN, and the C₁₋₃ alkyl is optionally substituted with 1, 2 or 3 halogens;
R₂ is selected from H and CH₃;
R₃ is selected from H and NH₂;
L₁ is selected from -(CH₂)ₘ-, and the -(CH₂)ₘ- is optionally substituted with 1, 2 or 3 halogens;
L₂ is selected from -(CH₂)ₙ-, and the -(CH₂)ₙ- is optionally substituted with 1, 2 or 3 halogens;
m and n are each independently selected from 1, 2 and 3;
T₁ is selected from CH and N;
T₂ is selected from CH and N.

There are still some embodiments of the present disclosure derived from any combination of the above-mentioned variables.

The present disclosure further provides the following compounds or pharmaceutically acceptable salts thereof, which are selected from:

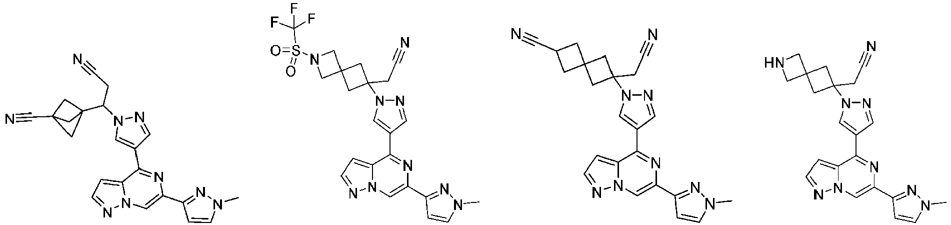

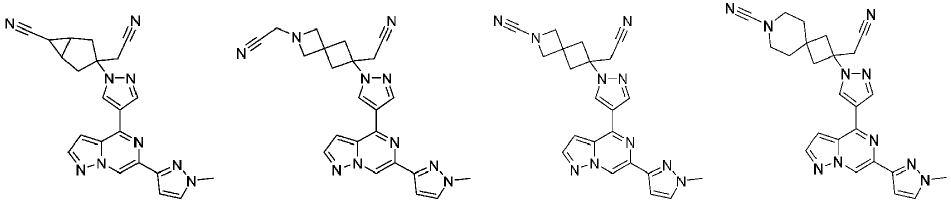

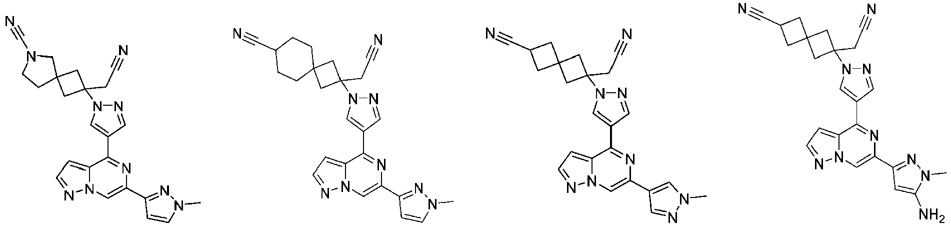

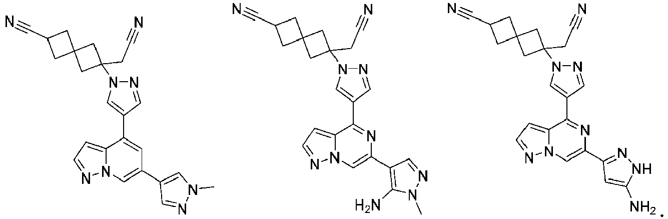

The present disclosure further provides the following compounds or pharmaceutically acceptable salts thereof, which compounds are selected from:

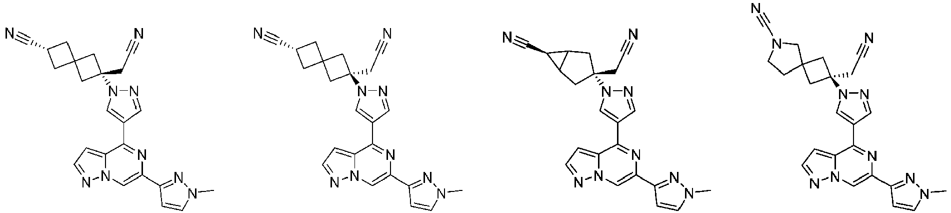

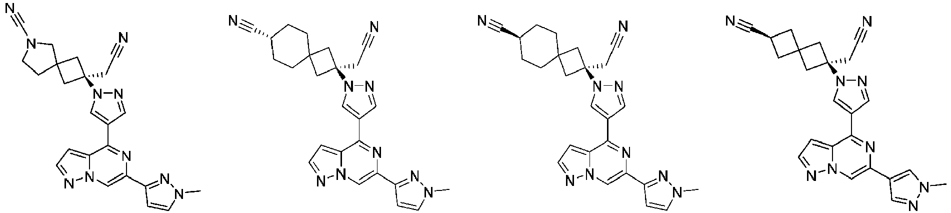

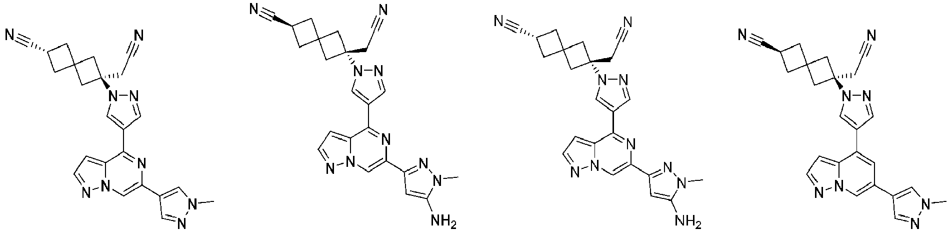

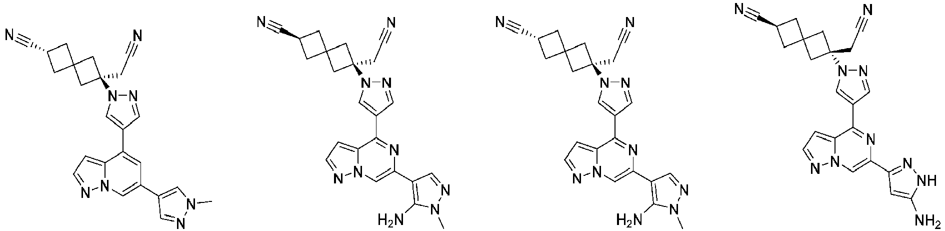

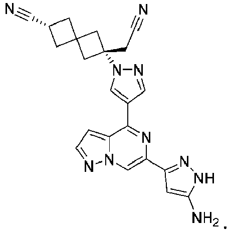

The present disclosure further provides the use of the above-mentioned compounds or pharmaceutically acceptable salts thereof in the preparation of a drug for treating psoriasis and/or inflammatory bowel disease.

The present disclosure further provides the following test methods:

### Test method 1: Test of the anti-inflammatory effect on type II collagen-induced toe swelling model in mice

Objectives: DBA/1 mice were subcutaneously injected with type II collagen at the tail base to establish a mouse arthritis model; this model was used to examine the anti-inflammatory effect of the test sample on type II collagen-induced toe swelling model in mice.

### Experimental method

### 1.1 Reagent formulation

1.43 mL of acetic acid was taken and 500 mL of ultrapure water was added to formulate a 0.05 mol.L-1 acetic acid solution.

An appropriate amount of type II collagen was weighed, an appropriate volume of 0.05 mol.L-1 acetic acid solution was added to a glass bottle containing the collagen, and the glass bottle was turned upside down to completely dissolve the collagen to obtain a collagen solution with a concentration of 2.0 mg.mL-1. An equal volume of Freund's complete adjuvant or Freund's incomplete adjuvant (Freund's complete adjuvant was used for the first immunization, and Freund's incomplete adjuvant was used for the second booster immunization) was taken and placed on ice, and the collagen solution was added dropwise under stirring slowly with a magnetic stirrer at the same time to obtain a uniform emulsion, and the judgment standard was that the emulsion does not diffuse when it is dropped to the water surface.

### 1.2 Animal model preparation

In experiment D1, a 1 mL syringe was used to aspirate the collagen emulsion and subcutaneously inject same into the mice at the tail base. Each animal is injected with 100 µL (1 mg.mL-1) of the emulsion. A total of 170 animals were immunized (the remaining 10 animals were not immunized and served as normal control group). Three weeks after immunization (D21), a booster immunization was performed, with an injection of 100 µL/animal (1 mg.mL-1). The animal status and toe swelling induction were observed regularly. When the increase rate of the foot sole thickness of the left and right hind paws was greater than 20%, the immunity was successful. The degree of swelling of all four paws was scored simultaneously.

The scoring rules for paw swelling are as follows:
1 point: Mild redness and swelling in a single area of the tarsus or the ankle joint;
2 points: Mild redness and swelling in the area from tarsus extending to the ankle joint;
3 points: Moderate redness and swelling in the area from the ankle joint to the metatarsus;
4 points: Severe redness and swelling of joints, foot soles and toes or stiff limbs.

The maximum score for a single limb is 4 points, and the maximum score for each mouse is 16 points.

Foot sole thickness measurement: A vernier caliper was used to measure the thickness of the mouse's foot sole or ankle j oint, and the measurement data was imported into excel.

### 1.3 Animal grouping and administration

The model animals were screened based on indicators such as animal weight, ankle joint thickness, and paw swelling degree scores, as well as the overall condition of the animals. Animals that failed to meet the criteria for animal weight, ankle joint thickness, and paw swelling degree scores were eliminated, and animals in good condition with obvious and similar increase in ankle joint thickness were randomly divided into groups and administration was started for 14 days. A normal group (no immunization) with no treatment was set.

### 1.4 Clinical observation and indicator measurement

After the second immunization, the animal status and toe swelling were observed every day (once/day). After most of the immunized animals showed obvious toe swelling, the animal limb was monitored by scoring. After the start of administration, the animal status was observed every day (once/day), the animal weight was recorded twice a week, the animal limb was scored (2 times/week), and the ankle joint thickness was measured (2 times/week).

### Technical Effects

The compound of the present disclosure shows good selective inhibition on TYK2 and/or JAK1 and/or JAK2 in the in vitro activity test of 4 kinase subtypes JAK1, JAK2, JAK3 and TYK2. The compounds of the present disclosure have good oral bioavailability and higher exposure in mice, which are beneficial to produce good in vivo efficacy. The compound of the present disclosure has no significant effect on the body weight of animals in the IL-23 induced auricle epidermal dysplasia model of C57BL/6 mice, can inhibit the increase in the ear thickness of the modeling side of mice to varying degrees, and can reduce the ear weight of the modeling side of animals. These effects all show a good dose-effect relationship. Oral administration of the compound of the present disclosure can significantly improve the degree of swelling of the paws of mice, improve the score and relative score, and at the same time has no obvious effect on the animal's weight.

### BRIEF DESCRIPTION OF THE DRAWINGS

FIG. 1 shows the three-dimensional structure ellipsoid diagram of compound 1;
FIG. 2 shows the crystal cell packing diagram of compound 1 along the b-axis direction;
FIG. 3 shows the absolute configuration diagram of compound 1.

### DETAILED DESCRIPTION OF THE PREFERRED EMBODIMENT

Unless otherwise stated, the following terms and phrases used herein are intended to have the following meanings. A specific term or phrase should not be considered uncertain or unclear unless specifically defined, but should be understood in its ordinary meaning. When a trade name appears herein, it is intended to refer to the corresponding commodity or an active ingredient thereof.

The term "pharmaceutically acceptable" as used herein refers to those compounds, materials, compositions and/or dosage forms, which are, within the scope of sound medical judgment, suitable for use in contact with human and animal tissues, without excessive toxicity, irritation, allergic reactions or other problems or complications, which is commensurate with a reasonable benefit/risk ratio.

The term "pharmaceutically acceptable salt" refers to a salt of the compound of the present disclosure, which is prepared from the compound having specific substituents found in the present disclosure with relatively non-toxic acids or bases. When compounds of the present disclosure contain relatively acidic functional groups, base addition salts can be obtained by contacting the neutral form of such compounds with a sufficient amount of base, either in pure solution or a suitable inert solvent. Pharmaceutically acceptable base addition salts include sodium, potassium, calcium, ammonium, organic amine or magnesium salts or similar salts. When compounds of the present disclosure contain relatively basic functional groups, acid addition salts can be obtained by contacting the neutral form of such compounds with a sufficient amount of acid, either in pure solution or a suitable inert solvent. Certain specific compounds of the present disclosure contain basic and acidic functional groups and thus can be converted to any base or acid addition salt.

The pharmaceutically acceptable salts of the present disclosure can be synthesized from a parent compound containing acid radicals or base radicals by conventional chemical methods. In general, the method for preparing such salts comprises: in water or an organic solvent or a mixture of both, reacting these compounds in free acid or base forms with a stoichiometric amount of a suitable base or acid to prepare the salts.

Unless otherwise stated, the term "isomer" is intended to include geometric isomers, cis-trans isomers, stereoisomers, enantiomers, optical isomers, diastereomers and tautomers.

The compounds of the present disclosure may exist in specific geometric or stereoisomeric forms. The present disclosure contemplates all such compounds, including cis and trans isomers, (-)- and (+)-enantiomers, (R)- and (S)-enantiomers, diastereomers, (D)-isomers, (L)-isomers, and racemic mixtures and other mixtures thereof, such as enantiomerically or diastereomerically enriched mixtures, all of which fall within the scope of the present disclosure. Additional asymmetric carbon atoms may be present in a substituent such as an alkyl group. All these isomers and mixtures thereof are included in the scope of the present disclosure.

Unless otherwise stated, the term "enantiomer" or "optical isomer" refers to stereoisomers that are mirror images of each other.

Unless otherwise stated, the term "cis-trans isomer" or "geometric isomer" is caused by the fact that double bonds or single bonds of ring-forming carbon atoms cannot rotate freely.

Unless otherwise stated, the term "diastereomer" refers to stereoisomers in which molecules have two or more chiral centers and are not mirror images of each other.

Unless otherwise stated, "(+)" represents right-handed, "(-)" represents left-handed, and "(±)" means racemic.

Unless otherwise stated, the wedge-shaped solid bond ( 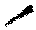 ) and the wedge-shaped dotted bond ( 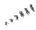 ) represent the absolute configuration of a stereoscopic center; the straight solid bond ( 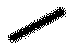 ) and the straight dotted bond ( 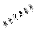 ) represent the relative configuration of a stereoscopic center; the wavy line ( 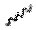 ) represents the wedge-shaped solid bond ( 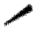 ) or the wedge-shaped dotted bond ( 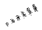 ); or the wavy line ( 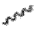 ) represents the straight solid bond ( 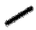 ) or the straight dotted bond ( 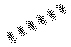 ).

The compounds of the present disclosure may exist in specific geometric or stereoisomeric forms. The present disclosure contemplates all such compounds, including cis and trans isomers, (-)- and (+)-enantiomers, (R)- and (S)-enantiomers, diastereomers, (D)-isomers, (L)-isomers, and racemic mixtures and other mixtures thereof, such as enantiomerically or diastereomerically enriched mixtures, all of which fall within the scope of the present disclosure. Additional asymmetric carbon atoms may be present in a substituent such as an alkyl group. All these isomers and mixtures thereof are included in the scope of the present disclosure.

Unless otherwise stated, the term "tautomer" or "tautomeric form" means that at room temperature, isomers with different functional groups are in dynamic equilibrium and can be quickly converted to each other. Where tautomerization is possible (such as in solution), a chemical equilibrium of tautomers can be achieved. For example, proton tautomers (also known as prototropic tautomers) include interconversion via migration of a proton, such as keto-enol isomerization and imine-enamine isomerization. Valence tautomers include some interconversions by recombination of some of bond-forming electrons. A specific example of keto-enol tautomerization is the interconversion between two tautomers, pentane-2,4-dione and 4-hydroxypent-3-en-2-one.

Unless otherwise stated, the term "rich in one isomer", "isomer enriched", "rich in one enantiomer" or "enantiomerically enriched" refers to that the content of one of the isomers or enantiomers is less than 100%, and the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise stated, the term "isomer excess" or "enantiomeric excess" refers to the difference between the relative percentages of two isomers or two enantiomers. For example, if the content of one isomer or enantiomer is 90%, and the content of the other isomer or enantiomer is 10%, the isomer or enantiomeric excess (ee value) is 80%.

Optically active (R)- and (S)-isomers and D and L isomers can be prepared using chiral synthesis or chiral reagents or other conventional techniques. If a particular enantiomer of a compound of the present disclosure is desired, it can be prepared by asymmetric synthesis or derivatization with a chiral auxiliary, wherein the resulting diastereomeric mixture is separated and the auxiliary groups are cleaved to provide pure desired enantiomers. Alternatively, where the molecule contains a basic functional group (such as an amino group) or an acidic functional group (such as a carboxyl group), diastereomeric salts can be formed with an appropriate optically active acid or base, followed by resolution of the diastereomers using conventional methods well known in the art, and subsequent recovery of the pure enantiomers. In addition, separation of enantiomers and diastereomers is frequently accomplished using chromatography, which uses chiral stationary phases, optionally in combination with chemical derivatization methods (e.g., formation of carbamates from amines).

The compounds of the present disclosure may contain unnatural proportions of atomic isotopes at one or more of the atoms constituting the compound. For example, the compounds may be radiolabeled with radioactive isotopes, such as tritium (³H), iodine-125 (¹²⁵I) or C-14 (¹⁴C). For another example, the hydrogen can be substituted with heavy hydrogen to form deuterated drugs. The bond formed by deuterium and carbon is stronger than the bond formed by ordinary hydrogen and carbon. Compared with undeuterated drugs, deuterated drugs have reduced toxic and side effects, increased drug stability, enhanced efficacy, prolonged biological half-life of drugs and other advantages. All isotopic variations of the compounds of the present disclosure, whether radioactive or not, are intended to be encompassed within the scope of the present disclosure.

Unless otherwise specified, the term "C₁₋₃ alkyl" is used to represent a linear or branched saturated hydrocarbon group consisting of 1 to 3 carbon atoms. The C₁₋₃ alkyl includes C₁₋₂ alkyl, C₂₋₃ alkyl, *etc.;* and it can be monovalent (such as methyl), divalent (such as methylene) or multivalent (such as methine). Examples of C₁₋₃ alkyl include, but are not limited to, methyl (Me), ethyl (Et), propyl (including n-propyl and isopropyl), etc.

Unless otherwise specified, the term "halo" or "halogen" by itself or as part of another substituent means a fluorine, chlorine, bromine or iodine atom.

Unless otherwise specified, "C₅₋₈ cycloalkyl" means a saturated cyclic hydrocarbon group composed of 5 to 8 carbon atoms, which includes a monocyclic system and a bicyclic system, where the bicyclic system includes a spiro ring, a fused ring and a bridged ring. The C₅₋₈ cycloalkyl includes C₃₋₆, C₃₋₅, C₅₋₈, C₅₋₇, C₅₋₆, C₅, C₆ or C₇ cycloalkyl, and the like; and it can be monovalent, bivalent or multivalent. Examples of C₅₋₈ cycloalkyl include, but are not limited to, bicyclo[1.1.1]pentyl, norbornyl, etc.

Unless otherwise specified, "C₅₋₁₂ cycloalkyl" means a saturated cyclic hydrocarbon group composed of 5 to 12 carbon atoms, which includes a monocyclic system, a bicyclic system and a tricyclic system, where the bicyclic system and the tricyclic ring system include a spiro ring, a fused ring and a bridged ring. The C₅₋₁₂ cycloalkyl includes C₅₋₁₀, C₅₋₈, C₅₋₆ cycloalkyl, etc; and it can be monovalent, bivalent or multivalent. Examples of C₅₋₁₂ cycloalkyl include, but are not limited to, bicyclo[1.1.1]pentyl, norbornyl, [2.2.2]bicyclooctane, [4.4.0]bicyclodecane, etc.

Unless otherwise specified, the term "5- to 12-membered heterocycloalkyl" by itself or in combination with other terms respectively represents a saturated cyclic group consisting of 5 to 12 ring atoms, of which 1, 2, 3 or 4 ring atoms are heteroatoms independently selected from O, S and N, and the rest of which are carbon atoms, wherein the carbon atoms are optionally oxo-substituted, the nitrogen atom is optionally quaternized, and the nitrogen and sulfur heteroatoms can be optionally oxidized (i.e., NO and S(O)ₚ, wherein p is 1 or 2). The 5- to 12-membered heterocycloalkyl includes a monocyclic system, a bicyclic system and a tricyclic ring system, wherein the bicyclic system and the tricyclic system include a spiro ring, a fused ring, and a bridge ring. In addition, in terms of the "5- to 12-membered heterocycloalkyl", the heteroatom may occupy the position at which the heterocycloalkyl is connected to the rest of the molecule. The 5- to 12-membered heterocycloalkyl includes 5-to 10-membered, 5- to 8-membered, 5- to 6-membered, 5-membered and 6-membered heterocycloalkyl, etc. Examples of 5- to 12-membered heterocycloalkyl include, but are not limited to, pyrrolidinyl, pyrazolidinyl, imidazolidinyl, tetrahydrothienyl (including tetrahydrothien-2-yl, tetrahydrothien-3-yl, etc.), tetrahydrofuranyl (including tetrahydrofuran-2-yl), tetrahydropyranyl, piperidinyl (including 1-piperidinyl, 2-piperidinyl, 3-piperidinyl, etc.), piperazinyl (including 1-piperazinyl, 2-piperazinyl, etc.), morpholinyl (including 3-morpholinyl, 4-morpholinyl, etc.), dioxanyl, dithianyl, isoxazolidinyl, isothiazolidinyl, 1,2-oxazinyl, 1,2-thiazinyl, hexahydropyridazinyl, homopiperazinyl, homopiperidinyl or dioxepanyl, and the like.

The term "optional" or "optionally" means that the subsequently described event or circumstance may, but not necessarily occur, and that the description includes instances where said event or circumstance occurs and instances where said event or circumstance does not occur.

The term "substituted" means that any one or more hydrogen atoms on the designated atom are substituted with a substituent, which may include heavy hydrogen and hydrogen variants, provided that the valence state of the designated atom is normal, and the substituted compound is stable. Where the substituent is oxygen (i.e., =O), it means that two hydrogen atoms are substituted. Oxygen substitution does not occur on aromatic groups. The term "optionally substituted" means that it may or may not be substituted. Unless otherwise specified, the type and number of substituents may be arbitrary on the basis that they can be achieved in chemistry.

Where any variable (such as R) appears more than once in the composition or structure of a compound, its definition in each case is independent. Thus, for example, if a group is substituted with 0-2 R, the group can optionally be substituted with up to two R, and R in each case has independent options. In addition, combinations of substituents and/or variants thereof are permissible only if such combinations result in stable compounds.

When the number of a linking group is 0, such as -(CRR)₀-, it means that the linking group is a single bond.

When one of the variables is selected from a single bond, it means that the two groups to which it is connected are directly connected. For example, when L represents a single bond in A-L-Z, it means that the structure is actually A-Z.

When a substituent is vacant, it means that the substituent does not exist. For example, when X is vacant in A-X, it means that the structure is actually A. When the linking group listed does not indicate the linking direction thereof, the linking direction is arbitrary, for example, the linking group L is -M-W- in 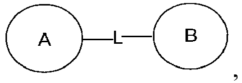 at this situation, -M-W- can connect ring A and ring B in the same direction as the reading order from left to right to form 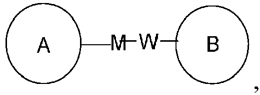 and can also connect ring A and ring B in the opposite direction as the reading order from left to right to form 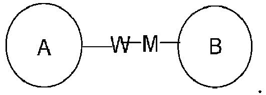 Combinations of the linking groups, substituents, and/or variants thereof are permissible only if such combinations result in stable compounds.

The compounds of the present disclosure can be prepared by various synthetic methods well known to a person skilled in the art, including the specific embodiments listed below, the embodiments formed by the combination with other chemical synthesis methods, and equivalent alternative embodiments well known to a person skilled in the art, wherein the preferred embodiments include but are not limited to the examples of the present disclosure.

The structure of the compound of the present disclosure can be confirmed by conventional methods well known to a person skilled in the art. If the present disclosure relates to the absolute configuration of the compound, the absolute configuration can be confirmed by conventional technical means in the art. For example, single-crystal X-ray diffraction (SXRD) uses a Bruker D8 venture diffractometer to collect the diffraction intensity data of the cultivated single crystal, with a light source of CuKα radiation, and a scanning mode of ϕ/ω scanning. After the related data is collected, a direct method (Shelxs97) is further used to resolve the crystal structure, so that the absolute configuration can be confirmed.

The solvents used in the present disclosure are commercially available. The present disclosure uses the following abbreviations: aq represents water; eq represents equivalent; M represents mol/L; DCM represents dichloromethane; PE represents petroleum ether; DMF represents N, N-dimethylformamide; DMSO represents dimethyl sulfoxide; EtOAc represents ethyl acetate; EtOH represents ethanol; MeOH represents methanol; CBz represents benzyloxycarbonyl, which is an amine protecting group; Boc represents tertbutoxycarbonyl, which is an amine protecting group; r.t. represents room temperature; O/N represents overnight; THF represents tetrahydrofuran; Boc₂O represents di-tert-butyl dicarbonate; Trifluoroacetic acid represents trifluoroacetic acid; DIPEA represents diisopropylethylamine; SOCl₂ represents thionyl chloride; Xphos-Pd-G2 represents chloro(2-dicyclohexylphosphino-2',4',6'-triisopropyl-1,1'-biphenyl)[2-(2'-amino-1,1'-biphenyl)]palladium(II); mp represents melting point; DBU represents 1.8-diazabicyclo[5.4.0]undec-7-ene; BrettPhos Pd G3 represents methanesulfonato(2-dicyclohexylphosphine)-3,6-dimethoxy-2',4',6'-triisopropyl-1,1'-biphenyl)(2'-amino-1,1'-biphenyl-2-yl)palladium(II).

Compounds are named according to conventional naming principles in the field or using ChemDraw^{®} software, and commercially available compounds are named using supplier catalog names.

### Detailed description of the preferred embodiment

The present disclosure will be described in detail with the following examples, but not imply any adverse limitation to the present disclosure. The present disclosure has been described in detail herein, and the specific embodiments thereof are also disclosed therein. For a person skilled in the art, without departing from the spirit and scope of the present disclosure, all the variations and improvements made to the specific embodiments of the present disclosure would have been obvious.

### Examples 1 and 2

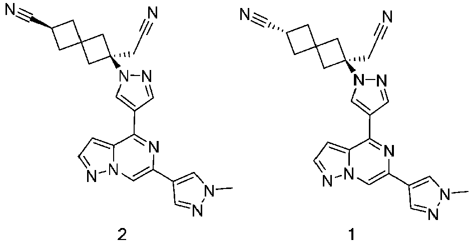

### Synthetic route:

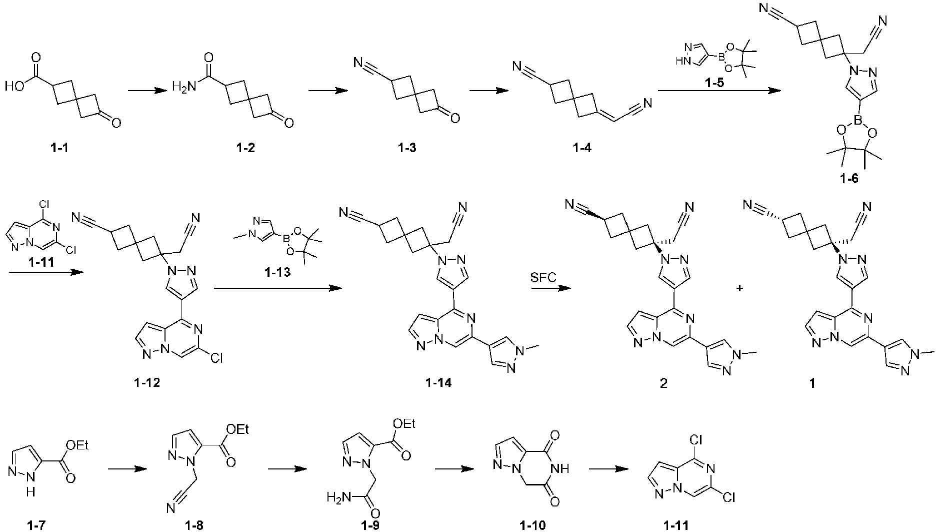

### Step 1: Synthesis of compound 1-2

Compound **1-1** (10 g, 64.87 mmol) was added to anhydrous DCM (100.00 mL), and (COCl)₂ (12.35 g, 97.30 mmol, 8.52 mL) and DMF (474.13 mg, 6.49 mmol, 499.09 µL) were added dropwise at 0-5 °C, and the mixture was stirred at 0-5 °C for 1 h under nitrogen protection. After the reaction was completed, the reaction liquid was concentrated to dryness under reduced pressure, and anhydrous DCM (120.00 mL) was added again. The resulting solution was cooled to 0 °C. Ammonia gas was continuously bubbled into the reaction system for 10 min, and then the reaction system was warmed to 25 °C and stirred for 30 min. The reaction liquid was concentrated to dryness under reduced pressure, and the residue was purified by silica gel column chromatography (dichloromethane:methanol = 10 : 1) to obtain compound 1-**2.** ¹HNMR(400MHz, CD₃OD)δ 3.15-3.25 (m, 3 H), 2.90-3.05 (m, 2 H), 2.45-2.55 (m, 2 H), 2.25-2.40 (m, 2 H).

### Step 2: Synthesis of compound 1-3

Under nitrogen protection at 0-5 °C, trifluoroacetic anhydride (18.87 g, 89.83 mmol, 12.49 mL) and triethylamine (22.72 g, 224.58 mmol, 31.26 mL) were added sequentially to a solution of compound **1-2** (6.88 g, 44.92 mmol) in dichloromethane (140 mL), and then the reaction liquid was stirred at 0-5 °C for another 1 h. After the reaction was completed, water (300 mL) was added, the mixture was extracted with dichloromethane (150 mL × 2), the combined extracts were washed with saturated brine (200 mL), dried over anhydrous sodium sulfate, and filtered, and the filtrate was concentrated. The residue was purified by silica gel column chromatography (petroleum ether:ethyl acetate = 3 : 1) to obtain compound **1-3.** ¹HNMR(400MHz,CD₃OD)δ 3.05-3.35 (m, 5 H), 2.56-2.75 (m, 4 H).

### Step 3: Synthesis of compound 1-4

Under nitrogen protection at 25 °C, a solution of diethyl cyanomethylphosphonate (3.33 g, 18.82 mmol, 3.03 mL), LiBr (2.04 g, 23.53 mmol, 590.56 µL) and triethylamine (3.17 g, 31.37 mmol, 4.37 mL) in tetrahydrofuran (20.00 mL) was added to a solution of compound **1-3** (2.12 g, 15.68 mmol) in tetrahydrofuran (10.00 mL). The resulting reaction liquid was stirred at 25 °C for 16 h. After the reaction was completed, water (100.00 mL) was added to the reactant, and the mixture was extracted with ethyl acetate (60 mL × 2), the combined extracts were washed with saturated brine (80 mL), dried over anhydrous sodium sulfate, and filtered, and the filtrate was concentrated. The residue was purified by silica gel column chromatography (petroleum ether:ethyl acetate = 10 : 1 to 3 : 1) to obtain compound 1-4. ¹H NMR(400MHz, CDCl₃)δ 5.13-5.75 (m, 1 H), 2.98-3.10 (m, 3 H), 2.88-2.96 (m, 2 H), 2.40-2.65 (m, 4 H).

### Step 4: Synthesis of compound 1-6

Under nitrogen protection at 25 °C, DBU (4.58 g, 30.10 mmol, 4.54 mL) and compound **1-4** (2 g, 12.64 mmol) were added in sequence to a solution of compound **1-5** (2.34 g, 12.04 mmol) in acetonitrile (40.00 mL) and the mixture was stirred at 25 °C for 16 h. After the reaction was completed, it was quenched with 1M KH₂PO₄ aqueous solution (100 mL), and the mixture was extracted with ethyl acetate (60 mL × 2), the combined extracts were washed with saturated brine (80 mL), dried over anhydrous sodium sulfate, and filtered, and the filtrate was concentrated. The residue was purified by silica gel column chromatography (petroleum ether:ethyl acetate = 10 : 1 to 1 : 1) to obtain compound **1-6.** ¹**H NMR**(400MHz, CDCl₃)6 7.84 (s, 1H), 7.83 (s, 1H), 3.00-3.10 (m, 1H), 2.80-2.95 (m, 4H), 2.55-2.75 (m, 4H), 2.30-2.40 (m, 2H), 1.32 (s, 12 H).

### Step 5: Synthesis of compound 1-8

Under nitrogen protection at 25 °C, compound **1-7** (200 g, 1.43 mol), 2-chloroacetonitrile (118.52 g, 1.57 mol) and cesium carbonate (557.99 g, 1.71 mol) were added to DMF (1 L) in sequence, the resulting reaction liquid was stirred at 25 °C for 16 h. After the reaction was completed, the reaction liquid was filtered through diatomaceous earth, and the filtrate was concentrated to dryness under reduced pressure. The crude product obtained was purified by silica gel column chromatography (petroleum ether:ethyl acetate = 3 : 1) to obtain compound **1-8. ¹H** NMR(400MHz, CDCl₃)6 7.60(d, *J*= 2.0 Hz, 1 H), 6.92 (d, *J*= 2.0 Hz, 1 H), 5.54 (s, 2 H), 4.40 (q, *J*=7.2 Hz, 2 H), 1.41 (t, *J*=7.2 Hz, 3 H).

### Step 6: Synthesis of compound 1-9

Under nitrogen protection at 20 °C, a mixture of compound **1-8** (65 g, 362.77 mmol), concentrated sulfuric acid (177.90 g, 1.81 mol) and trifluoroacetic acid (206.82 g, 1.81 mol, 134.30 mL) was stirred for 16 h. After the reaction was completed, concentration was performed under reduced pressure to remove excess trifluoroacetic acid, the residue was poured into ice, the resulting aqueous solution was extracted with ethyl acetate (150 mL × 6), and the combined extracts was neutralized with saturated sodium bicarbonate aqueous solution until no bubbles appeared, liquid separation was performed, the organic phase was washed with saturated brine (200 mL), dried over anhydrous sodium sulfate, and filtered, and the filtrate was concentrated to obtain compound **1-9. ¹H NMR**(400MHz , CDCl₃)δ 7.57 (d, *J* = 2.0 Hz, 1 H), 6.90 (d, *J* = 2.0 Hz, 1 H), 6.25 (brs, 1 H), 5.87 (brs, 1 H), 5.25 (s, 2 H), 4.33 (q, *J* = 7.2 Hz, 2 H), 1.36 (t, *J* = 7.2 Hz, 3 H).

### Step 7: Synthesis of compound 1-10

Under nitrogen protection, t-BuOK (1 M, 370.10 mL) solution was added to a solution of compound **1-9** (36.49 g, 185.05 mmol) in ethanol (600 mL), and the resulting mixture was heated to 70 °C and stirred for 16 h. After the reaction was completed, the reaction mixture was cooled to 20 °C, adjusted to pH 6 with concentrated hydrochloric acid, and the resulting suspension was concentrated to dryness under reduced pressure to obtain crude compound 1-**10. ¹H NMR**(400MHz**,** DMSO-*d*₆)δ 11.82 (brs, 1H), 7.75 (s, 1 H), 6.95 (s, 1H), 5.16 (s, 2H).

### Step 8: Synthesis of compound 1-11

Under nitrogen protection, a mixture of compound **1-10** (28 g, 185.28 mmol), POCl₃ (298.30 g, 1.95 mol, 180.79 mL) and pyridine hydrochloride (21.41 g, 185.28 mmol) was heated to 120 °C and stirred for 16 h. After the reaction was completed, concentration was performed under reduced pressure to remove excess POCl₃. The solid obtained was dissolved in ethyl acetate (500 mL), and then the solution was slowly poured into 1 M NaH₂PO₄ aqueous solution (500 mL), the organic phase was separated, the aqueous phase was extracted with ethyl acetate (200 mL × 2), the combined organic phases was washed with saturated brine (350 mL), dried over anhydrous sodium sulfate, and filtered, the filtrate was concentrated, and the crude product was purified by silica gel column chromatography (petroleum ether : ethyl acetate : dichloromethane = 5 : 1 : 1) to obtain compound **1-11. ¹H NMR**(400MHz , CDCl₃)δ 8.45 (s, 1H), 8.06 (s, 1H), 6.94 (s, 1H).

### Step 9: Synthesis of compound 1-12

Under nitrogen protection, dioxane (20 mL) and water (4 mL) were added to a mixture of compound **1-11** (1 g, 5.32 mmol), compound **1-6** (2.06 g, 5.85 mmol), bis(tert-butylphosphine)palladium (271.82 mg, 531.88 µmol) and K₃PO₄ (3.39 g, 15.96 mmol). Vacuumization under reduced pressure and nitrogen filling was performed three times for the resulting mixture, and then the mixture was stirred at 25 °C for 16 h. After the reaction was completed, the reaction liquid was diluted with water (200 mL), the aqueous phase was extracted with ethyl acetate (80 mL × 2), the combined organic phases were washed with saturated brine (60 mL), dried over anhydrous sodium sulfate, and filtered, and the filtrate was concentrated. The crude product obtained was purified by silica gel column chromatography (petroleum ether : ethyl acetate = 5 : 1 to 1 : 2) to obtain compound **1-12.** MS ESI calculated for C₁₉H₁₆ClN₇ [M + H]⁺ 378, found 378.

### Step 10: Synthesis of compounds 1 and 2

Under nitrogen protection, dioxane (8 mL) and water (2 mL) were added a mixture of compound **1-12** (0.3 g, 794.01 µmol), compound **1-13** (247.81 mg, 1.19 mmol), Xphos-Pd-G2 (62.47 mg, 79.40 µmol) and Na₂CO₃ (252.47 mg, 2.38 mmol). Vacuumization under reduced pressure and nitrogen filling was performed three times for the resulting mixture, and then the mixture was heated to 100 °C and stirred for 16 h. After the reaction was completed, the reaction liquid was diluted with water (60 mL), the aqueous phase was extracted with ethyl acetate (30 mL × 2), the combined organic phases were washed with saturated brine (30 mL), dried over anhydrous sodium sulfate, and filtered, and the filtrate was concentrated. The crude product obtained was purified by silica gel column chromatography (petroleum ether : ethyl acetate = 5 : 1 to 1 : 5) to obtain compound **1-14.** Compound **1-14** was subjected to chiral resolution (chiral column model: DAICEL CHIRALPAK IG (250 mm*30 mm*10 µm); mobile phase: A: CO₂, B: 0.05% diethylamine/isopropanol, isopropanol (0.05% diethylamine) and supercritical fluid CO₂ from 55% to 55%, flow rate at 3 mL/min), compound 1 and compound **2** were obtained after resolution, and the retention time was 1.086 min and 1.241 min, respectively (analysis method: column model Chiralpak AD-3 50 × 4.6 mm I.D., 3 µm; mobile phase: A: CO₂, B: 0.05% diethylamine/isopropanol; gradient: B% = 40%; flow rate at 3 mL/min; column temperature 35 °C). Compound **1: ¹H NMR**(400MHz, CDCl₃)δ 8.48 (s, 1 H), 8.30 (d, *J* = 4.0 Hz, 2 H), 8.05 (d, *J* = 2.4 Hz, 1 H), 7.95 (s, 2 H), 6.93 (d, J =1.6 Hz, 1 H), 4.01 (s, 3 H), 2.95-3.19 (m, 5 H), 2.85-2.95 (m, 2H), 2.62-2.72 (m, 2H), 2.44-2.54 (m, 2H). MS ESI calculated for C₂₃H₂₁N₉ [M + H]⁺ 424, found 424. Compound **2: ¹H NMR**, CDCl₃)8 8.48 (s, 1 H), 8.30 (d, *J* = 4.0 Hz, 2 H), 8.05 (d, *J* = 2.4 Hz, 1 H), 7.95 (s, 2 H), 6.93 (d, *J* = 1.6 Hz, 1 H), 4.01 (s, 3 H), 2.95-3.18 (m, 5 H), 2.74-2.87 (m, 2H), 2.60-2.73 (m, 2H), 2.40-2.56 (m, 2H). MS ESI calculated for C₂₃H₂₁N₉ [M + H]⁺ 424, found 424.

The absolute configuration of compound 1 was determined by single crystal X-ray diffraction analysis:
The crystal of compound **1** was obtained from the compound **1** obtained in the above examples by using the solvent evaporation method under methanol conditions and culturing at room temperature for 5 days. The crystal belongs to the monoclinic system, with space group P2₁, and the crystal cell parameters are: a = 16.4650(3) Å, b = 6.85270(10) Å, c = 19.4487(4) Å, *α* = *y* = 90 °, *β* = 102.6250(10)°, volume V = 2141.33(7) Å³.

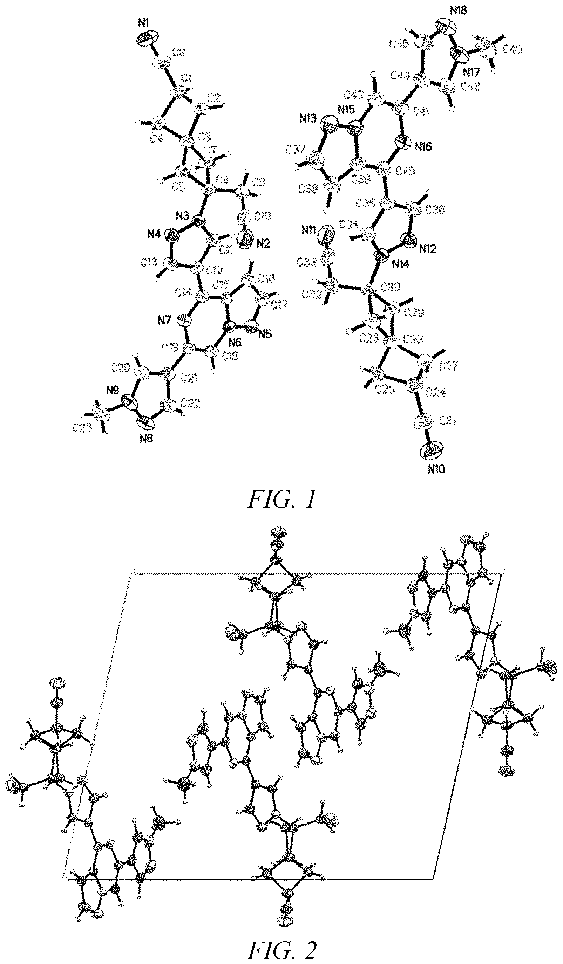

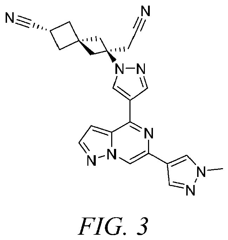

The three-dimensional structure ellipsoid diagram of compound **1,** the crystal cell packing diagram along the b-axis direction, and the absolute configuration diagram of the compound are shown in Figs. 1, 2 and 3. The crystal structure data and parameters of compound **1** are shown in Tables 1, 2, 3, 4, 5 and 6.

**Table 1 Crystal structure refinement information table**

| | |
|---|---|
| Identification code | compound 1 |
| Empirical formula | C23 H21 N9 |
| Formula weight | 423.49 |
| Temperature | 173(2) K |
| Wavelength | 1.54178 A |
| Crystal system, space group | Monoclinic, P2(1) |
| Unit cell dimensions | a = 16.4650(3) A alpha = 90 deg. |
| | b = 6.85270(10) A beta = 102.6250(10) deg. |
| | c = 19.4487(4) A gamma = 90 deg. |
| Volume | 2141.33(7) A^3 |
| Z, Calculated density | 4, 1.314 Mg/m^3 |
| Absorption coefficient | 0.680 mm^-1 |
| F(000) | 888 |
| Crystal size | 0.180 x 0.160 x 0.140 mm |
| Theta range for data collection | 2.328 to 68.308 deg. |
| Limiting indices | -19<=h<=19, -8<=k<=8, -23<=1<=23 |
| Reflections collected / unique | 32705 / 7815 [R(int) = 0.0432] |
| Completeness to theta= 67.679 | 99.6 % |
| Absorption correction | Semi-empirical from equivalents |
| Max. and min. transmission | 0.7531 and 0.6455 |
| Refinement method | Full-matrix least-squares on F^2 |
| Data / restraints / parameters | 7815 / 1 / 579 |
| Goodness-of-fit on F^2 | 1.045 |
| Final R indices [I>2sigma(I)] | R₁ = 0.0373, wR2 = 0.0954 |
| R indices(all data) | R₁ = 0.0460, wR2 = 0.1009 |
| Absolute structure parameter | 0.1(2) |
| Extinction coefficient | n/a |
| Largest diff. peak and hole | 0.194 and -0.195 e.A^-3 |

**Table 2: Atomic coordinates (× 10⁴) and equivalent isotropic displacement parameters (Å² × 10³) of the crystal**

| | x | y | z | U(eq) |
|---|---|---|---|---|
| N(4) | 3254(2) | 3835(4) | 74(1) | 30(1) |
| C(4) | 4785(2) | -2118(5) | 494(2) | 31(1) |
| N(5) | -1121(2) | 4182(5) | -1142(2) | 36(1) |
| C(5) | 4266(2) | 1212(5) | 987(2) | 28(1) |
| N(6) | -389(2) | 5067(5) | -1151(1) | 28(1) |
| C(6) | 3311(2) | 1157(5) | 914(2) | 25(1) |
| N(7) | 1163(2) | 6557(4) | -1080(1) | 27(1) |
| C(7) | 3317(2) | -1047(5) | 721(2) | 31(1) |
| N(8) | 429(2) | 12306(5) | -2310(1) | 38(1) |
| C(8) | 5790(2) | -4611(6) | 1219(2) | 41(1) |
| N(9) | 1250(2) | 11885(4) | -2103(2) | 36(1) |
| C(9) | 3025(2) | 1497(5) | 1606(2) | 31(1) |
| N(10) | -1353(2) | 15203(6) | 3741(2) | 59(1) |
| C(10) | 3031(2) | 3575(7) | 1786(2) | 34(1) |
| N(1) | 6437(2) | -5286(6) | 1324(2) | 60(1) |
| C(1) | 4958(2) | -3748(4) | 1064(2) | 32(1) |
| N(11) | 1961(2) | 4389(6) | 3083(2) | 54(1) |
| C(11) | 2018(2) | 2512(5) | 116(2) | 27(1) |
| N(12) | 1719(2) | 5818(4) | 4926(2) | 32(1) |
| C(12) | 1861(2) | 4037(5) | -359(2) | 25(1) |
| N(13) | 6094(2) | 5412(5) | 6130(2) | 37(1) |
| C(13) | 2657(2) | 4791(5) | -362(2) | 29(1) |
| N(14) | 2134(1) | 7180(4) | 4633(1) | 26(1) |
| C(14) | 1084(2) | 4899(5) | -755(2) | 24(1) |
| N(15) | 5363(2) | 4539(5) | 6142(1) | 28(1) |
| C(15) | 288(2) | 4044(5) | -776(2) | 26(1) |
| N(16) | 3810(2) | 3062(4) | 6086(1) | 28(1) |
| C(16) | -40(2) | 2403(6) | -516(2) | 31(1) |
| N(17) | 3732(2) | -2256(5) | 7124(2) | 37(1) |
| C(17) | -898(2) | 2563(6) | -756(2) | 37(1) |
| N(18) | 4553(2) | -2664(5) | 7333(2) | 40(1) |
| C(18) | -314(2) | 6803(5) | -1486(2) | 29(1) |
| C(19) | 466(2) | 7520(5) | -1439(2) | 26(1) |
| C(20) | 1389(2) | 10158(5) | -1777(2) | 32(1) |
| C(21) | 624(2) | 9372(5) | -1762(2) | 28(1) |
| C(22) | 49(2) | 10780(6) | -2099(2) | 34(1) |
| C(23) | 1864(2) | 13221(6) | -2271(2) | 49(1) |
| C(24) | -462(2) | 12034(4) | 3830(2) | 35(1) |
| C(25) | 248(2) | 11877(6) | 3418(2) | 33(1) |
| C(26) | 746(2) | 10735(5) | 4059(2) | 28(1) |
| C(27) | 206(2) | 11704(6) | 4512(2) | 36(1) |
| C(28) | 1705(2) | 10672(5) | 4269(2) | 33(1) |
| C(29) | 732(2) | 8467(5) | 4001(2) | 27(1) |
| C(30) | 1682(2) | 8484(5) | 4076(2) | 27(1) |
| C(3 1) | -962(2) | 13824(7) | 3773(2) | 44(1) |
| C(32) | 1978(2) | 8104(6) | 3392(2) | 34(1) |
| C(33) | 1964(2) | 6028(7) | 3209(2) | 36(1) |
| C(34) | 2963(2) | 7071(5) | 4881(2) | 28(1) |
| C(35) | 3112(2) | 5569(5) | 5366(2) | 25(1) |
| C(36) | 2310(2) | 4854(5) | 5365(2) | 31(1) |
| C(37) | 5867(2) | 7032(6) | 5743(2) | 37(1) |
| C(3 8) | 5013(2) | 7208(6) | 5512(2) | 32(1) |
| C(39) | 4682(2) | 5564(5) | 5774(2) | 26(1) |
| C(40) | 3885(2) | 4718(5) | 5761(2) | 25(1) |
| C(41) | 4510(2) | 2101(5) | 6442(2) | 27(1) |
| C(42) | 5291(2) | 2807(5) | 6475(2) | 30(1) |
| C(43) | 3594(2) | -529(6) | 6786(2) | 36(1) |
| C(44) | 4358(2) | 257(6) | 6769(2) | 29(1) |
| C(45) | 4929(2) | -1135(5) | 7117(2) | 34(1) |
| C(46) | 3125(2) | -3581(6) | 7299(2) | 51(1) |
| N(2) | 3032(2) | 5194(6) | 1913(2) | 50(1) |
| C(2) | 4724(2) | -2261(5) | 1590(2) | 29(1) |
| N(3) | 2849(1) | 2430(4) | 365(1) | 24(1) |
| C(3) | 4279(2) | -1029(5) | 950(2) | 26(1) |

**Table 3: Bond length (Å) and bond angle (°) of bonding atoms**

| | | | |
|---|---|---|---|
| N(4)-C(13) | 1.323(4) | C(14)-C(15) | 1.428(4) |
| N(4)-N(3) | 1.362(4) | N(15)-C(42) | 1.369(4) |
| C(4)-C(3) | 1.536(4) | N(15)-C(39) | 1.382(4) |
| C(4)-C(1) | 1.555(4) | C(15)-C(16) | 1.389(5) |
| N(5)-C(17) | 1.345(5) | N(16)-C(40) | 1.317(4) |
| N(5)-N(6) | 1.352(4) | N(16)-C(41) | 1.376(4) |
| C(5)-C(3) | 1.538(5) | C(16)-C(17) | 1.391(4) |
| C(5)-C(6) | 1.547(4) | N(17)-C(43) | 1.349(5) |
| N(6)-C(18) | 1.374(4) | N(17)-N(18) | 1.354(4) |
| N(6)-C(15) | 1.381(4) | N(17)-C(46) | 1.445(4) |
| C(6)-N(3) | 1.458(4) | N(18)-C(45) | 1.331(5) |
| C(6)-C(9) | 1.539(5) | C(18)-C(19) | 1.360(4) |
| C(6)-C(7) | 1.557(5) | C(19)-C(21) | 1.465(5) |
| N(7)-C(14) | 1.320(4) | C(20)-C(21) | 1.375(4) |
| N(7)-C(19) | 1.375(4) | C(21)-C(22) | 1.409(5) |
| C(7)-C(3) | 1.549(4) | C(24)-C(31) | 1.468(5) |
| N(8)-C(22) | 1.328(4) | C(24)-C(27) | 1.544(4) |
| N(8)-N(9) | 1.355(4) | C(24)-C(25) | 1.559(4) |
| C(8)-N(1) | 1.139(4) | C(25)-C(26) | 1.545(5) |
| C(8)-C(1) | 1.461(4) | C(26)-C(27) | 1.532(5) |
| N(9)-C(20) | 1.339(4) | C(26)-C(28) | 1.542(4) |
| N(9)-C(23) | 1.453(4) | C(26)-C(29) | 1.559(5) |
| C(9)-C(10) | 1.466(5) | C(28)-C(30) | 1.544(5) |
| N(10)-C(31) | 1.138(5) | C(29)-C(30) | 1.538(4) |
| C(10)-N(2) | 1.136(5) | C(30)-C(32) | 1.536(5) |
| C(1)-C(2) | 1.552(4) | C(32)-C(33) | 1.466(6) |
| N(11)-C(33) | 1.149(6) | C(34)-C(35) | 1.381(4) |
| C(11)-N(3) | 1.348(3) | C(35)-C(36) | 1.407(4) |
| C(11)-C(12) | 1.381(4) | C(35)-C(40) | 1.458(4) |
| N(12)-C(36) | 1.323(4) | C(37)-C(38) | 1.384(4) |
| N(12)-N(14) | 1.355(4) | C(38)-C(39) | 1.396(5) |
| C(12)-C(13) | 1.410(4) | C(39)-C(40) | 1.430(4) |
| C(12)-C(14) | 1.467(4) | C(41)-C(42) | 1.362(4) |
| N(13)-C(37) | 1.348(5) | C(41)-C(44) | 1.461(5) |
| N(13)-N(15) | 1.350(4) | C(43)-C(44) | 1.375(5) |
| N(14)-C(34) | 1.346(3) | C(44)-C(45) | 1.405(5) |
| N(14)-C(30) | 1.474(4) | C(2)-C(3) | 1.550(4) |
| C(13)-N(4)-N(3) | 104.7(2) | C(20)-C(21)-C(19) | 126.7(3) |
| C(3)-C(4)-C(1) | 88.6(2) | C(22)-C(21)-C(19) | 128.9(3) |
| C(17)-N(5)-N(6) | 104.0(3) | N(8)-C(22)-C(21) | 111.6(3) |
| C(3)-C(5)-C(6) | 89.8(3) | C(31)-C(24)-C(27) | 118.0(3) |
| N(5)-N(6)-C(18) | 124.5(3) | C(31)-C(24)-C(25) | 119.4(3) |
| N(5)-N(6)-C(15) | 112.6(3) | C(27)-C(24)-C(25) | 87.5(2) |
| C(18)-N(6)-C(15) | 123.0(3) | C(26)-C(25)-C(24) | 86.9(2) |
| N(3)-C(6)-C(9) | 109.8(3) | C(27)-C(26)-C(28) | 123.2(3) |
| N(3)-C(6)-C(5) | 114.2(3) | C(27)-C(26)-C(25) | 88.4(2) |
| C(9)-C(6)-C(5) | 114.5(3) | C(28)-C(26)-C(25) | 124.0(3) |
| N(3)-C(6)-C(7) | 115.7(3) | C(27)-C(26)-C(29) | 118.2(3) |
| C(9)-C(6)-C(7) | 112.1(3) | C(28)-C(26)-C(29) | 89.4(3) |
| C(5)-C(6)-C(7) | 89.2(3) | C(25)-C(26)-C(29) | 116.8(3) |
| C(14)-N(7)-C(19) | 119.8(3) | C(26)-C(27)-C(24) | 87.9(2) |
| C(3)-C(7)-C(6) | 89.0(2) | C(26)-C(28)-C(30) | 89.7(3) |
| C(22)-N(8)-N(9) | 104.3(3) | C(30)-C(29)-C(26) | 89.3(3) |
| N(1)-C(8)-C(1) | 178.5(4) | N(14)-C(30)-C(32) | 109.2(3) |
| C(20)-N(9)-N(8) | 112.7(3) | N(14)-C(30)-C(29) | 113.9(3) |
| C(20)-N(9)-C(23) | 127.5(3) | C(32)-C(30)-C(29) | 115.1(3) |
| N(8)-N(9)-C(23) | 119.8(3) | N(14)-C(30)-C(28) | 115.4(3) |
| C(10)-C(9)-C(6) | 111.7(3) | C(32)-C(30)-C(28) | 112.3(3) |
| N(2)-C(10)-C(9) | 178.7(5) | C(29)-C(30)-C(28) | 90.0(3) |
| C(8)-C(1)-C(2) | 119.1(3) | N(10)-C(31)-C(24) | 178.7(4) |
| C(8)-C(1)-C(4) | 117.1(3) | C(33)-C(32)-C(30) | 112.6(3) |
| C(2)-C(1)-C(4) | 88.0(2) | N(11)-C(33)-C(32) | 178.3(5) |
| N(3)-C(11)-C(12) | 107.4(3) | N(14)-C(34)-C(35) | 107.5(3) |
| C(36)-N(12)-N(14) | 104.3(2) | C(34)-C(35)-C(36) | 103.4(3) |
| C(11)-C(12)-C(13) | 104.0(3) | C(34)-C(35)-C(40) | 131.5(3) |
| C(11)-C(12)-C(14) | 132.1(3) | C(36)-C(35)-C(40) | 124.9(3) |
| C(13)-C(12)-C(14) | 123.7(3) | N(12)-C(36)-C(35) | 112.6(3) |
| C(37)-N(13)-N(15) | 103.6(3) | N(13)-C(37)-C(38) | 113.0(3) |
| N(4)-C(13)-C(12) | 112.1(3) | C(37)-C(38)-C(39) | 105.2(3) |
| C(34)-N(14)-N(12) | 112.1(3) | N(15)-C(39)-C(38) | 105.2(3) |
| C(34)-N(14)-C(30) | 127.4(3) | N(15)-C(39)-C(40) | 116.1(3) |
| N(12)-N(14)-C(30) | 120.4(2) | C(38)-C(39)-C(40) | 138.7(3) |
| N(7)-C(14)-C(15) | 121.7(3) | N(16)-C(40)-C(39) | 121.5(3) |
| N(7)-C(14)-C(12) | 115.5(3) | N(16)-C(40)-C(35) | 115.7(3) |
| C(15)-C(14)-C(12) | 122.7(3) | C(39)-C(40)-C(35) | 122.7(3) |
| N(13)-N(15)-C(42) | 124.2(3) | C(42)-C(41)-N(16) | 122.1(3) |
| N(13)-N(15)-C(39) | 113.0(3) | C(42)-C(41)-C(44) | 122.5(3) |
| C(42)-N(15)-C(39) | 122.8(3) | N(16)-C(41)-C(44) | 115.4(3) |
| N(6)-C(15)-C(16) | 105.7(3) | C(41)-C(42)-N(15) | 117.6(3) |
| N(6)-C(15)-C(14) | 115.8(3) | N(17)-C(43)-C(44) | 107.4(3) |
| C(16)-C(15)-C(14) | 138.5(3) | C(43)-C(44)-C(45) | 104.0(3) |
| C(40)-N(16)-C(41) | 119.8(3) | C(43)-C(44)-C(41) | 126.4(3) |
| C(15)-C(16)-C(17) | 105.1(3) | C(45)-C(44)-C(41) | 129.6(3) |
| C(43)-N(17)-N(18) | 112.2(3) | N(18)-C(45)-C(44) | 112.2(3) |
| C(43)-N(17)-C(46) | 128.0(3) | C(3)-C(2)-C(1) | 88.2(2) |
| N(18)-N(17)-C(46) | 119.8(3) | C(11)-N(3)-N(4) | 111.8(3) |
| N(5)-C(17)-C(16) | 112.7(3) | C(11)-N(3)-C(6) | 127.9(3) |
| C(45)-N(18)-N(17) | 104.2(3) | N(4)-N(3)-C(6) | 120.2(2) |
| C(19)-C(18)-N(6) | 117.5(3) | C(4)-C(3)-C(5) | 121.8(3) |
| C(18)-C(19)-N(7) | 122.1(3) | C(4)-C(3)-C(7) | 118.6(3) |
| C(18)-C(19)-C(21) | 122.6(3) | C(5)-C(3)-C(7) | 89.8(3) |
| N(7)-C(19)-C(21) | 115.2(3) | C(4)-C(3)-C(2) | 88.8(2) |
| N(9)-C(20)-C(21) | 107.1(3) | C(5)-C(3)-C(2) | 121.0(3) |
| C(20)-C(21)-C(22) | 104.3(3) | C(7)-C(3)-C(2) | 120.0(3) |

**Table 4: Interatomic torsion angle value (°)**

| | U11 | U22 | U33 | U23 | U13 | U12 |
|---|---|---|---|---|---|---|
| N(4) | 28(1) | 28(2) | 34(2) | 5(1) | 4(1) | -4(1) |
| C(4) | 32(2) | 26(2) | 36(2) | -1(2) | 9(2) | 5(2) |
| N(5) | 25(1) | 35(2) | 49(2) | 9(2) | 10(1) | -1(2) |
| C(5) | 24(2) | 23(2) | 37(2) | 1(2) | 6(2) | 2(2) |
| N(6) | 22(1) | 30(2) | 31(2) | 3(1) | 5(1) | 3(1) |
| C(6) | 23(2) | 22(2) | 30(2) | 3(2) | 5(1) | 0(2) |
| N(7) | 29(1) | 24(2) | 27(2) | 2(1) | 3(1) | 1(1) |
| C(7) | 25(2) | 22(2) | 42(2) | 2(2) | 1(2) | 0(2) |
| N(8) | 52(2) | 28(2) | 31(2) | 7(2) | 0(1) | 0(2) |
| C(8) | 38(2) | 33(2) | 49(2) | 2(2) | 6(2) | 7(2) |
| N(9) | 46(2) | 29(2) | 33(2) | 4(1) | 6(1) | -9(2) |
| C(9) | 25(2) | 35(2) | 32(2) | 5(2) | 6(2) | 2(2) |
| N(10) | 48(2) | 60(2) | 66(2) | 4(2) | 8(2) | 23(2) |
| C(10) | 28(2) | 45(3) | 33(2) | 1(2) | 12(2) | 0(2) |
| N(1) | 38(2) | 62(2) | 79(2) | 11(2) | 9(2) | 22(2) |
| C(1) | 28(1) | 23(1) | 42(2) | 1(1) | 2(1) | 1(1) |
| N(11) | 59(2) | 52(2) | 58(2) | -3(2) | 31(2) | 4(2) |
| C(11) | 21(1) | 28(2) | 32(2) | 3(2) | 5(1) | 0(2) |
| N(12) | 26(1) | 32(2) | 37(2) | 9(1) | 6(1) | -2(1) |
| C(12) | 25(2) | 23(2) | 26(2) | -2(1) | 6(1) | -1(2) |
| N(13) | 24(1) | 40(2) | 46(2) | 7(2) | 9(1) | -2(2) |
| C(13) | 28(2) | 27(2) | 30(2) | 7(2) | 2(1) | -1(2) |
| N(14) | 22(1) | 26(2) | 29(2) | 4(1) | 5(1) | 1(1) |
| C(14) | 24(2) | 23(2) | 23(2) | -1(2) | 3(1) | 2(2) |
| N(15) | 23(1) | 28(2) | 32(2) | 3(1) | 7(1) | 0(1) |
| C(15) | 27(2) | 27(2) | 23(2) | 1(2) | 6(1) | 2(2) |
| N(16) | 30(1) | 26(2) | 26(2) | 3(1) | 3(1) | 2(1) |
| C(16) | 29(2) | 31(2) | 33(2) | 7(2) | 7(1) | 2(2) |
| N(17) | 46(2) | 29(2) | 34(2) | 7(1) | 4(1) | -5(2) |
| C(17) | 30(2) | 34(2) | 48(2) | 7(2) | 13(2) | -3(2) |
| N(18) | 51(2) | 30(2) | 36(2) | 6(2) | 0(1) | 0(2) |
| C(18) | 28(2) | 28(2) | 31(2) | 6(2) | 4(1) | 4(2) |
| C(19) | 28(2) | 24(2) | 24(2) | 1(2) | 4(1) | 3(2) |
| C(20) | 36(2) | 26(2) | 30(2) | 5(2) | 2(2) | -3(2) |
| C(21) | 33(2) | 23(2) | 26(2) | -1(2) | 3(1) | 0(2) |
| C(22) | 39(2) | 31(2) | 29(2) | 4(2) | -1(2) | 4(2) |
| C(23) | 65(2) | 36(2) | 48(2) | 4(2) | 20(2) | -14(2) |
| C(24) | 27(1) | 34(2) | 43(2) | 1(1) | 7(1) | 3(1) |
| C(25) | 30(2) | 37(2) | 33(2) | 7(2) | 9(1) | 4(2) |
| C(26) | 28(2) | 24(2) | 31(2) | 4(1) | 5(2) | 2(2) |
| C(27) | 39(2) | 39(2) | 28(2) | 4(2) | 7(2) | 9(2) |
| C(28) | 29(2) | 28(2) | 41(2) | 4(2) | 3(2) | 1(2) |
| C(29) | 20(2) | 29(2) | 31(2) | 4(2) | 3(1) | 0(2) |
| C(30) | 21(2) | 29(2) | 30(2) | 7(2) | 1(1) | 3(2) |
| C(3 1) | 33(2) | 50(2) | 48(2) | 3(2) | 5(2) | 12(2) |
| C(32) | 27(2) | 43(2) | 31(2) | 10(2) | 6(2) | 3(2) |
| C(33) | 27(2) | 51(3) | 34(2) | 4(2) | 12(2) | 5(2) |
| C(34) | 22(1) | 28(2) | 32(2) | 5(2) | 5(1) | 0(2) |
| C(35) | 25(2) | 24(2) | 25(2) | -1(1) | 3(1) | 2(2) |
| C(36) | 29(2) | 27(2) | 34(2) | 8(2) | 4(1) | -2(2) |
| C(37) | 32(2) | 36(2) | 42(2) | 9(2) | 10(2) | -3(2) |
| C(3 8) | 32(2) | 28(2) | 35(2) | 5(2) | 9(1) | -1(2) |
| C(39) | 25(2) | 25(2) | 25(2) | 1(2) | 4(1) | 5(2) |
| C(40) | 29(2) | 24(2) | 22(2) | -2(2) | 7(1) | 0(2) |
| C(41) | 30(2) | 26(2) | 24(2) | 1(2) | 2(1) | 0(2) |
| C(42) | 28(2) | 26(2) | 33(2) | 5(2) | 5(1) | 6(2) |
| C(43) | 37(2) | 32(2) | 36(2) | 5(2) | 5(2) | 0(2) |
| C(44) | 34(2) | 27(2) | 24(2) | 3(2) | 1(1) | 0(2) |
| C(45) | 38(2) | 26(2) | 35(2) | 4(2) | 0(2) | -3(2) |
| C(46) | 63(2) | 40(2) | 48(2) | 7(2) | 12(2) | -18(2) |
| N(2) | 58(2) | 42(2) | 58(2) | -4(2) | 31(2) | 0(2) |
| C(2) | 29(2) | 24(2) | 32(2) | 5(2) | 2(1) | 4(2) |
| N(3) | 22(1) | 22(1) | 28(2) | 3(1) | 4(1) | 0(1) |
| C(3) | 24(2) | 23(2) | 29(2) | 2(1) | 3(1) | 1(2) |

**Table 5: List of hydrogen bonds**

| | x | y | z | U(eq) |
|---|---|---|---|---|
| H(4A) | 5290 | -1413 | 435 | 37 |
| H(4B) | 4450 | -2558 | 34 | 37 |
| H(5A) | 4440 | 1860 | 588 | 34 |
| H(5B) | 4573 | 1747 | 1443 | 34 |
| H(7A) | 3044 | -1897 | 1015 | 37 |
| H(7B) | 3105 | -1320 | 214 | 37 |
| H(9A) | 2455 | 974 | 1559 | 37 |
| H(9B) | 3397 | 780 | 1992 | 37 |
| H(1) | 4525 | -4792 | 951 | 38 |
| H(11) | 1615 | 1675 | 244 | 33 |
| H(13) | 2753 | 5861 | -645 | 35 |
| H(16) | 257 | 1391 | -234 | 37 |
| H(17) | -1284 | 1629 | -659 | 44 |
| H(18) | -790 | 7476 | -1740 | 35 |
| H(20) | 1916 | 9583 | -1590 | 38 |
| H(22) | -538 | 10653 | -2167 | 41 |
| H(23A) | 2414 | 12905 | -1983 | 73 |
| H(23B) | 1716 | 14563 | -2173 | 73 |
| H(23C) | 1874 | 13097 | -2771 | 73 |
| H(24) | -835 | 10869 | 3730 | 42 |
| H(25A) | 101 | 11100 | 2980 | 40 |
| H(25B) | 499 | 13144 | 3335 | 40 |
| H(27A) | 445 | 12923 | 4745 | 43 |
| H(27B) | 30 | 10810 | 4852 | 43 |
| H(28A) | 1932 | 10935 | 4775 | 40 |
| H(28B) | 1980 | 11498 | 3970 | 40 |
| H(29A) | 550 | 7805 | 4394 | 33 |
| H(29B) | 421 | 7972 | 3540 | 33 |
| H(32A) | 1616 | 8830 | 3002 | 41 |
| H(32B) | 2552 | 8607 | 3446 | 41 |
| H(34) | 3371 | 7879 | 4747 | 33 |
| H(36) | 2206 | 3795 | 5649 | 37 |
| H(37) | 6252 | 7958 | 5639 | 44 |
| H(38) | 4717 | 8228 | 5235 | 38 |
| H(42) | 5769 | 2122 | 6720 | 36 |
| H(43) | 3067 | 40 | 6595 | 43 |
| H(45) | 5516 | -1001 | 7189 | 41 |
| H(46A) | 2568 | -3010 | 7156 | 76 |
| H(46B) | 3253 | -3812 | 7809 | 76 |
| H(46C) | 3143 | -4822 | 7052 | 76 |
| H(2A) | 4345 | -2792 | 1876 | 35 |
| H(2B) | 5209 | -1602 | 1892 | 35 |

**Table 6: Torsion angle list**

| | | | |
|---|---|---|---|
| C(17)-N(5)-N(6)-C(18) | -179.6(3) | C(34)-N(14)-C(30)-C(29) | -170.8(3) |
| C(17)-N(5)-N(6)-C(15) | -0.3(4) | N(12)-N(14)-C(30)-C(29) | 13.5(4) |
| C(3)-C(5)-C(6)-N(3) | -129.1(3) | C(34)-N(14)-C(30)-C(28) | -68.6(4) |
| C(3)-C(5)-C(6)-C(9) | 103.0(3) | N(12)-N(14)-C(30)-C(28) | 115.7(3) |
| C(3)-C(5)-C(6)-C(7) | -11.1(3) | C(26)-C(29)-C(30)-N(14) | 127.8(3) |
| N(3)-C(6)-C(7)-C(3) | 127.7(3) | C(26)-C(29)-C(30)-C(32) | -105.0(4) |
| C(9)-C(6)-C(7)-C(3) | -105.3(3) | C(26)-C(29)-C(30)-C(28) | 9.8(3) |
| C(5)-C(6)-C(7)-C(3) | 11.0(3) | C(26)-C(28)-C(30)-N(14) | -126.6(3) |
| C(22)-N(8)-N(9)-C(20) | 0.3(4) | C(26)-C(28)-C(30)-C(32) | 107.4(3) |
| C(22)-N(8)-N(9)-C(23) | 177.6(3) | C(26)-C(28)-C(30)-C(29) | -9.9(3) |
| N(3)-C(6)-C(9)-C(10) | -50.5(4) | N(14)-C(30)-C(32)-C(33) | 50.2(4) |
| C(5)-C(6)-C(9)-C(10) | 79.6(4) | C(29)-C(30)-C(32)-C(33) | -79.3(4) |
| C(7)-C(6)-C(9)-C(10) | 179.3(3) | C(28)-C(30)-C(32)-C(33) | 179.5(3) |
| C(3)-C(4)-C(1)-C(8) | -140.9(3) | N(12)-N(14)-C(34)-C(35) | -0.2(4) |
| C(3)-C(4)-C(1)-C(2) | -18.9(2) | C(30)-N(14)-C(34)-C(35) | -176.2(3) |
| N(3)-C(11)-C(12)-C(13) | 0.4(3) | N(14)-C(34)-C(35)-C(36) | 0.3(4) |
| N(3)-C(11)-C(12)-C(14) | -175.3(3) | N(14)-C(34)-C(35)-C(40) | 175.4(3) |
| N(3)-N(4)-C(13)-C(12) | 0.4(4) | N(14)-N(12)-C(36)-C(35) | 0.2(4) |
| C(11)-C(12)-C(13)-N(4) | -0.5(4) | C(34)-C(35)-C(36)-N(12) | -0.3(4) |
| C(14)-C(12)-C(13)-N(4) | 175.6(3) | C(40)-C(35)-C(36)-N(12) | -175.8(3) |
| C(36)-N(12)-N(14)-C(34) | 0.0(4) | N(15)-N(13)-C(37)-C(38) | 0.2(4) |
| C(36)-N(12)-N(14)-C(30) | 176.4(3) | N(13)-C(37)-C(38)-C(39) | -0.2(4) |
| C(19)-N(7)-C(14)-C(15) | 1.2(5) | N(13)-N(15)-C(39)-C(38) | -0.1(4) |
| C(19)-N(7)-C(14)-C(12) | -177.2(3) | C(42)-N(15)-C(39)-C(38) | -179.4(3) |
| C(11)-C(12)-C(14)-N(7) | 169.5(3) | N(13)-N(15)-C(39)-C(40) | 179.6(3) |
| C(13)-C(12)-C(14)-N(7) | -5.4(5) | C(42)-N(15)-C(39)-C(40) | 0.4(5) |
| C(11)-C(12)-C(14)-C(15) | -8.8(6) | C(37)-C(38)-C(39)-N(15) | 0.2(4) |
| C(13)-C(12)-C(14)-C(15) | 176.3(3) | C(37)-C(38)-C(39)-C(40) | -179.5(4) |
| C(37)-N(13)-N(15)-C(42) | 179.2(3) | C(41)-N(16)-C(40)-C(39) | -0.6(5) |
| C(37)-N(13)-N(15)-C(39) | 0.0(4) | C(41)-N(16)-C(40)-C(35) | 176.7(3) |
| N(5)-N(6)-C(15)-C(16) | 0.2(4) | N(15)-C(39)-C(40)-N(16) | 0.3(5) |
| C(18)-N(6)-C(15)-C(16) | 179.5(3) | C(38)-C(39)-C(40)-N(16) | 180.0(4) |
| N(5)-N(6)-C(15)-C(14) | -179.5(3) | N(15)-C(39)-C(40)-C(35) | -176.8(3) |
| C(18)-N(6)-C(15)-C(14) | -0.1(5) | C(38)-C(39)-C(40)-C(35) | 2.9(6) |
| N(7)-C(14)-C(15)-N(6) | -0.5(5) | C(34)-C(35)-C(40)-N(16) | -168.0(3) |
| C(12)-C(14)-C(15)-N(6) | 177.7(3) | C(36)-C(35)-C(40)-N(16) | 6.2(5) |
| N(7)-C(14)-C(15)-C(16) | -179.9(4) | C(34)-C(35)-C(40)-C(39) | 9.3(5) |
| C(12)-C(14)-C(15)-C(16) | -1.7(6) | C(36)-C(35)-C(40)-C(39) | -176.5(3) |
| N(6)-C(15)-C(16)-C(17) | 0.0(4) | C(40)-N(16)-C(41)-C(42) | 0.1(5) |
| C(14)-C(15)-C(16)-C(17) | 179.5(4) | C(40)-N(16)-C(41)-C(44) | -178.6(3) |
| N(6)-N(5)-C(17)-C(16) | 0.3(4) | N(16)-C(41)-C(42)-N(15) | 0.6(5) |
| C(15)-C(16)-C(17)-N(5) | -0.2(4) | C(44)-C(41)-C(42)-N(15) | 179.2(3) |
| C(43)-N(17)-N(18)-C(45) | 0.2(4) | N(13)-N(15)-C(42)-C(41) | -180.0(3) |
| C(46)-N(17)-N(18)-C(45) | -177.3(3) | C(39)-N(15)-C(42)-C(41) | -0.8(5) |
| N(5)-N(6)-C(18)-C(19) | 179.3(3) | N(18)-N(17)-C(43)-C(44) | -0.2(4) |
| C(15)-N(6)-C(18)-C(19) | 0.0(5) | C(46)-N(17)-C(43)-C(44) | 177.1(3) |
| N(6)-C(18)-C(19)-N(7) | 0.7(5) | N(17)-C(43)-C(44)-C(45) | 0.1(4) |
| N(6)-C(18)-C(19)-C(21) | -178.8(3) | N(17)-C(43)-C(44)-C(41) | 178.7(3) |
| C(14)-N(7)-C(19)-C(18) | -1.3(5) | C(42)-C(41)-C(44)-C(43) | 177.4(4) |
| C(14)-N(7)-C(19)-C(21) | 178.3(3) | N(16)-C(41)-C(44)-C(43) | -3.9(5) |
| N(8)-N(9)-C(20)-C(21) | 0.0(4) | C(42)-C(41)-C(44)-C(45) | -4.4(6) |
| C(23)-N(9)-C(20)-C(21) | -177.1(3) | N(16)-C(41)-C(44)-C(45) | 174.3(3) |
| N(9)-C(20)-C(21)-C(22) | -0.2(4) | N(17)-N(18)-C(45)-C(44) | -0.1(4) |
| N(9)-C(20)-C(21)-C(19) | -178.0(3) | C(43)-C(44)-C(45)-N(18) | 0.0(4) |
| C(18)-C(19)-C(21)-C(20) | -176.1(3) | C(41)-C(44)-C(45)-N(18) | -178.5(3) |
| N(7)-C(19)-C(21)-C(20) | 4.3(5) | C(8)-C(1)-C(2)-C(3) | 138.9(3) |
| C(18)-C(19)-C(21)-C(22) | 6.6(5) | C(4)-C(1)-C(2)-C(3) | 18.7(2) |
| N(7)-C(19)-C(21)-C(22) | -172.9(3) | C(12)-C(11)-N(3)-N(4) | -0.2(4) |
| N(9)-N(8)-C(22)-C(21) | -0.4(4) | C(12)-C(11)-N(3)-C(6) | 175.3(3) |
| C(20)-C(21)-C(22)-N(8) | 0.4(4) | C(13)-N(4)-N(3)-C(11) | -0.1(4) |
| C(19)-C(21)-C(22)-N(8) | 178.2(3) | C(13)-N(4)-N(3)-C(6) | -176.0(3) |
| C(31)-C(24)-C(25)-C(26) | 143.5(3) | C(9)-C(6)-N(3)-C(11) | -59.1(4) |
| C(27)-C(24)-C(25)-C(26) | 22.5(3) | C(5)-C(6)-N(3)-C(11) | 170.7(3) |
| C(24)-C(25)-C(26)-C(27) | -22.7(3) | C(7)-C(6)-N(3)-C(11) | 69.1(4) |
| C(24)-C(25)-C(26)-C(28) | -152.7(3) | C(9)-C(6)-N(3)-N(4) | 116.0(3) |
| C(24)-C(25)-C(26)-C(29) | 98.4(3) | C(5)-C(6)-N(3)-N(4) | -14.2(4) |
| C(28)-C(26)-C(27)-C(24) | 153.5(3) | C(7)-C(6)-N(3)-N(4) | -115.7(3) |
| C(25)-C(26)-C(27)-C(24) | 22.9(3) | C(1)-C(4)-C(3)-C(5) | 145.4(3) |
| C(29)-C(26)-C(27)-C(24) | -96.9(3) | C(1)-C(4)-C(3)-C(7) | -105.0(3) |
| C(31)-C(24)-C(27)-C(26) | -144.9(3) | C(1)-C(4)-C(3)-C(2) | 18.9(2) |
| C(25)-C(24)-C(27)-C(26) | -22.7(3) | C(6)-C(5)-C(3)-C(4) | 135.4(3) |
| C(27)-C(26)-C(28)-C(30) | 133.7(4) | C(6)-C(5)-C(3)-C(7) | 11.2(3) |
| C(25)-C(26)-C(28)-C(30) | -112.6(4) | C(6)-C(5)-C(3)-C(2) | -114.4(3) |
| C(29)-C(26)-C(28)-C(30) | 9.8(3) | C(6)-C(7)-C(3)-C(4) | -137.9(3) |
| C(27)-C(26)-C(29)-C(30) | -137.8(3) | C(6)-C(7)-C(3)-C(5) | -11.1(3) |
| C(28)-C(26)-C(29)-C(30) | -9.8(3) | C(6)-C(7)-C(3)-C(2) | 115.3(3) |
| C(25)-C(26)-C(29)-C(30) | 118.6(3) | C(1)-C(2)-C(3)-C(4) | -19.0(2) |
| C(34)-N(14)-C(30)-C(32) | 59.0(4) | C(1)-C(2)-C(3)-C(5) | -146.1(3) |
| N(12)-N(14)-C(30)-C(32) | -116.8(3) | C(1)-C(2)-C(3)-C(7) | 103.8(3) |

### Example 3

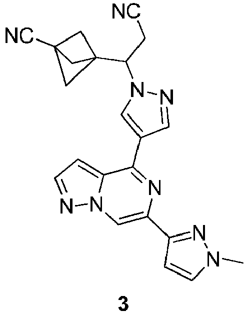

### Synthetic route:

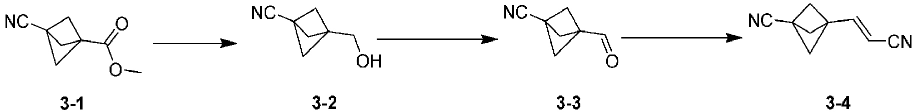

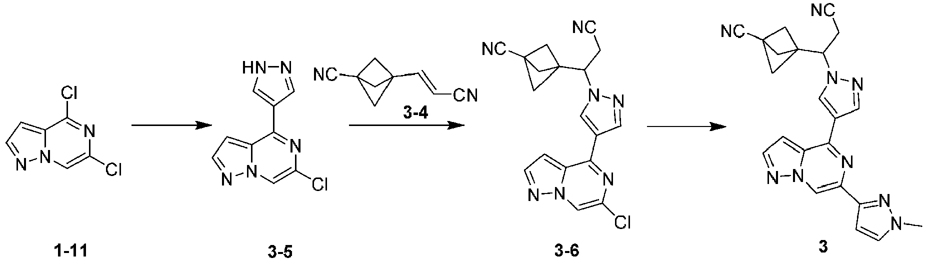

### Step 1: Synthesis of compound 3-2

Compound **3-1** (1.03 g, 6.81 mmol) was dissolved in THF (25 mL), a solution of lithium borohydride in tetrahydrofuran (2 M, 3.41 mL) was added dropwise at 0 °C, and then the mixture was stirred at 15 °C for 16 h. The reaction mixture was quenched by adding 10 mL of 1M hydrochloric acid, then the tetrahydrofuran was removed under reduced pressure, the residue was diluted with 30 mL of H₂O, and extracted with 40 mL of ethyl acetate (20 mL × 2), and the combined organic layer was washed with 20 mL of brine (20 mL), dried, filtered, concentrated under reduced pressure, and purified by column chromatography (petroleum ether : ethyl acetate = 2 : 1) to obtain compound **3-2.** ¹HNMR (400 MHz, CDCl₃) δ 3.60 (s, 2H), 2.20 (s, 6H).

### Step 2: Synthesis of compound 3-3

10 mL of DCM and oxalyl chloride (896.66 mg, 7.06 mmol, 618.39 µL) were added into a 50 mL round-bottomed flask, and the mixture was cooled to -65 °C. At -65 °C, dimethyl sulfoxide (1.10 g, 14.13 mmol, 1.10 mL) was added dropwise within 10 min, the mixture was stirred for 10 min, compound **3-2** (580 mg, 4.71 mmol) was dissolved in 5 ml of dichloromethane and the obtained mixture was added dropwise to the reaction system at -65 °C. The mixture was reacted under stirring at the same temperature for 1 h and TEA (2.38 g, 23.55 mmol, 3.28 mL) was added within 10 min at -65 °C. The reaction mixture was diluted with water and extracted with dichloromethane. The combined organic layers were washed with 1M aqueous hydrochloric acid solution and brine, dried and concentrated to obtain compound **3-3.** ¹HNMR (400MHz, CDCl₃) δ 9.52 (s, 1H), 2.52 (s, 6H).

### Step 3: Synthesis of compound 3-4

Diethyl cyanomethylphosphate (754.56 mg, 4.26 mmol, 685.96 µL), lithium bromide (462.44 mg, 5.32 mmol, 133.65 µL) and triethylamine (718.39 mg, 7.10 mmol, 988.16 µL) were added to THF (6 mL) to form a system, compound **3-3** (430 mg, 3.55 mmol) was dissolved in THF (4 mL), added to the system, and stirred at 15 °C for 16 h. The mixture was diluted with water and extracted with ethyl acetate (20 mL × 1). The combined organic layers were washed with 20 mL of brine (20 mL × 1), dried and concentrated, and purified by column chromatography (petroleum ether : ethyl acetate = 3 : 1) to obtain compound **3-4.** ¹HNMR (400 MHz, CDCl₃) δ 6.64 (d, *J* = 16.4 Hz, 1H), 5.32 (d, *J* = 16.4 Hz, 1H), 2.40 (s, 6H).

### Step 4: Synthesis of compound 3-5

Compound **1-11** (5.5 g, 29.25 mmol), 4-pyrazolepinacolborate (6.81 g, 35.10 mmol), Pd(PPh₃)₄ (3.38 g, 2.93 mmol), and sodium carbonate (6.20 g, 58.51 mmol) were added to dioxane (60 mL) and water (15 mL), and the mixture was stirred at 50 °C for 16 h under a nitrogen atmosphere. The obtained product was diluted with water, the mother liquor was extracted with EA 120 mL (60 mL × 2), the combined organic layers was washed with 60 mL of brine (60 mL × 1), dried and concentrated, and purified by column chromatography (petroleum ether : ethyl acetate = 0 : 1) to obtain compound **3-5.** ¹HNMR (400 MHz, DMSO-d₆) δ 13.55 (brs, 1H), 8.96 (s, 1H), 8.73 (s, 1H), 8.33 (s, 1H), 8.25 (s, 1H), 7.50 (dd, *J* = 1.2, 2.4 Hz, 1H).

### Step 5: Synthesis of compound 3-6

**3-5** (150 mg, 682.97 µmol) was dissolved in acetonitrile (4 mL), DBU (103.97 mg, 682.97 µmol) and **3-4** (108.31 mg, 751.27 µmol) were added, and the mixture was stirred at 15 °C for 1 h. The obtained product was concentrated under reduced pressure and purified by column chromatography (petroleum ether : ethyl acetate = 1 : 1) to obtain compound **3-6.** ¹HNMR (400 MHz, CDCl₃) δ 8.39 (d, *J* = 0.8 Hz, 1H), 8.27 (s, 1H), 8.22 (s, 1H), 8.09 (d, *J* = 2.4 Hz, 1H), 6.98 (dd, *J* = 0.8, 2.4 Hz, 1H), 4.63 (dd, *J* = 6.4, 8.0 Hz, 1H), 3.08-3.20 (m, 1H), 2.93-3.03 (m, 1H), 2.38-2.24 (m, 6H).

### Step 6: Synthesis of the trifluoroacetate salt of compound 3

Compound **3-6** (180 mg, 494.77 µmol), 1-methylpyrazole-3-pinacol borate (113.24 mg, 544.25 µmol), Xphos-Pd-G2 (77.86 mg, 98.95 µmol) and sodium carbonate (157.32 mg, 1.48 mmol) were added to water (1 mL) and dioxane (4 mL), and the mixture was stirred at 100 °C for 16 h under nitrogen. The reaction mixture was concentrated to dryness under reduced pressure, diluted with 20 mL of water, and extracted with ethyl acetate (15 mL × 2). The combined organic layers were washed with brine (20 mL), dried and concentrated, and purified by column chromatography (petroleum ether : ethyl acetate = 0 : 1) and high performance liquid chromatography (column model: Phenomenex Synergi C18 150 mm*25 mm*10 µm; mobile phase: [water (0.1% trifluoroacetic acid)-acetonitrile]; B (acetonitrile)%: 38%-58%, 10 min) to obtain compound **3.** ¹HNMR (400 MHz, DMSO-d₆) δ 8.86 (s, 1 H), 8.84 (s, 1H), 8.47 (s, 1H), 8.24 (d, *J* = 2.4 Hz, 1H), 7.82 (d, *J* = 2.4 Hz, 1H), 7.38 (d, *J* = 1.6 Hz, 1H), 6.94 (d, *J* = 2.0 Hz, 1H), 4.99 (dd, *J* = 5.6, 9.2 Hz, 1H), 3.94 (s, 3H), 3.39-3.20 (m, 2H), 2.28-2.12 (m, 6H). MS ESI calculated for C₂₂Ht₉N₉ [M + H]⁺ 410, found 410.

### Examples 4 and 5

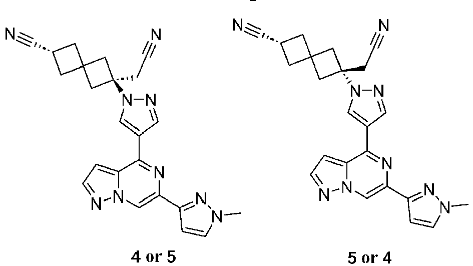

### Synthetic route:

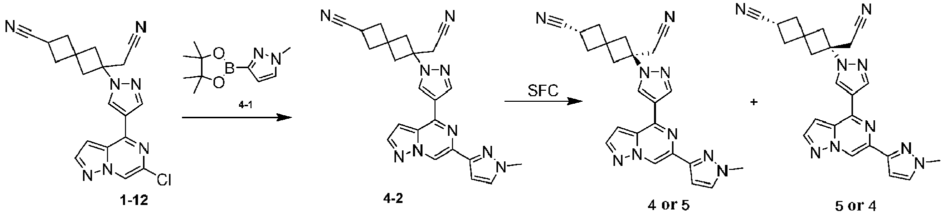

### Step 1: Synthesis of compound 4-2

Under nitrogen protection at 25 °C, dioxane (8 mL) and water (2 mL) were added to a mixture of compound **1-12** (0.36 g, 952.81 µmol), compound **4-1** (218.07 mg, 1.05 mmol), BrettPhos Pd G3 (149.93 mg, 190.56 µmol) and Na₂CO₃ (302.96 mg, 2.86 mmol). Vacuumization under reduced pressure and nitrogen filling was performed three times for the resulting mixture, and then the mixture was heated to 100 °C and stirred for 16 h. After the reaction was completed, the reaction liquid is diluted with water (5 mL), the aqueous phase was extracted with ethyl acetate (20 mL × 3), the combined organic phases were dried over anhydrous sodium sulfate, and filtered, and the filtrate was concentrated, and the crude product obtained was purified by silica gel column chromatography (petroleum ether : ethyl acetate = 3 : 1 to 2 : 1) to obtain compound **4-2.** MS ESI calculated for C₂₃H₂₁N₉ [M + H]⁺ 424, found 424.

### Step 2: Synthesis of compounds 4 and 5

Compound **4-2** was subjected to chiral resolution (chiral column model: DAICEL CHIRALPAK AD (250 mm*30 mm*10 µm); mobile phase: A: carbon dioxide, B: 0.1% ammonia/methanol, 0.1% ammonia/methanol and supercritical fluid carbon dioxide from 60% to 60%, flow rate at 70 mL/min), compound **4** and compound **5** were obtained after resolution, (analysis method: column model Amycoat 50 × 4.6 mm I.D., 3 µm; mobile phase: A: CO₂, B: methanol + acetonitrile (0.05% diethylamine); gradient: 60% methanol + acetonitrile (0.05% diethylamine); flow rate at 3 mL/min; column temperature 35 °C). The retention times were 1.045 min and 1.375 min respectively.

Compound **4:** ¹H NMR (400 MHz, CDCl₃) δ 8.89 (s, 1H), 8.36 (s, 1H), 8.31 (s, 1H), 8.09 (d, *J* = 2.4 Hz, 1H), 7.46 (d, *J* = 2.2 Hz, 1H), 6.96 (d, *J* = 1.8 Hz, 1H), 6.88 (d, *J* = 2.2 Hz, 1H), 4.01 (s, 3H), 3.07-3.15 (m, 1H), 2.98-3.07 (m, 4H), 2.79 (dt, *J* = 2.3, 13.8 Hz, 2H), 2.61-2.72 (m, 2H), 2.43-2.55 (m, 2H). MS ESI calculated for C₂₃H₂₁N₉ [M + H]⁺ 424, found 424.

Compound **5:** ¹H NMR (400 MHz, CDCl₃) δ 8.90 (s, 1H), 8.36 (s, 1H), 8.31 (s, 1H), 8.09 (d, *J* = 2.3 Hz, 1H), 7.46 (d, *J* = 2.2 Hz, 1H), 6.96 (d, *J* = 1.6 Hz, 1H), 6.89 (d, *J* = 2.2 Hz, 1H), 4.02 (s, 3H), 3.08-3.17 (m, 1H), 2.99-3.07 (m, 4H), 2.79 (dt, *J* = 2.2, 13.7 Hz, 2H), 2.60-2.72 (m, 2H), 2.44-2.56 (m, 2H). MS ESI calculated for C₂₃H₂₁N₉ [M + H]⁺ 424, found 424.

### Example 6

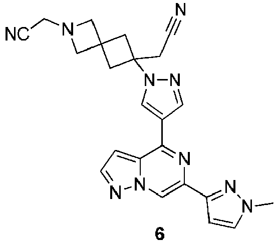

### Synthetic route:

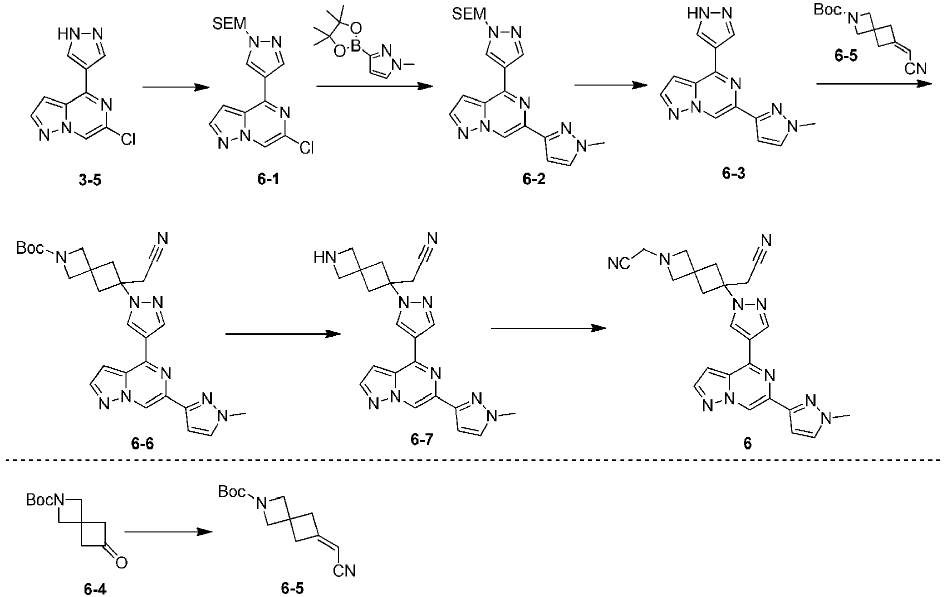

### Step 1: Synthesis of compound 6-1

Compound **3-5** (1.19 g, 5.42 mmol) was dissolved in anhydrous THF (18 mL), and sodium hydride (325.06 mg, 8.13 mmol, 60% purity) was added in portions at 0 °C, and the mixture was stirred at 0 °C for 0.5 h. 2-(trimethylsilyl)ethoxymethyl chloride (993.66 mg, 5.96 mmol) was added dropwise at 0 °C, and the mixture was warmed to 20 °C and stirred for 0.5 h. The reaction mixture was diluted with 60 mL of water, and extracted with ethyl acetate (30 mL × 2). The combined organic layers were washed with brine (30 mL × 1), dried over sodium sulfate, filtered, concentrated under reduced pressure, and purified by column chromatography (petroleum ether : ethyl acetate = 5 : 1) to obtain compound **6-1.** ¹H NMR (400 MHz, CDCl₃) δ 8.38 (d, *J* = 0.8 Hz, 1H), 8.36 (s, 1H), 8.27 (s, 1H), 8.05-8.11 (d, *J* =2.4 Hz, 1H), 7.01 (dd, *J* = 0.8, 2.4 Hz, 1H), 5.54 (s, 2H), 3.64 (d, *J* = 6.8 Hz, 2H), 0.96 (s, 2H), 0.03 (s, 9H).

### Step 2: Synthesis of compound 6-2

Compound **6-1** (1.65 g, 4.72 mmol), 1-methylpyrazole-3-pinacol borate (1.08 g, 5.19 mmol), X-Phos Pd G2 (742.08 mg, 943.15 µmol) and sodium carbonate (1.50 g, 14.15 mmol) were added to H₂O (6 mL) and dioxane (25 mL), and the reaction was carried out at 100 °C for 16 h under a nitrogen atmosphere. The reaction liquid was diluted with 80 mL of water and filtered. The mother liquor was extracted with ethyl acetate (50 mL × 2). The combined organic layers were washed with brine (60 mL × 1), dried over sodium sulfate, filtered, concentrated under reduced pressure, and purified by column chromatography (petroleum ether : ethyl acetate=1 : 2) to obtain compound **6-2.** ¹H NMR (400 MHz, CDCl₃) δ 8.91 (d, *J* = 0.8 Hz, 1H), 8.38 (s, 1H), 8.32 (s, 1H), 8.04-8.13 (m, 1H), 7.45 (d, *J* = 2.4 Hz, 1H), 6.96 (dd, *J* = 0.8, 2.4 Hz, 1H), 6.91 (d, *J*= 2.4 Hz, 1H), 5.55 (s, 2H), 4.01 (s, 3H), 3.63-3.70 (m, 2H), 0.93-1.00 (m, 2H), 0.00 (s, 9H).

### Step 3: Synthesis of compound 6-3

Compound **6-2** (1.76 g, 4.45 mmol) was dissolved in methanol hydrochloride (4M, 25 mL), the mixture was reacted at 50 °C for 16 h, concentrated under reduced pressure, and diluted with methyl tert-butyl ether (20 mL), the mixture was stirred at 15 °C for 30 min, filtered, and dried under vacuum to obtain compound **6-3.** MS ESI calculated for C₁₃H₁₁N₇ [M + H]⁺ 266, found 266.

### Step 4: Synthesis of compound 6-5

Compound **6-4** (0.5 g, 2.37 mmol) was dissolved in tetrahydrofuran (5 mL), and a solution of diethyl cyanomethylphosphonate (503.09 mg, 2.84 mmol), lithium bromide (308.31 mg, 3.55 mmol) and triethylamine (478.99 mg, 4.73 mmol, 658.86 µL) in tetrahydrofuran (10 mL) was added, and the mixture was reacted at 15 °C for 2 h. The reaction liquid was diluted with 50 mL of water, and extracted with ethyl acetate (30 mL × 2). The combined organic layers were washed with brine (30 mL × 1), dried over sodium sulfate, filtered, concentrated under reduced pressure, and purified by column chromatography (petroleum ether : ethyl acetate = 3 : 1) to obtain compound **6-5.** ¹H NMR (400 MHz, CDCl₃) δ 5.13-5.27 (m, 1H), 3.92-4.03 (m, 4H), 3.10-3.18 (m, 2H), 3.02-3.07 (m, 2H), 1.43 (s, 9H).

### Step 5: Synthesis of compound 6-6

Compound **6-3** (0.3 g, 994.25 µmol) was dissolved in acetonitrile (6 mL), and 1,8-diazabicyclo[5.4.0]undecan-7-ene (454.10 mg, 2.98 mmol, 449.60 µL) and compound 6-5 (256.24 mg, 1.09 mmol) were added, and the mixture was reacted at 15 °C for 16 h. The obtained product was concentrated under reduced pressure and purified by column chromatography (petroleum ether/ethyl acetate = 1/3) to obtain compound **6-6.** ¹H NMR (400 MHz, CDCl₃) δ 8.88 (d, *J* = 0.8 Hz, 1H), 8.36 (s, 1H), 8.30 (s, 1H), 8.08 (d, *J* = 2.4 Hz, 1H), 7.45 (d, *J* = 2.4 Hz, 1H), 6.95 (dd, *J* = 0.8, 2.4 Hz, 1H), 6.88 (d, *J* = 2.4 Hz, 1H), 4.13 (s, 2H), 4.00 (s, 3H), 3.94 (s, 2H), 3.11 (s, 1H), 3.08 (s, 1H), 3.02 (s, 2H), 2.86 (s, 1H), 2.83 (s, 1H), 1.44 (s, 9H).

### Step 6: Synthesis of compound 6-7

Compound **6-6** was dissolved in dichloromethane (1 mL), trifluoroacetic acid (308.00 mg, 2.70 mmol, 0.2 mL) was added, and the mixture was reacted at 15 °C for 0.5 h. The reaction liquid was adjusted to pH 7 with 25% ammonia water, concentrated to dryness under reduced pressure, and purified by high performance liquid chromatography (column: Waters Xbridge 150 × 25 mm × 5 µm; mobile phase: [water (10 mM sodium bicarbonate)-acetonitrile]; B (acetonitrile)%: 8%-38%, 10 min) to obtain compound **6-7.** ¹H NMR (400 MHz, CD₃OD) δ 8.82 (s, 1H), 8.72 (s, 1H), 8.41 (s, 1H), 8.10 (d, *J* = 1.6 Hz, 1H), 7.64 (d, *J* = 1.6 Hz, 1H), 7.23 (d, *J* = 2.4 Hz, 1H), 6.90 (d, *J=* 2.4 Hz, 1H), 4.02 (s, 2H), 3.97 (s, 3H), 3.86 (s, 2H), 3.24 (s, 2H), 3.06-3.20 (m, 2H), 2.73-2.96 (m, 2H). MS ESI calculated for C₂₁H₂₁N₉ [M + H]⁺ 400, found 400.

### Step 7: Synthesis of compound 6

Compound **6-7** (70 mg, 140.12 µmol) was dissolved in dichloromethane (1 mL), trifluoroacetic acid (299.44 mg, 2.63 mmol, 194.44 µL) was added, and the mixture was stirred at 15 °C for 0.5 h. Then triethylamine (318.06 mg, 3.14 mmol, 437.50 µL) and bromoacetonitrile (25.21 mg, 210.18 µmol) were added, and the reaction liquid was stirred at 15 °C for 16 h. The obtained product was concentrated to dryness under reduced pressure and purified twice by high performance liquid chromatography, the first purification (column model: Xtimate C18 150*40 mm*10 µm; mobile phase: [water (0.05% ammonia v/v)-ACN]; B(ACN)%: 20%-50%, 10 min), the second purification (column model: WatersXbridge 150*25 mm*5 µm; mobile phase: [water (10 mM sodium bicarbonate)-ACN]; B(ACN)%: 14%-44%, 10 min) to obtain compound **6.** ¹H NMR (400 MHz, CD₃OD) δ 8.85 (s, 1 H), 8.75 (s, 1H), 8.43 (s, 1H), 8.07-8.18 (m, 1H), 7.67 (d, *J=* 2.4 Hz, 1H), 7.21-7.33 (m, 1H), 6.99 - 6.86 (m, 1H), 3.99 (s, 3H), 3.58 (s, 2H), 3.56 (s, 2H), 3.41 (s, 2H), 3.23 (s, 2H), 3.13 (s, 1H), 3.10 (s, 1H), 2.83 (s, 1H), 2.80 (s, 1H). MS ESI calculated for C₂₃H₂₂N₁₀ [M + H]⁺ 439, found 439.

### Example 7

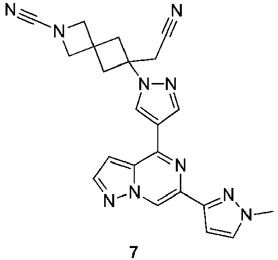

### Synthetic route:

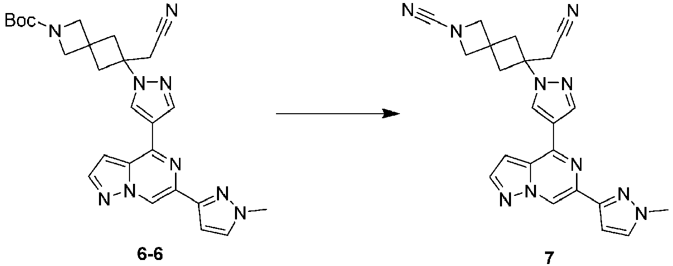

### Synthesis of compound 7

Compound **6-6** (100 mg, 200.17 µmol) was dissolved in dichloromethane (1 mL), trifluoroacetic acid (308.00 mg, 2.70 mmol, 0.2 mL) was added, and the mixture was stirred at 15 °C for 0.5 h. Then triethylamine (324.09 mg, 3.20 mmol, 445.79 µL) was added, and the mixture was cooled to 0 °C, bromonitrile (0.32 g, 3.02 mmol) was added, and the mixture was stirred at 0 °C for 0.5 h. The reaction liquid was diluted with 30 mL of water and extracted with 40 mL of dichloromethane (20 mL × 2). The combined organic layers were washed with 20 mL of brine, dried over sodium sulfate, filtered, concentrated under reduced pressure, and purified by high performance liquid chromatography (column: Waters Xbridge 150 × 25 mm × 5 µm; mobile phase: [water (10 mM sodium bicarbonate)-acetonitrile]; B(ACN)%: 14%-44%, 10 min) to obtain compound **7.** ¹HNMR (400 MHz , CD₃OD) δ 8.88 (d, *J* = 1.2 Hz, 1H), 8.76 (s, 1H), 8.45 (s, 1H), 8.14 (d, *J* = 2.4 Hz, 1H), 7.68 (d, *J* = 2.4 Hz, 1H), 7.28 (dd, *J=* 1.0, 2.4 Hz, 1H), 6.94 (d, *J=* 2.4 Hz, 1H), 4.37 (s, 2H), 4.20 (s, 2H), 4.00 (s, 3H), 3.23 (s, 2H), 3.15-3.22 (m, 2H), 2.85-2.95 (m, 2H). MS ESI calculated for C₂₂H₂₀N₁₀ [M + H]⁺ 425, found 425.

### Example 8

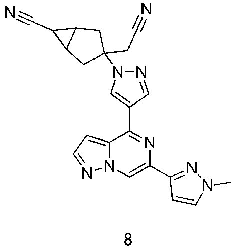

### Synthetic route:

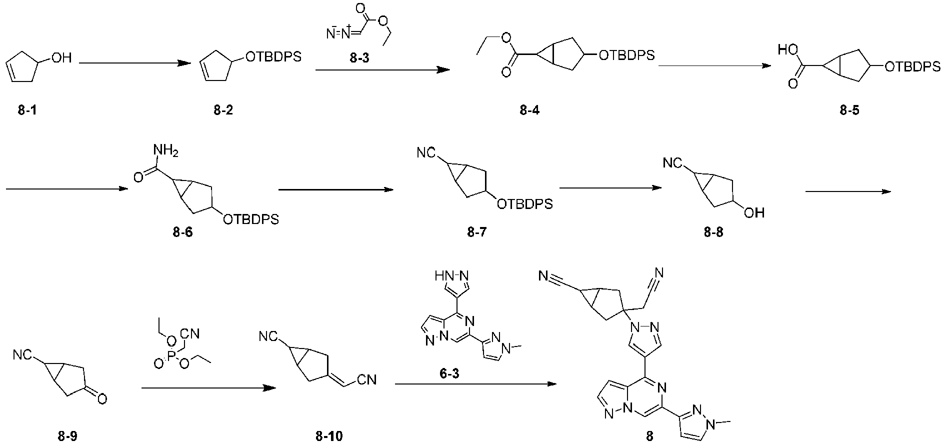

### Step 1: Synthesis of compound 8-2

Compound **8-1** (20 g, 237.77 mmol) and imidazole (32.37g, 475.53 mmol) were added to anhydrous dichloromethane (300 mL), and tert-butyldiphenylchlorosilane (68.62 g, 249.65 mmol, 64.13 mL) was added dropwise at 0 °C, the mixture was stirred at 20 °C for 16 h under nitrogen protection. After the reaction was completed, water (200mL) was added to the reaction liquid, the mixture was extracted with dichloromethane (100 mL × 3), the combined extracts were washed with water (200 mL × 2), and brine (200 mL × 1), dried over anhydrous sodium sulfate, and filtered and the filtrate was concentrated, and the crude product was purified by silica gel column chromatography (petroleum ether : ethyl acetate = 1 : 0) to obtain compound **8-2.** ¹H NMR (400 MHz, CDCl₃) δ 7.65-7.72 (m, 4H), 7.37-7.46 (m, 6H), 5.63 (s, 2H), 4.52-4.62 (m, 1H), 2.36-2.50 (m, 4H), 1.07 (s, 9H).

### Step 2: Synthesis of compound 8-4

Compound **8-2** (66 g, 204.64 mmol) and rhodium acetate (904.48 mg, 4.09 mmol) were added to anhydrous dichloromethane (600 mL), and then a solution of compound **8-3** (28.02 g, 245.57 mmol, 25.71 mL) in dichloromethane (200 mL) was added dropwise over about 5 h. The mixture was stirred at 20 °C for 15 h under nitrogen protection. After the reaction was completed, the reaction liquid was filtered through diatomaceous earth, the filtrate was concentrated, and the crude product was purified by silica gel column chromatography (petroleum ether : ethyl acetate = 1 : 0 to 20 : 1) to obtain compound **8-4.** ¹H NMR (400 MHz, CDCl₃) δ 7.60-7.71 (m, 4H), 7.34-7.47 (m, 6H), 4.19-4.59 (m, 1H), 3.84-4.18 (m, 2H), 2.12-2.49 (m, 1H), 2.00-2.07 (m, 1H), 1.77-1.98 (m, 4H), 1.17-1.55 (m, 3H), 1.09-1.15 (m, 1H), 1.02-1.08 (m, 9H).

### Step 3: Synthesis of compound 8-5

Compound **8-4** (55 g, 134.60 mmol) was added to absolute ethanol (200 mL), and then a solution of sodium hydroxide (16.15 g, 403.81 mmol) in water (50 mL) was added dropwise, the mixture was stirred at 20 °C for 16 h. After the reaction was completed, the reaction liquid was concentrated to remove ethanol, and adjusted to pH 3-4 with 1 M hydrochloric acid, and extracted with ethyl acetate (100 mL × 2), the extracts were combined, and concentrated. The crude product obtained was added to petroleum ether (450 mL), and the mixture was stirred at 20 °C for 1 h. The filter cake was obtained by filtration, and then dried to obtain compound **8-5.** ¹H NMR (400 MHz, DMSO-*d*₆) δ 11.22-12.70 (brs, 1H), 7.52-7.60 (m, 4H), 7.39-7.49 (m, 6H), 3.87-4.33 (m, 1H), 1.92-2.00 (m, 2H), 1.62-1.83 (m, 4H), 1.04 (t, *J* = 2.9 Hz, 1H), 0.85-0.98 (m, 9H).

### Step 4: Synthesis of compound 8-6

Compound **8-5** (30 g, 78.83 mmol) was added to N,N-dimethylformamide (100 mL), and 2-(7-azobenzotriazole)-N,N,N,N-tetramethylurea hexafluorophosphate (44.96 g, 118.25 mmol) and N,N-diisopropylethylamine (30.57 g, 236.50 mmol) were added successively at 15 °C, the mixture was stirred at 25 °C for 0.5 h. Then ammonium chloride (12.65 g, 236.50 mmol) was added in portions at 15 °C and the mixture was stirred at 25 °C for 1 h. After the reaction was completed, the reaction liquid was diluted with water (200 mL), extracted with ethyl acetate (300 mL × 3), the extracts were combined and washed with brine (500 mL × 3), dried over anhydrous sodium sulfate, and filtered, and the filtrate was concentrated to obtain compound **8-6.** ¹H NMR (400 MHz, DMSO-d₆) δ 7.55-7.62 (m, 4H), 7.41-7.51 (m, 6H), 7.21 (brs, 1H), 6.55-6.70 (m, 1H), 3.75-4.20 (m, 1H), 1.92-2.05 (m, 2H), 1.65-1.85 (m, 2H), 1.45-1.60 (m, 1H), 1.05-1.10 (m, 1H), 0.85-1.04 (m, 9H).

### Step 5: Synthesis of compound 8-7

Compound **8-6** (25.00 g, 65.86 mmol) and triethylamine (33.32 g, 329.32 mmol, 45.84 mL) were added to anhydrous dichloromethane (200 mL), and trifluoroacetic anhydride (44.96 g, 118.25 mmol) was added dropwise at 0 °C, and the mixture was stirred at 0 °C for 1 h. After the reaction was completed, the reaction liquid was quenched by adding saturated sodium bicarbonate aqueous solution (100 mL), extracted with dichloromethane (50 mL × 3), the extracts were combined and washed with saturated sodium bicarbonate aqueous solution (100 mL × 3), and dried over anhydrous sodium sulfate, and filtered, and the filtrate was concentrated. The obtained crude product was purified by silica gel column chromatography (petroleum ether : ethyl acetate = 20 : 1 to 10 : 1) to obtain compound **8-7.** ¹H NMR (400 MHz, DMSO-*d*₆) δ 7.54-7.60 (m, 4H), 7.39-7.49 (m, 6H), 3.92-4.06 (m, 1H), 1.95-2.10 (m, 2H), 1.80-1.85 (m, 2H), 1.70-1.82 (m, 2H), 1.39 (t, *J* = 3.4 Hz, 1H), 0.96 (s, 9H).

### Step 6: Synthesis of compound 8-8

Compound **8-7** (22.00 g, 60.85 mmol) was added to tetrahydrofuran (200 mL), and tetrabutylammonium fluoride (1 M solution in THF, 91.27 mL) was added dropwise at 15 °C, and the mixture was stirred at 25 °C for 16 h. After the reaction was completed, the reaction liquid was quenched by adding saturated ammonium chloride aqueous solution (200 mL), extracted with ethyl acetate (200 mL × 3), the extracts were combined and washed with brine (200 mL × 3), dried over anhydrous sodium sulfate, and filtered, and the filtrate was concentrated. The obtained crude product was purified by silica gel column chromatography (petroleum ether : ethyl acetate = 10 : 1 to 1 : 2) to obtain compound **8-8.**

### Step 7: Synthesis of compound 8-9

Compound **8-8** (5.5 g, 44.66 mmol) was added to dichloromethane (200 mL), Dess-Martin oxidant (28.41 g, 66.99 mmol) was added dropwise at 0 °C, and the mixture was stirred at 25 °C for 16 h. After the reaction was completed, the reaction liquid was quenched by adding saturated sodium thiosulfate aqueous solution (200 mL) and saturated sodium bicarbonate aqueous solution (200 mL), the mixture was stirred at 20 °C for 10 min, extracted with dichloromethane (200 mL × 3), the extracts were combined and washed with saturated sodium bicarbonate aqueous solution (100 mL × 3), dried over anhydrous sodium sulfate, and filtered, and the filtrate was concentrated. The obtained crude product was purified by silica gel column chromatography (petroleum ether : ethyl acetate = 10 : 1 to 2 : 1) to obtain compound **8-9.** ¹H NMR (400 MHz, CDCl₃) δ 2.63-2.73 (m, 2H), 2.23-2.35 (m, 4H), 0.95-1.05 (m, 1H).

### Step 8: Synthesis of compound 8-10

Compound **8-9** (7.72 g, 43.59 mmol), LiBr (5.16 g, 59.44 mmol) and TEA (8.02 g, 79.25 mmol, 11.03 mL) were added to anhydrous tetrahydrofuran (50 mL), a solution of diethyl cyanomethylphosphate (4.80 g, 39.62 mmol) in anhydrous tetrahydrofuran (50 mL) was added dropwise at 25 °C, and the mixture was stirred at 25 °C for 16 h. After the reaction was completed, water (100 mL) was added to the reaction liquid, and the mixture was extracted with ethyl acetate (100 mL × 3). The extracts were combined, dried over anhydrous sodium sulfate, and filtered, and the filtrate was concentrated. The obtained crude product was purified by silica gel column chromatography (petroleum ether : ethyl acetate = 10 : 1 to 3 : 1) to obtain compound **8-10.** ¹H NMR (400 MHz, CDCl₃) δ 5.33 (s, 1H), 2.94-3.03 (m, 1H), 2.84-2.93 (m, 2H), 2.60-2.75 (m, 1H), 2.10-2.24 (m, 2H), 0.82-0.93 (m, 1H).

### Step 9: Synthesis of compound 8

Compound **8-10** (0.60 g, 1.99 mmol) and potassium carbonate (824.47 mg, 5.95 mmol) were added to water (12 mL), a solution of compound **6-3** (430.03 mg, 2.98 mmol) in dioxane (8 mL) was added dropwise at 20 °C, and the mixture was stirred at 80 °C for 4 h. After the reaction was completed, water (50 mL) was added to the reaction liquid, and the mixture was extracted with ethyl acetate (50 mL × 3). The extracts were combined, dried over anhydrous sodium sulfate, and filtered, and the filtrate was concentrated. The crude product obtained was purified by preparative thin layer chromatography (petroleum ether : ethyl acetate = 0 : 1) and preparative high performance liquid chromatography (column model: Phenomenex Gemini-NX C18 75*30 mm*3 µm; mobile phase: [water (0.1%TFA)-ACN]; B(ACN)%: 25%-55%, 7 min) to obtain compound **8.** ¹H NMR (400 MHz, CD₃OD) δ 7.28-7.33 (m, 1H), 7.04-7.18 (m, 1H), 6.80-6.89 (m, 1H), 6.53-6.60 (m, 1H), 6.09-6.15 (m, 1H), 5.64-5.72 (m, 1H), 5.35-5.40 (m, 1H), 4.01-4.08 (m, 1H), 3.18-3.26 (m, 2H), 2.43 (s, 3H), 1.75-1.80 (m, 1H), 1.35-1.65 (m, 3H), 0.66-1.17 (m, 2H). MS ESI calculated for C₂₂H₁₉N₉ [M + H]⁺ 410, found 410.

### Example 9

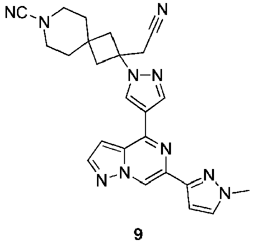

### Synthetic route:

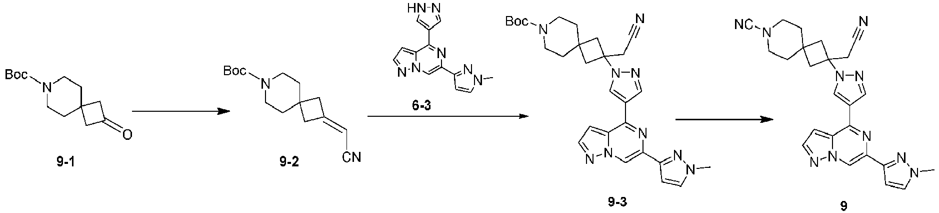

### Step 1: Synthesis of compound 9-2

Under nitrogen atmosphere, potassium tert-butoxide (1 M, 4.60 mL) was added to tetrahydrofuran (5 mL), and diethyl cyanomethylphosphonate (814.22 mg, 4.60 mmol, 740.20 µL) was added to the above solution at 0 °C and stirred at 0 °C for 20 min. **9-1** (1g, 4.18 mmol) was dissolved in tetrahydrofuran (5 mL), the obtained mixture was added to the above solution and stirred at 20 °C for 2 h. The reaction mixture was poured into water (100 mL), extracted with ethyl acetate (60 mL × 3), washed with brine (80 mL), dried over sodium sulfate, concentrated to dryness, and purified by silica gel column (petroleum ether : ethyl acetate = 5 : 1) to obtain compound **9-2.** ¹H NMR (400 MHz, CDCl₃) δ 5.20-5.30 (m, 1 H), 3.20-3.45 (m, 4 H), 2.72 (d, *J* = 1.6 Hz, 2 H), 2.62 (d, *J* = 1.6 Hz, 2 H), 1.50-1.65 (m, 4 H), 1.46 (s, 9 H).

### Step 2: Synthesis of compound 9-3

**6-3** (0.3 g, 994.25 µmol) was dissolved in acetonitrile (4 mL), 1,8-diazabicyclo[5.4.0]undecan-7-ene (454.10 mg, 2.98 mmol, 449.60 µL) and compound **9-2** (286.92 mg, 1.09 mmol) were add, and the mixture was stirred at 15 °C for 16 h. The mixture was concentrated under reduced pressure and purified by silica gel column (petroleum ether : ethyl acetate = 10 : 1 to 1 : 3) to obtain compound **9-3.** MS ESI calculated for C₂₈H₃₃N₉O₂ [M+H]⁺ 528, found 528.

### Step 3: Synthesis of compound 9

Compound **9-3** (70 mg, 125.51 µmol) was dissolved in dichloromethane (1 mL), trifluoroacetic acid (291.37 mg, 2.56 mmol, 189.20 µL) was added, and the mixture was stirred at 15 °C for 0.5 h. Then triethylamine (317.50 mg, 3.14 mmol, 436.73 µL) was added, the mixture was cooled to 0 °C, bromonitrile (15.95 mg, 150.61 µmol) was added, and the mixture was stirred at 0 °C for 0.5 h. 30 mL of water was added, and the mixture was extracted with 40 mL of dichloromethane (20 mL × 2). The combined organic layer was washed with 20 mL of brine, dried over sodium sulfate, concentrated to dryness, and purified by high performance liquid chromatography (column model: WatersXbridge 150*25 mm*5 µm; mobile phase: [water (0.1% ammonium bicarbonate)-acetonitrile]; B(acetonitrile)%: 16%-46%, 10 min) to obtain compound **9.** ¹H NMR(400 MHz, CD₃OD) δ 8.88 (d, *J* = 1.2 Hz, 1H), 8.84 (s, 1H), 8.46 (s, 1H), 8.14 (d, *J* = 2.4 Hz, 1H), 7.68 (d, *J* = 2.4 Hz, 1H), 7.28 (dd, *J* = 0.8, 2.4 Hz, 1H), 6.95 (d, *J* = 2.4 Hz, 1H), 3.92 (s, 3H), 3.27 (s, 2H), 3.22-3.26 (m, 2H), 3.16-3.21 (m, 2H), 2.92 (d, *J* = 14.4 Hz, 2H), 2.53 (d, *J* = 14.4 Hz, 2H), 1.82-1.92 (m, 2H), 1.56-1.72 (m, 2H). MS ESI calculated for C₂₄H₂₄N₁₀ [M + H]⁺ 453, found 453.

### Examples 10 and 11

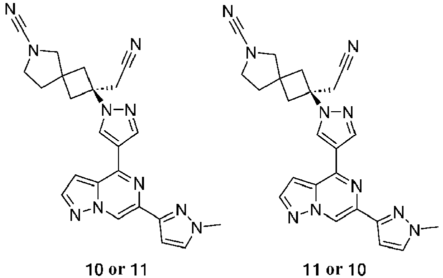

### Synthetic route:

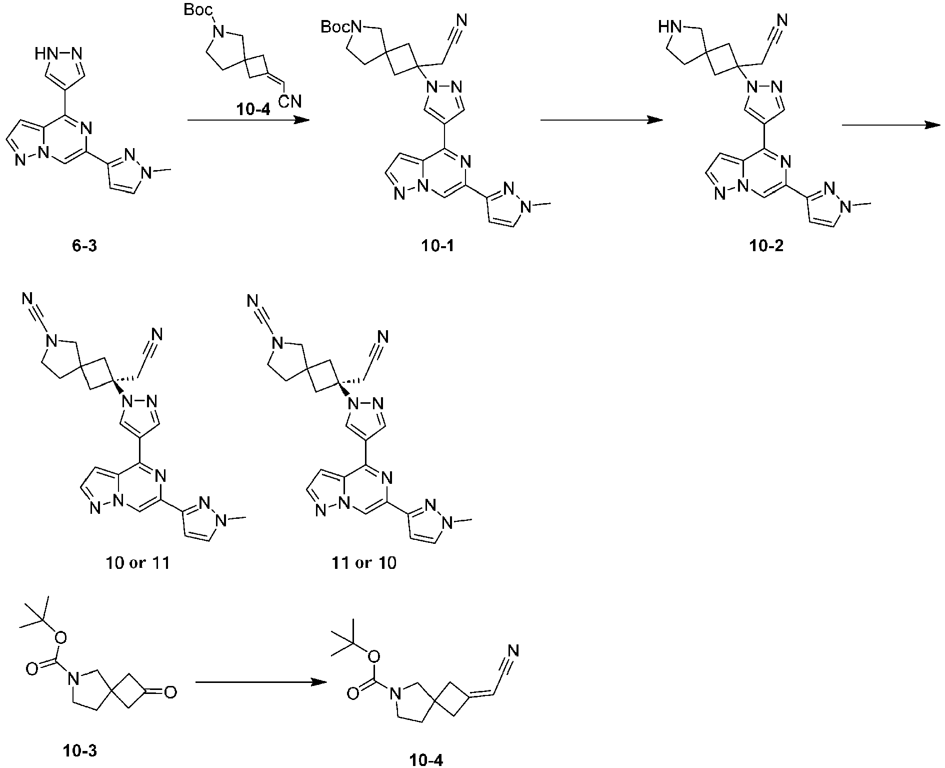

### Step 1: Synthesis of compound 10-1

1,8-diazabicyclo[5.4.0]undecan-7-ene (302.72 mg, 1.99 mmol, 299.73 µE) and **10-4** (181.05 mg, 729.12 µmol) were added to a solution of 6-3 (0.2 g, 662.84 µmol) in acetonitrile (3 mL), and the mixture was reacted at 25 °C for 16 h. The obtained product was concentrated under reduced pressure and purified by silica gel chromatography column (petroleum ether : ethyl acetate = 2 : 1) to obtain compound **10-1.** MS ESI calculated for C₂₇H₃₁N₉O₂ [M + H]⁺ 514, found 514.

### Step 2: Synthesis of compound 10-2

Compound **10-1** (330 mg, 642.53 µmol) was dissolved in dichloromethane (6 mL), trifluoroacetic acid (1.85 g, 16.21 mmol, 1.2 mL) was added, and the reaction was carried out at 25 °C for 1 h. The obtained product was concentrated to dryness under reduced pressure to obtain compound **10-2.** MS ESI calculated for C₂₂H₂₃N₉ [M + H]⁺ 414, found 414.

### Step 3: Synthesis of compound 10-4

A solution of diethyl cyanomethylphosphonate (943.56 mg, 5.33 mmol, 857.78 µL), anhydrous lithium bromide (578.27 mg, 6.66 mmol) and triethylamine (898.33 mg, 8.88 mmol) in tetrahydrofuran (5 mL) was added to a solution of compound 10-3 (1 g, 4.44 mmol) in tetrahydrofuran (5 mL), and the mixture was reacted at 25 °C for 16 h. The reaction liquid was diluted with 100 mL of water, extracted with 120 mL of ethyl acetate (60 mL × 2), and the organic layers were combined and washed with 80 mL of brine, dried over sodium sulfate, concentrated under reduced pressure, and purified by silica gel chromatography column (petroleum ether : ethyl acetate = 3 : 1) to obtain compound 10-4. ¹HNMR(400MHz, CDCl₃) δ 5.17-5.31 (m, 1H), 3.32-3.49 (m, 4H), 2.73-3.02 (m, 4H), 1.86-1.99 (m, 2H), 1.46 (s, 9H).

### Step 4: Synthesis of compounds 10 and 11

Compound **10-2** (0.34 g, 644.55 µmol) was dissolved in 8 mL of dichloromethane, and nitrile bromide (136.54 mg, 1.29 mmol, 94.82 µL) and triethylamine (326.11 mg, 3.22 mmol, 448.57 µL) were added, and the mixture was reacted at 25 °C for 2 h. The obtained product was diluted with 100 mL of water and extracted with dichloromethane (50 mL × 2). The combined organic layers were washed with 50 mL of brine, dried over sodium sulfate, and purified by silica gel chromatography column (petroleum ether/ethyl acetate = 1/2), and then subjected to chiral resolution (chiral column model: DAICEL CHIRALPAK AD(250 mm*30 mm*10 µm); mobile phase: [0.1% ammonia/ethanol]; B(ethanol)%: 60%-60%, 4.1 min), compound **10** and compound **11** were obtained after resolution (analysis method: column model Chiralpak AD-3 50 × 4.6 mm I.D., 3 µm; mobile phase: A: CO₂, B: ethanol (0.05% diethylamine); gradient: Phase B 40%; flow rate at 3 mL/min; column temperature 35 °C). The retention times were 1.288 min and 2.367 min respectively. Compound 10: ¹H NMR (400 MHz, DMSO-d₆) δ 8.87 (d, *J=* 2.0 Hz, 2H), 8.49 (s, 1H), 8.25 (d, *J=* 2.4 Hz, 1H), 7.83 (d, J = 2.0 Hz, 1H), 7.48 (d, *J =* 1.6 Hz, 1H), 6.98 (d, J = 2.0 Hz, 1H), 3.94 (s, 3H), 3.53 (s, 2H), 3.46 (s, 2H), 3.35-3.38 (m, 2H), 2.97 (d, *J=* 14 Hz, 2H), 2.63 (d, *J=* 14 Hz, 2H), 1.83 (t, *J* = 7.2 Hz, 2H). MS ESI calculated for C₂₃H₂₂N₁₀ [M + H]⁺ 439, found 439. Compound **11:** ¹HNMR(400 MHz, DMSO-d₆)δ 8.86 (d, *J=* 3.6 Hz, 2H), 8.49 (s, 1H), 8.25 (d, *J=* 2.4 Hz, 1H), 7.83 (d, *J=* 2.0 Hz, 1H), 7.49 (d, *J=* 1.6 Hz, 1H), 6.99 (d, *J=* 2.4 Hz, 1H), 3.94 (s, 3H), 3.48 (s, 2H), 3.42 (t, *J=* 6.8 Hz, 2H), 3.31 (s, 2H), 3.01 (d, *J=* 13.6 Hz, 2H), 2.57 (d, *J=* 13.6 Hz, 2H), 2.09 (t, *J* = 6.8 Hz, 2H). MS ESI calculated for C₂₃H₂₂N₁₀ [M + H]⁺ 439, found 439.

### Examples 12 and 13

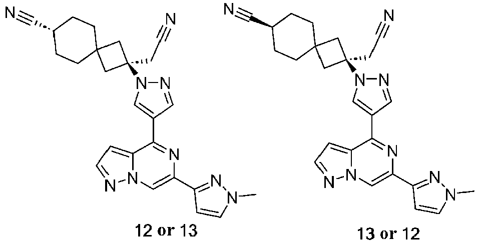

### Synthetic route:

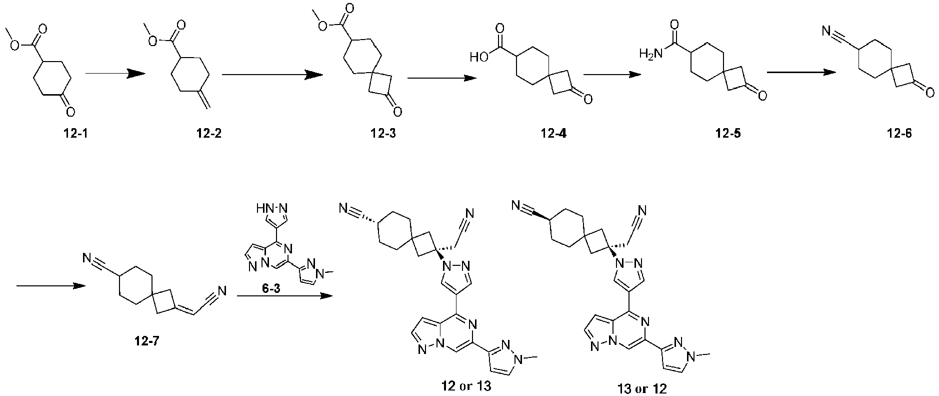

### Step 1: Synthesis of compound 12-2

Under nitrogen protection at -10 °C, n-butyllithium (2.5 M, 17.93 mL) was slowly added dropwise to a turbid solution of methyltriphenylphosphine bromide (17.15 g, 48.02 mmol) in tetrahydrofuran (50 mL). After the dropwise addition was completed, stirring was continued for 1 h at this temperature. Compound **12-1** (5 g, 32.01 mmol) was added to the above obtained suspension, and the reaction system was warmed to 20 °C and stirred for 3 h. After the reaction was completed, acetone (100 mL) was added to quench the reaction and the obtained product was concentrated under reduced pressure. The residue was purified by silica gel column chromatography (petroleum ether : ethyl acetate = 5 : 1) to obtain compound 12-2. ¹HNMR(400MHz,CDCl₃)δ 4.65 (s, 2 H), 3.67 (s, 3 H), 2.40-2.55 (m, 1 H), 2.25-2.39 (m, 2 H), 1.90-2.10 (m, 4 H), 1.45-1,65 (m, 4 H).

### Step 2: Synthesis of compound 12-3

Under nitrogen protection at 0 °C, 2,2,2-trichloroacetyl chloride (2.83 g, 15.56 mmol, 1.74 mL) was slowly added dropwise into a turbid solution of compound **12-2** (2 g, 12.97 mmol) and zinc-copper reagent (5.02 g, 38.91 mmol) in diethyl ether (30 mL). In the process of dropwise addition, the reaction was violently exothermic. After the temperature was warmed rapidly, the internal temperature should be strictly controlled to be around 0 °C before adding dropwise again. After the dropwise addition was completed, the reaction was slowly warmed to 20 °C, and then the reaction liquid was stirred at this temperature for 1 h. The reaction was then cooled to -5 °C, methanol (10 mL) was added, and then zinc powder (2.54 g, 38.91 mmol) was added in portions within 1 h. After the addition was completed, the temperature was slowly warmed to 20 °C, then diatomaceous earth was added, and the resulting mixture was filtered through diatomaceous earth. The filter cake was rinsed with ethyl acetate (50 mL × 3), and the resulting filtrate was washed with saturated brine (80 mL) by shaking, dried over anhydrous sodium sulfate, and filtered, the filtrate was concentrated, and the residue was purified by silica gel column chromatography (petroleum ether : ethyl acetate = 0 : 1 to 3 : 1) to obtain compound **12-3.** ¹HNMR(400MHz,CDCl₃)δ 3.61 (s, 3 H), 2.69 (s, 4 H), 2.20-2.30 (m, 1 H), 1.80-1.95 (m, 2 H), 1.65-1.75 (m, 2 H), 1.40-1.60 (m, 4 H).

### Step 3: Synthesis of compound 12-4

A solution of compound **12-3** (0.4 g, 2.04 mmol) and sodium hydroxide (163.05 mg, 4.08 mmol) in water (10 mL) and methanol (50 mL) was heated to 65 °C and stirred for 1 h. After the reaction was completed, the reactant was cooled to 0 °C, and adjusted to pH 6 with 2 M hydrochloric acid, and extracted with ethyl acetate (10 mL × 3). The combined extracts were washed with saturated brine (15 mL) by shaking, and dried over sodium sulfate, and filtered, and the filtrate was concentrated to obtain compound **12-4.** ¹H NMR(400MHz, DMSO-D₆)δ 2.72 (s, 4 H), 2.05-2.15 (m, 1 H), 1.15-1.90 (m, 8 H).

### Step 4: Synthesis of compound 12-5

Under nitrogen protection at 0 °C, oxalyl chloride (313.46 mg, 2.47 mmol, 216.18 µL) was added dropwise to a solution of compound **12-4** (0.3 g, 1.65 mmol) in tetrahydrofuran (40.00 mL) and DMF (0.05 mL). After the dropwise addition was completed, stirring was continued at 0 °C for 1 h. The obtained product was concentrated under reduced pressure, and the residue was redissolved in dichloromethane (10 mL) solution. Excess liquid ammonia was added to the solution and stirred at 20 °C for 1 h. After the reaction was completed, the obtained product was concentrated under reduced pressure, the residue was rinsed with water (2 mL × 2), and the solid was dried under vacuum and reduced pressure to obtain compound **12-5.** MS ESI calculated for C₁₀H₁₅NO₂ [M+H]⁺ 182, found 182.

### Step 5: Synthesis of compound 12-6

Under nitrogen protection at 0 °C, trifluoroacetic anhydride (417.21 mg, 1.99 mmol, 276.30 µL) was added dropwise to a solution of compound **12-5** (0.18 g, 993.21 µmol) and triethylamine (502.51 mg, 4.97 mmol, 691.21 µL) in dichloromethane (5 mL). After the dropwise addition was completed, the mixture was stirred at 0 °C for half an hour. After the reaction was completed, saturated sodium bicarbonate (30 mL) was added to the reaction liquid, the mixture was extracted with dichloromethane (15 mL × 3), the combined organic phase was washed with saturated brine (20 mL) by shaking, dried over anhydrous sodium sulfate and filtered, and the filtrate was concentrated to obtain compound **12-6.** MS ESI calculated for C₁₀H₁₃NO [M + H]⁺ 164, found 164.

### Step 6: Synthesis of compound 12-7

Under nitrogen protection at 0 °C, a solution of diethyl cyanomethylphosphonate (119.38 mg, 673.95 µmol, 108.53 µL) in tetrahydrofuran (6 mL) was added dropwise to a solution of 1 M potassium tert-butoxide (1 M, 673.95 µL) in tetrahydrofuran, after the dropwise addition was completed, the mixture was warmed to 20 °C and stirred for 20 min, then cooled to 0 °C again, and a solution of compound **12-6** (0.1 g, 612.69 µmol) in tetrahydrofuran (2 mL) was added dropwise to the solution. After the dropwise addition was completed, the obtained product was warmed to 20 °C and stirred for 1 h. After the reaction was completed, the reactants were poured into water (50 mL), and the resulting aqueous solution was extracted with ethyl acetate (10 mL × 3). The combined extracts were washed with saturated brine (10 mL) by shaking, dried over anhydrous sodium sulfate, and filtered, the filtrate was concentrated, and the residue was purified by silica gel column chromatography (dichloromethane : methanol = 50 : 1) to obtain compound **12-7. ¹H NMR**(400MHz, CDCl₃)δ 5.15-5.20 (m, 1 H), 2.90 (s, 1 H), 2.85 (s, 1 H), 2.50-2.75 (m, 4 H), 1.75-1.90 (m, 4 H).

### Step 7: Synthesis of compounds 12 and 13

Under nitrogen protection, a solution of compound **12-7** (80 mg, 429.52 µmol), compound **6-3** (113.94 mg, 429.52 µmol) and DBU (98.08 mg, 644.29 µmol, 97.11 µL) in acetonitrile (3 mL) was heated to 66 °C and stirred for 6 h. After the reaction was completed, the reactant was concentrated, and the residue was separated by preparative HPLC (separation column model: Welch × timate C18 150*30 mm*5 µm; Mobile phase: [water (0.225% formic acid)-acetonitrile]; B (acetonitrile)%: 40%-50%, 8min) to obtain the racemate. The racemate was subjected to chiral resolution (chiral column model: DAICEL CHIRALPAK AD (250 mm*30 mm, 10 µm); mobile phase: A: CO₂, B: 0.1% ammonia ethanol, ethanol (0.1% ammonia) and supercritical fluid CO₂ from 50% to 50%, flow rate at 4 mL/min), compound **12** and compound **13** were obtained after resolution, (analysis method: column model Chiralpak AD-3 50 × 4.6 mm I.D., 3 µm; mobile phase: A: CO₂, B: ethanol (0.05% diethylamine); gradient: phase B from 5% to 40% in 2 min, then holding at 40% for 1.2 min, and finally 5% for 0.8 min; flow rate at 4 mL/min; column temperature 35 °C). The retention times were 2.282 min and 2.566 min respectively. Compound **12: ¹H NMR**(400MHz, CD₃OD)δ 8.88 (s, 1 H), 8.84 (s, 1 H), 8.46 (s, 1 H), 8.13 (d, *J=* 2.4 Hz, 1 H), 7.68 (d, *J=* 2.0 Hz, 1 H), 7.28 (d, *J* = 2.0 Hz, 1 H), 6.94 (d, *J=* 2.0 Hz, 1 H), 4.00 (s, 3 H), 3.24 (s, 2 H), 2,70-2.90 (m, 3 H), 2.40-2.50 (m, 2H), 1.40-2.00 (m, 8H). MS ESI calculated for C₂₅H₂₅N₉ [M + H]⁺ 452, found 452. Compound **13:** ¹ **H NMR**(400MHz , CD₃OD)δ 8.88 (s, 1 H), 8.84 (s, 1 H), 8.46 (s, 1 H), 8.13 (d, *J =* 2.4 Hz, 1 H), 7.68 (d, *J* =2.4 Hz, 1 H), 7.28 (d, *J =* 2.4 Hz, 1 H), 6.94 (d, *J =* 2.4 Hz, 1 H), 4.00 (s, 3 H), 3.24 (s, 2H), 2,70-2.90 (m, 3 H), 2.40-2.50 (m, 2H), 1.40-2.00 (m, 8H). MS ESI calculated for C₂₅H₂₅N₉ [M + H]⁺ 452, found 452.

### Examples 14 and 15

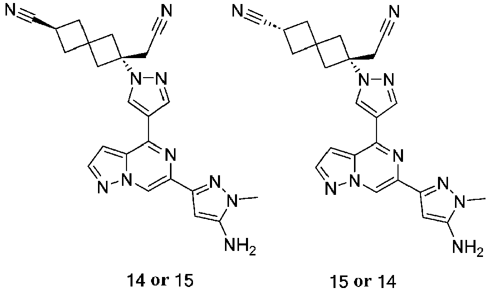

### Synthetic route:

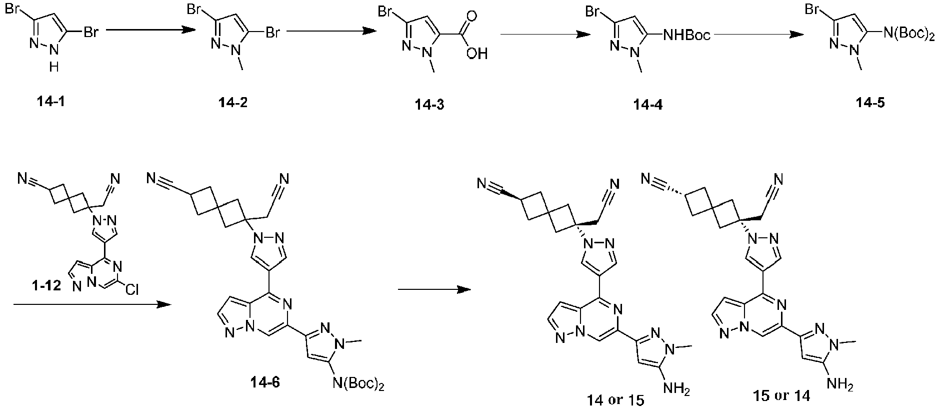

### Step 1: Synthesis of compound 14-2

Compound **14-1** (5 g, 22.14 mmol) was dissolved in tetrahydrofuran (80 mL), and sodium hydride (1.33 g, 33.21 mmol, 60% purity) was added in portions at 0 °C, and the mixture was stirred at this temperature for 30 min, then methyl iodide (4.08 g, 28.78 mmol, 1.79 mL) was added dropwise at 0 °C, and the mixture was reacted at 20 °C for 2 h. Saturated ammonium chloride aqueous solution (100 mL) was added to quench the reaction, the obtained product was diluted with 100 mL of water, and extracted with ethyl acetate (100 mL × 2). The combined organic layer was washed with 100 mL of brine, dried over sodium sulfate, filtered, concentrated under reduced pressure and purified by silica gel column (petroleum ether : ethyl acetate = 10 : 1) to obtain the compound **14-2.** ¹H NMR (400 MHz, CDCl₃) δ 6.29 (s, 1H), 3.85 (s, 3H).

### Step 2: Synthesis of compound 14-3

At -65 °C, n-butyllithium (2.5 M, 8.68 mL) was added dropwise to a solution of **14-2** (4.34 g, 18.09 mmol) in tetrahydrofuran (80 mL). After the addition, the reaction was carried out at this temperature for 0.5 h, then dry ice (20 g) was added at -65 °C, and the reaction was carried out at -65 °C for 30 min. The reaction mixture was quenched by adding 1 M hydrochloric acid aqueous solution to pH = 4, then diluted with 200 mL of water, and extracted with ethyl acetate (100 mL × 2). The combined organic layer was washed with 100 mL of brine, dried over sodium sulfate, and purified by silica gel column (petroleum ether : ethyl acetate = 10 : 1 to 1 : 1) to obtain compound **14-3.** ¹HNMR (400 MHz, CDCl₃) δ 10.61 (brs, 1 H), 6.96 (s, 1 H), 4.19 (s, 3 H).

### Step 3: Synthesis of compound 14-4

Compound **14-3** (2.47 g, 12.05 mmol) was dissolved in tert-butanol (40 mL), diphenyl azidophosphonate (3.98 g, 14.46 mmol, 3.13 mL) and diisopropylethylamine (3.11 g, 24.10 mmol, 4.20 mL) were added, the mixture was stirred at 45 °C for 0.5 h, and then heated to 100 °C and reacted for 16 h. The reaction mixture was diluted with 200 mL of water and extracted with 200 mL of ethyl acetate. The combined organic layers were washed with saturated sodium bicarbonate aqueous solution (100 mL × 2) and brine 100 mL, dried over sodium sulfate, filtered, concentrated under reduced pressure, and purified by silica gel column (petroleum ether : ethyl acetate = 30 : 1 to 10 : 1) to obtain compound **14-4.** ¹HNMR (400 MHz, CDCl₃) δ 6.55 (brs, 1 H), 6.18 (s, 1 H), 3.71 (s, 3 H), 1.50 (s, 9 H).

### Step 4: Synthesis of compound 14-5

**14-4** (2.5 g, 9.05 mmol) was dissolved in dichloromethane (40 mL), and 4-dimethylaminopyridine (221.22 mg, 1.81 mmol), di-tert-butyl dicarbonate (3.95 g, 18.11 mmol, 4.16 mL) and triethylamine (2.75g, 27.16 mmol, 3.78 mL) were added, and the mixture was reacted at 20 °C for 16 h. The reaction mixture was diluted with 200 mL of water, and extracted with dichloromethane (100 mL × 2), and the combined organic layer was washed with 100 mL of brine, dried over sodium sulfate, filtered, concentrated under reduced pressure, and purified by silica gel column (petroleum ether/ethyl acetate = 1/0 to 10/1) to obtain compound **14-5.** ¹HNMR (400 MHz, CDCl₃) δ 6.14 (s, 1 H), 3.65 (s, 3 H), 1.45 (s, 18 H).

### Step 5: Synthesis of compound 14-6

**14-5** (597.48 mg, 1.59 mmol), dipinacol borate (604.89 mg, 2.38 mmol), Xphos-Pd-G2 (124.95 mg, 158.80 µmol) and potassium acetate (389.62 mg, 3.97 mmol) were added to dioxane (15 mL), the mixture was purged with nitrogen three times, and stirred at 65 °C for 4 h under nitrogen atmosphere. Then the mixture was cooled to 20 °C and Xphos-Pd-G2 (124.95 mg, 158.80 µmol), potassium phosphate (842.72 mg, 3.97 mmol),**1-12** (0.3 g, 794.01 µmol) and water (2 mL) were added, and the obtained mixture was reacted at 90 °C for 3 h under nitrogen atmosphere. The reaction mixture was diluted with 80 mL of water and extracted with ethyl acetate (60 mL × 2). The combined organic layer was washed with 60 mL of brine, dried over sodium sulfate, and purified by silica gel column (petroleum ether : ethyl acetate = 10 : 1 to 1 : 1) to obtain compound **14-6.** MS ESI calculated for C₃₃H₃₈N₁₀O₄ [M + H]⁺ 639, found 639.

### Step 6: Synthesis of compounds 14 and 15

**14-6** (0.22 g, 308.96 µmol) was dissolved in dichloromethane (5 mL), trifluoroacetic acid (13.51 mmol, 1 mL) was added, and the mixture was stirred at 20 °C for 3 h. The reactant was concentrated under reduced pressure and purified by high performance liquid chromatography (column model: Shim-pack C18 150 × 25 × 10 µm; mobile phase: [water (0.225% formic acid)-acetonitrile]; B(acetonitrile)%: 23%-43%, 9 min) and subjected to chiral resolution (chiral column model: DAICELCHIRALPAK IG(250 mm*30 mm*10 µm); mobile phase: [0.1% ammonia/methanol]; B(methanol)%: 50%-50%, 9.7; 110 min), compounds **14** and **15** were obtained respectively after resolution (analysis method: column model Chiralpak IG-3 50 × 4.6 mm I.D., 3 µm; mobile phase: A: CO₂, B: methanol + acetonitrile (0.05% diethylamine); gradient: phase B 60% methanol + acetonitrile (0.05% diethylamine); flow rate at 3 mL/min; column temperature 35 °C). The retention times were 2.453 min and 3.274 min respectively. Compound 14: ¹HNMR (400 MHz, DMSO-d₆) δ 8.73 (s, 2H), 8.38 (s, 1H), 8.21 (d, *J* = 2.4 Hz, 1H), 7.43 (d, *J=* 1.6 Hz, 1H), 6.01 (s, 1H), 5.36 (s, 2H), 3.61 (s, 3H), 3.21-3.29 (m, 3H), 2.90-3.05 (m, 2H), 2.58-2.66 (m, 3H), 2.51-2.55 (m, 1H), 2.31-2.42 (m, 2H). MS ESI calculated for C₂₃H₂₂N₁₀ [M + H]+ 439, found 439. Compound 15: ¹HNMR (400 MHz, DMSO-d₆) δ 8.73 (s, 2H), 8.38 (s, 1H), 8.21 (d, *J=* 2.4 Hz, 1H), 7.43 (d, *J=* 1.6 Hz, 1H), 6.01 (s, 1H), 5.35 (s, 2H), 3.61 (s, 3H), 3.21-3.29 (m, 3H), 2.90-3.04 (m, 2H), 2.57-2.66 (m, 3H), 2.53 (s, 1H), 2.30-2.42 (m, 2H). MS ESI calculated for C₂₃H₂₂N₁₀ [M + H]+ 439, found 439.

### Examples 16 and 17

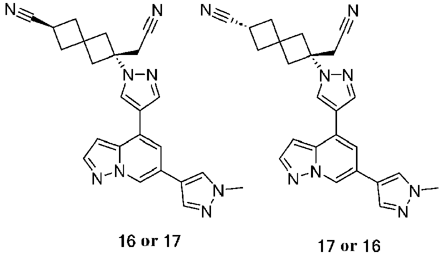

### Synthetic route:

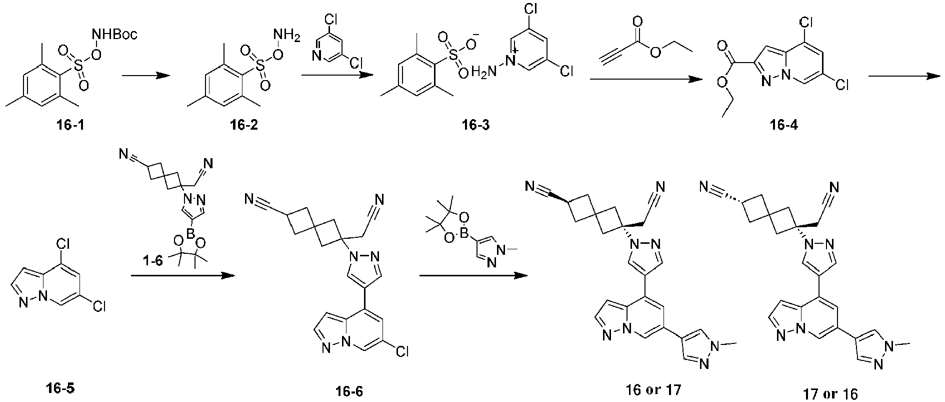

### Step 1: Synthesis of compound 16-2

**16-1** (20 g, 63.41 mmol) was added to trifluoroacetic acid (61.60 g, 540.24 mmol, 40.00 mL) at 0 °C, and the mixture was reacted at 0 °C for 2 h. The reaction liquid was poured into ice water (200 mL), the resulting white precipitate was filtered off, and then washed with water (100 mL) to obtain compound **16-2.**

### Step 2: Synthesis of compound 16-3

**16-2** (13.09 g, 60.81 mmol) was dissolved in dichloromethane (80 mL) at 0 °C, and 3,5-dichloropyridine (6 g, 40.54 mmol) was added, the mixture was stirred at 15 °C for 16 h. A white solid was precipitated and filtered, and the filter cake was dried under vacuum to obtain compound **16-3.** ¹HNMR (400 MHz, CD₃OD) δ 8.93 (d, *J* = 1.6 Hz, 2H), 8.56 (t, *J* = 1.6 Hz, 1H), 6.87 (s, 2H), 2.61 (s, 6 H), 2.24 (s, 3H).

### Step 3: Synthesis of compound 16-4

Compound **16-3** (10.56 g, 29.07 mmol) was dissolved in dimethylformamide (120 mL), silver carbonate (8.02 g, 29.07 mmol) and ethyl propiolate (2.85 g, 29.07 mmol, 2.85 mL) were added, the mixture was stirred at 30-40 °C for 16 h. The reaction mixture was concentrated under reduced pressure and purified by silica gel column (petroleum ether : ethyl acetate = 50 : 1 to 0 : 1) to obtain compound **16-4.** ¹HNMR (400 MHz, CDCl₃) δ 8.50 (s, 1H), 7.23-7.27 (m, 2H), 4.49 (q, *J=* 7.2 Hz, 2H), 1.46 (t, *J=* 7.2 Hz, 3H).

### Step 4: Synthesis of compound 16-5

Compound **16-4** (2.05 g, 7.91 mmol) was added to hydrobromic acid aqueous solution (45.82 g, 226.51 mmol, 30.75 mL, 40% purity), and the mixture was reacted at 100 °C for 16 h. After cooling to room temperature, 60 mL of water was added, and the precipitate was precipitated and filtered. The filter cake was dried and then purified by silica gel column (petroleum ether : ethyl acetate = 20 : 1 to 0 : 1) to obtain compound **16-5.** ¹HNMR (400 MHz, CDCl₃) δ 8.47 (s, 1H), 7.98 (d, *J =* 2.0 Hz, 1H), 7.18 (d, *J =* 1.6 Hz, 1H), 6.69 (d, *J =* 1.6 Hz, 1H).

### Step 5: Synthesis of compound 16-6

Compound **16-5** (0.1 g, 534.69 µmol), compound **1-6** (207.17 mg, 588.15 µmol), potassium phosphate (340.49 mg, 1.60 mmol), and tri-tert-butyl phosphine palladium (27.33 mg, 53.47 µmol) was added to dioxane (3 mL) and water (0.8 mL), nitrogen replacement was performed 3 times, and the mixture was reacted at 30-40 °C for 16 h under nitrogen atmosphere. The reaction was quenched by adding saturated ammonium chloride aqueous solution (3 mL), then diluted with 20 mL of water, and extracted with ethyl acetate (20 mL × 2). The combined organic layer was washed with 20 mL of brine, dried over sodium sulfate, and purified by silica gel column (petroleum ether: ethyl acetate=10 : 1 to 2 : 1) to obtain compound **16-6.** ¹HNMR (400 MHz, CDCl₃) δ 8.48 (s, 1H), 7.99 (d, *J=* 2.4 Hz, 1H), 7.96 (s, 2H), 7.17 (d, *J=* 2.0 Hz, 1H), 6.72 (dd, *J=* 0.8, 2.4 Hz, 1H), 3.06-3.15 (m, 1H), 3.02 (s, 3H), 2.94 (d, *J=* 14.0 Hz, 1H), 2.71-2.81 (m, 2H), 2.66 (m, 2H), 2.44-2.52 (m, 2H).

### Step 6: Synthesis of compounds 16 and 17

Compound **16-6** (0.15 g, 398.05 µmol), 1-methyl-4-pyrazole pinacol borate (99.38 mg, 477.65 µmol), Xphos-Pd-G2 (36.08 mg, 39.80 µmol), and potassium phosphate (253.47 mg, 1.19 mmol) were added to dioxane (4 mL) and water (0.8 mL), nitrogen replacement was performed 3 times, and the mixture was stirred at 100 °C for 16 h under nitrogen atmosphere. The reaction mixture was diluted with 60 mL of water and extracted with ethyl acetate (30 mL × 2). The combined organic layer was washed with 30 mL of brine, dried over sodium sulfate, and purified by silica gel column (petroleum ether : ethyl acetate = 5 : 1 to 0 : 1), and then subjected to chiral resolution (column model: DAICEL CHIRALPAKAD(250 mm*30 mm* 10 µm); mobile phase: [0.1% ammonia/ethanol]; B(ethanol)%: 40%-40%, 3.8 min; 60 min), compound **16** and compound **17** were obtained after resolution (analysis method: column model Chiralpak AD-3 50 × 4.6 mm I.D., 3 µm; mobile phase: A: CO₂, B: ethanol (0.05% diethylamine); gradient: phase B 40% ethanol (0.05% diethylamine); flow rate at 3 mL/min; column temperature 35 °C). The retention times were 2.270 min and 2.754 min respectively. Compound **16:** ¹HNMR (400 MHz, DMSO-d₆) δ 8.91 (s, 1H), 8.58 (s, 1H), 8.31 (s, 1H), 8.26 (s, 1H), 8.07 (s, 1H), 8.05 (d, *J=* 2.4 Hz, 1H), 7.75 (d, *J=* 1.6 Hz, 1H), 7.00 (dd, *J=* 0.8, 2.4 Hz, 1H), 3.89 (s, 3H), 3.26-3.31 (m, 3H), 2.89-2.98 (m, 2H), 2.51-2.67 (m, 4H), 2.31-2.42 (m, 2H). MS ESI calculated for C₂₄H₂₂N₈ [M + H]⁺ 423, found 423. Compound **17:** ¹HNMR(400 MHz, DMSO-d₆) δ 8.91 (s, 1H), 8.59 (s, 1H), 8.31 (s, 1H), 8.26 (s, 1H), 8.07 (s, 1H), 8.05 (d, *J* = 2.0 Hz, 1H), 7.75 (s, 1H), 7.00 (d, *J=* 1.6 Hz, 1H), 3.89 (s, 3H), 3.24-3.31 (m, 3H), 2.84-3.01 (m, 2H), 2.51-2.69 (m, 4H), 2.29-2.43 (m, 2H). MS ESI calculated for C₂₄H₂₂N₈ [M + H]⁺ 423, found 423.

### Examples 18 and 19

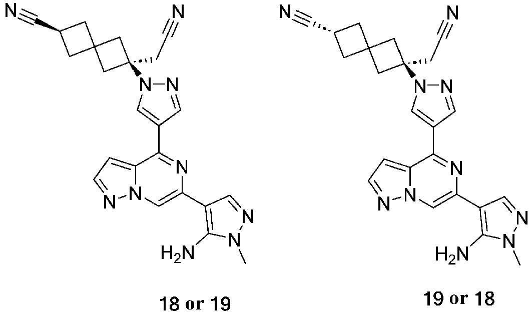

### Synthetic route:

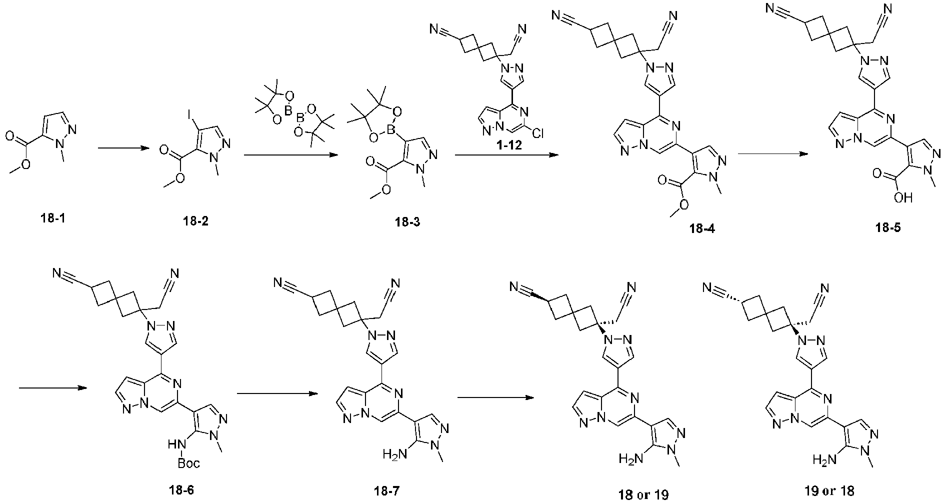

### Step 1: Synthesis of compound 18-2

Compound **18-1** (2.0 g, 14.27 mmol) was added to acetonitrile (20 mL), N-iodosuccinimide (3.53 g, 15.70 mmol) and trifluoroacetic acid (488.18 mg, 4.28 mmol, 317.00 µL) were added, and the mixture was stirred at 80 °C for 2 h. After the reaction was completed, the reaction liquid was concentrated to obtain a residue, which was diluted with ethyl acetate (100 mL), washed with brine (100 mL × 3) by shaking, dried over anhydrous sodium sulfate, and filtered, and the filtrate was concentrated. The obtained crude product was purified by silica gel column chromatography (petroleum ether : ethyl acetate = 1 : 0 to 50 : 1) to obtain compound **18-2.** ¹H NMR (400 MHz, CDCl₃) δ 7.56 (s, 1H), 4.19 (s, 3H), 3.94 (s, 3H).

### Step 2: Synthesis of compound 18-3

Compound **18-2** (2.0 g, 7.52mmol), compound dipinacol borate (3.82 g, 15.04 mmol), 1,1-bis(diphenylphosphino)ferrocenepalladium chloride (550.08 mg, 751.78 µmol,) and potassium acetate (2.21 g, 22.55 mmol) were added to dimethyl sulfoxide (30 mL), and the mixture was stirred at 100 °C for 1 h under nitrogen protection. After the reaction was completed, the reaction liquid was diluted with ethyl acetate (100 mL), washed with brine (50 mL × 3), dried over anhydrous sodium sulfate, and filtered, and the filtrate was concentrated. The obtained crude product was purified by silica gel column chromatography (petroleum ether : ethyl acetate = 100 : 1 to 5 : 1) to obtain compound **18-3,** MS ESI calculated for C₁₂H₁₉BN₂O4M + H]⁺ 267, found 267.

### Step 3: Synthesis of compound 18-4

Dioxane (8 mL) and water (2 mL) were added to a mixture of compound **18-3** (704.29 g, 2.65 mmol), compound **1-12** (0.50 g, 1.32 mmol), Xphos-Pd-G2 (104.12 mg, 132.33 µmol) and potassium phosphate (842.71 mg, 3.97 mmol), and the obtained mixture was stirred at 100 °C for 16 h under nitrogen protection. After the reaction was completed, the reaction liquid was diluted with ethyl acetate (50 mL), washed with brine (50 mL × 3), dried over anhydrous sodium sulfate, and filtered, and the filtrate was concentrated. The crude product was purified by silica gel column chromatography (petroleum ether : ethyl acetate = 4 : 1 to 1 : 3) to obtain compound **18-4,** MS ESI calculated for C₂₅H₂₃N₉O_{2[}M + H]⁺ 482, found 482.

### Step 4: Synthesis of compound 18-5

A solution of lithium hydroxide (38.20 g, 1.59 mmol) in water (0.5 mL) was added dropwise to a solution of compound **18-4** (0.4 g, 797.49 mmol) in methanol (10 mL), and the mixture was stirred at 20 °C for 16 h. After the reaction was completed, the reaction liquid was adjusted to pH 3-4 with 0.5 M hydrochloric acid, and concentrated to obtain a residue, which was diluted with dichloromethane (10 mL) and methanol (1 mL), and filtered, and the filtrate was concentrated to obtain compound **18-5,** MS ESI calculated for C₂₄H₂₁N₉O_{2[}M + H]⁺ 468, found 468.

### Step 5: Synthesis of compound 18-6

A solution of compound **18-5** (0.4 g, 658.85 mmol), diphenyl azidophosphate (271.97 mg, 998.27 µmol, 214.15 µL) and N,N-diisopropylethylamine (127.73 mg, 998.27 µmol, 172.14 µL) in tert-butanol (10 mL) was stirred at 60 °C for 0.5 h and then at 100 °C for 1.5 h under nitrogen protection. After the reaction was completed, the reaction liquid was diluted with ethyl acetate (10 mL), washed with saturated sodium bicarbonate aqueous solution (10 mL × 3), dried over anhydrous sodium sulfate, and filtered, and the filtrate was concentrated. The obtained crude product was purified by silica gel column chromatography (petroleum ether : ethyl acetate = 3 : 1 to 1 : 3) to obtain compound **18-6,** MS ESI calculated for C₂₈H_{3O}N₁₀O₂[M + H]⁺ 539, found 539.

### Step 6: Synthesis of compound 18-7

Compound **18-6** (0.3 g, 537.50 mmol) was added to anhydrous dichloromethane (10 mL), then trifluoroacetic acid (4.62 g, 40.52 mmol, 3 mL) was added, and the mixture was stirred at 30 °C for 1 h. After the reaction was completed, the reaction liquid was concentrated. The reaction liquid was diluted with ethyl acetate (10 mL), ammonia water (30% purity, 1 mL) was added, and the mixture was concentrated to obtain a residue, which was subjected to prep-HPLC (column model: Waters Xbridge 150*25 mm*5 µm; mobile phase: [H₂O (10 mM NH₄HCO₃)-ACN]; ACN %: 18%-48%, 9 min) to obtain compound **18-7.** MS ESI calculated for C₂₃H₂₂N₁₀[M + H]⁺ 439, found 439.

### Step 7: Synthesis of compounds 18 and 19

Compound **18-7** was subjected to chiral resolution (chiral column model: DAICEL CHIRALPAK AD (250 mm*30 mm*10 µm); mobile phase: [0.1%NH₃H₂O IPA]; B(IPA)%: 40%-40%, 4.5 min; 70 min, compound **18** and compound **19** were obtained after resolution, (analysis method: column model Chiralpak AD-3 50 × 4.6 mm I.D., 3 µm; mobile phase: A: CO₂, B: isopropanol (0.05% diethylamine); gradient: phase B 40% isopropanol (0.05% diethylamine); flow rate at 3 mL/min; column temperature 35 °C). The retention times were 1.099 min and 1.422 min respectively. Compound **18:** ¹H NMR (400 MHz, DMSO-d₆) δ 8.87 (s, 1H), 8.73 (s, 1H), 8.34 (s, 1H), 8.14 (d, *J* = 2.4 Hz, 1H), 7.85 (s, 1H), 7.37 (d, *J* = 1.8 Hz, 1H), 6.04 (s, 2H), 3.61 (s, 3H), 3.32-3.35 (m, 2H), 3.23-3.31 (m, 1H), 2.93-3.03 (m, 2H), 2.52-2.68 (m, 4H), 2.30-2.41 (m, 2H). MS ESI calculated for C₂₃H₂₂N₁₀[M + H]⁺ 439, found 439. Compound **19:** ¹H NMR (400 MHz, DMSO-d₆) δ 8.87 (s, 1H), 8.74 (s, 1H), 8.34 (s, 1H), 8.15 (d, *J* = 2.4 Hz, 1H), 7.85 (s, 1H), 7.38 (d, *J* = 1.8 Hz, 1H), 6.05 (s, 2H), 3.62 (s, 3H), 3.36-3.34 (m, 2H), 3.23-3.30 (m, 1H), 2.93-3.04 (m, 2H), 2.53-2.68 (m, 4H), 2.30-2.43 (m, 2H). MS ESI calculated for C₂₃H₂₂N₁₀[M + H]⁺ 439, found 439.

### Examples 20 and 21

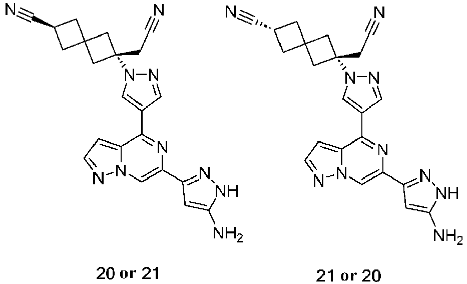

### Synthetic route:

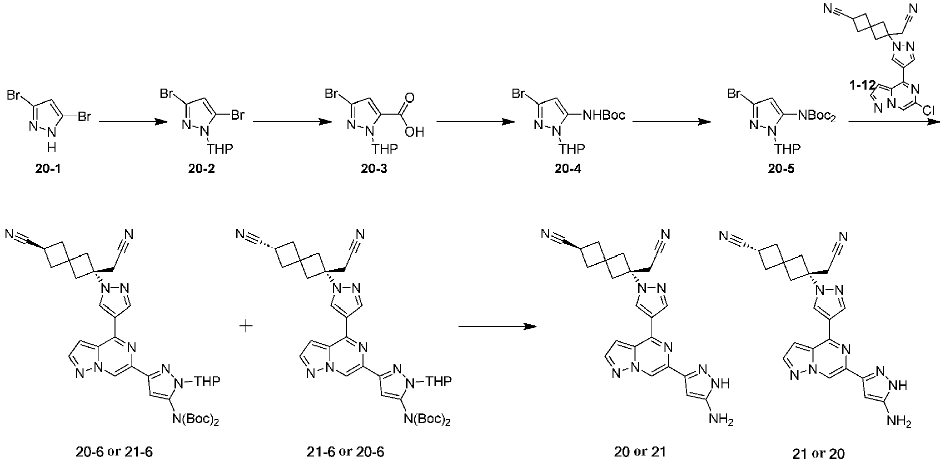

### Step 1: Synthesis of compound 20-2

Trifluoroacetic acid (50.48 mg, 442.73 µmol, 32.78 µL) was added to compound **20-1** (10 g, 44.27 mmol) and 3,4-dihydro-2H-pyran (14.90 g, 177.09 mmol, 16.19 mL), and the mixture was stirred at 100 °C for 16 h. The reaction was diluted with 60 mL of n-heptane, and the mixture was stirred at 20 °C for 0.5 h, filtered, and dried under vacuum to obtain compound **20-2.** ¹HNMR (CDCl₃) δ 6.36 (s, 1H), 5.43 (dd, *J* = 9.6, 2.8 Hz, 1H), 3.96-4.13 (m, 1H), 3.67 (td, *J* = 11.2, 2.8 Hz, 1H), 2.35-2.47 (m, 1H), 2.07-2.18 (m, 1H), 1.85-1.97 (m, 1H), 1.56-1.75 (m, 3H).

### Step 2: Synthesis of compound 20-3

Compound **20-2** (5.68 g, 18.32 mmol) was dissolved in tetrahydrofuran (100 mL), and n-butyllithium (2.5 M, 8.80 mL) was added dropwise at -65 °C. After the addition was completed, the mixture was stirred at this temperature for 0.5 h, then dry ice (20 g) was added at -65 °C and the obtained mixture was stirred for 30 min. The reaction was quenched by adding 1 M hydrochloric acid aqueous solution, adjusted to pH = 4, then diluted with 200 mL of water, and extracted with ethyl acetate (100 mL × 2), and the combined organic layer was washed with 100 mL of brine (100 mL × 1), dried over with sodium sulfate and purified by silica gel column (petroleum ether : ethyl acetate = 10 : 1 to 1 : 1) to obtain compound **20-3.** ¹HNMR (DMSO-d₆) δ 13.84 (brs, 1H), 6.99 (s, 1H), 6.16 (dd, *J=* 10.0, 2.4 Hz, 1H), 3.84-3.97 (m, 1H), 3.50-3.57 (m, 1H), 2.10-2.27 (m, 1H), 1.95 (m, 1H), 1.80-1.91 (m, 1H), 1.56-1.72 (m, 1H), 1.46-1.56 (m, 2H).

### Step 3: Synthesis of compound 20-4

Compound **20-3** (3.4 g, 12.36 mmol) was dissolved in tert-butanol (40 mL), and diphenyl azidophosphonate (6.80 g, 24.72 mmol, 5.36 mL) and triethylamine (3.19 g, 24.72 mmol, 4.31 mL) were added, the mixture was stirred at 45 °C for 0.5 h, and then heated to 100 °C and reacted for 16 h. The reaction mixture was diluted with 200 mL of water and extracted with ethyl acetate (100 mL × 2). The combined organic layer was washed with saturated sodium bicarbonate aqueous solution (100 mL × 2) and 100 mL of brine, dried over sodium sulfate, filtered, concentrated under reduced pressure, and purified by silica gel column (petroleum ether : ethyl acetate = 30 : 1 to 10 : 1) to obtain compound **20-4.** ¹HNMR (CDCl₃) δ 7.41 (brs, 1H), 6.39 (s, 1H), 5.37 (dd, *J* = 8.0, 2.8 Hz, 1H), 3.92 (dt, *J* = 11.2, 3.6 Hz, 1H), 3.69 (dt, *J* = 12.0, 6.0 Hz, 1H), 2.15-2.35 (m, 1H), 2.04-2.09 (m, 1H), 1.59-1.67 (m, 4H), 1.52 (s, 9H).

### Step 4: Synthesis of compound 20-5

Compound **20-4** (5.78 g, 9.18 mmol) was dissolved in dichloromethane (40 mL), and 4-dimethylaminopyridine (224.35 mg, 1.84 mmol), di-tert-butyl dicarbonate (4.01 g, 18.36 mmol, 4.22 mL) and triethylamine (2.79 g, 27.55 mmol, 3.83 mL) were added. The mixture was stirred at 20 °C for 16 h. The reaction mixture was diluted with 200 mL of water, and extracted with dichloromethane (100 mL × 2). The combined organic layer was washed with 100 mL of brine, dried over sodium sulfate, filtered, concentrated under reduced pressure, and purified by silica gel column (petroleum ether : ethyl acetate = 1 : 0 to 10 : 1) to obtain compound **20-5.** ¹HNMR (CDCl₃) δ 6.16 (s, 1H), 5.12 (dd, *J* = 9.6, 2.8 Hz, 1H), 3.93-4.01 (m, 1H), 3.55 (td, *J* = 11.2, 2.4 Hz, 1H), 2.29-2.43 (m, 1H), 2.05-2.15 (m, 1H), 1.76-1.91 (m, 1H), 1.53-1.70 (m, 3H), 1.41 (s, 18H).

### Step 5: Synthesis of compounds 20-6 and 21-6

Compound **20-5** (472.53 mg, 1.06 mmol), dipinacol borate (403.26 mg, 1.59 mmol), Xphos-Pd-G2 (83.30 mg, 105.87 µmol) and potassium acetate (259.75 mg, 2.65 mmol) were added to dioxane (10 mL), the mixture was stirred at 65 °C for 3 h under nitrogen atmosphere, and then cooled to 20 °C. Xphos-Pd-G2 (83.30 mg, 105.87 µmol), potassium phosphate (561.81 mg, 2.65 mmol), **1-12** (0.2 g, 529.34 µmol) and water (2 mL) were added, and the mixture was stirred at 90 °C for 16 h under nitrogen atmosphere. 80 mL of water was added to the reaction liquid, and the mixture was extracted with ethyl acetate (60 mL × 2). The combined organic layer was washed with brine (60 mL × 1), dried over sodium sulfate, and purified by silica gel column (petroleum ether : ethyl acetate = 5 : 1 to 1 : 1) and high performance liquid chromatography (column model: Phenomenex Luna C18 150 × 25 mm × 10 µm; mobile phase: [water (0.225% formic acid)-acetonitrile]; acetonitrile%: 56%-86%, 11 min) and then purified by SFC (column model: DAICEL CHIRALPAKIE(250 mm × 30 mm, 10 µm); mobile phase: [0.1% ammonia/ethanol]; [0.1% ammonia/ethanol]%: 50%-50%, 11.0 min; 77 min) to obtain compounds **20-6** and **21-6,** (analysis method: column model Chiralpak ID-3 100 × 4.6 mm I.D., 3 µm; mobile phase: A: n-heptane (0.05% diethylamine), B: ethanol (0.05% diethylamine); gradient: Phase B 40%; flow rate at 1 mL/min; column temperature 35 °C). The retention times were 4.703 min and 6.547 min respectively. Compound **20-6:** MS ESI calculated for C₃₇H₄₄N₁₀O₅ [M + H]⁺ 709, found 709. Compound **21-6:** MS ESI calculated for C₃₇H₄₄N₁₀O₅ [M + H]⁺ 709, found 709.

### Step 6: Synthesis of compound 20

Compound **20-6** (60 mg, 84.65 µmol) was dissolved in dichloromethane (1 mL), trifluoroacetic acid (1.54 g, 13.51 mmol, 1 mL) was added, and the mixture was stirred at 25 °C for 0.5 h. The reaction mixture was concentrated under reduced pressure and purified by high performance liquid chromatography (column model: Waters Xbridge 150 × 25 mm × 5 µm; mobile phase: [0.01% ammonium bicarbonate aqueous solution-acetonitrile]; acetonitrile%: 11%-41%, 10 min) to obtain compound **20.** ¹HNMR (DMSO-d₆) δ 12.15 (s, 1H), 9.08 (s, 1H), 8.99 (s, 1H), 8.62 (s, 1H), 8.24 (d, *J* = 2.4 Hz, 1.2H), 7.50 (s, 1H), 6.14 (s, 1H), 4.69 (s, 2H), 3.23-3.33 (m, 3H), 2.88-3.04 (m, 2H), 2.58-2.70 (m, 3H), 2.52-2.56 (m, 1H), 2.29-2.43 (m, 2H). MS ESI calculated for C₂₂H₂₀N₁₀ [M + H]⁺ 425, found 425.

### Step 7: Synthesis of compound 21

Compound **21-6** (70 mg, 98.76 µmol) was dissolved in dichloromethane (1 mL), trifluoroacetic acid (1.54g, 13.51mmol, 1mL) was added, and the mixture was stirred at 25 °C for 0.5 h. The reaction mixture was concentrated under reduced pressure and purified by high performance liquid chromatography (column: Waters Xbridge 150 × 25 mm × 5 µm; mobile phase: [0.01% ammonium bicarbonate aqueous solution-acetonitrile]; acetonitrile%: 11%-41%, 10 min) to obtain compound **21.** ¹HNMR (DMSO-d₆) δ: 12.10 (brs, 1H), 8.86-9.17 (m, 2H), 8.59 (s, 1H), 8.24 (d, *J* = 2.4 Hz, 2H), 7.50 (d, *J* = 1.6 Hz, 1H), 6.12 (s, 1H), 4.58-4.99 (m, 2H), 3.25-3.34 (m, 3H), 2.97 (td, *J* = 14.6, 1.6 Hz, 2H), 2.58-2.69 (m, 3H), 2.52-2.56 (m, 1H), 2.30-2.43 (m, 2H). MS ESI calculated for C₂₂H₂₀N₁₀ [M + H]⁺ 425, found 425.

### Bioactivity test

### Experimental example 1: Jak1, Jak2, Jak3, Tyk2 kinase activity test in vitro

### Experimental material

Recombinant human JAK1, JAK2, JAK3 and Tyk2 proteases, main instruments and reagents are all provided by the British company Eurofins

### Experimental method

JAK2, JAK3 and TYK2 dilution: 20 mM 3-(N-morpholine)propanesulfonic acid (MOPS), 1 mM EDTA, 0.01% Brij-35.5% glycerol, 0.1% β-mercaptoethanol, 1 mg/mL BSA; JAK1 dilution: 20 mM TRIS, 0.2 mM EDTA, 0.1% β-mercaptoethanol, 0.01% Brij-35.5% glycerol. All compounds were prepared as 100% DMSO solutions and brought to 50 times the final assay concentration. The test compound was subjected to 3-fold serial concentration dilution with a final concentration from 10 µM to 0.001 µM of a total of 9 concentrations. The content of DMSO in the detection reaction was 2%. A working stock solution of this compound was added to the assay well as the first component of the reaction, and then the remaining components were added according to the protocol detailed in the following determination.

### JAK1(h) enzyme reaction

JAK1(h) was incubated with 20 mM Tris/HCl pH 7.5, 0.2 mM EDTA, 500 µM MGEEPLYWSFPAKKK, 10 mM magnesium acetate and [γ-³³P]-ATP (activity and concentration were determined as required). The reaction was started by adding the Mg/ATP mixture, and after incubation at room temperature for 40 min, the reaction was terminated by adding 0.5% phosphoric acid. Then 10 µL of the reaction product was spotted on a P30 filter pad which was washed three times with 0.425% phosphoric acid and once with methanol within 4 min, dried, and subjected to scintillation counting.

### JAK2(h) enzyme reaction

JAK2(h) was incubated with 8 mM MOPS pH 7.0, 0.2 mM EDTA, 100 µM KTFCGTPEYLAPEVRREPRILSEEEQEMFRDFDYIADWC, 10 mM magnesium acetate, and [γ-³³P]-ATP (activity and concentration were determined as required). The reaction was started by adding the Mg/ATP mixture, and after incubation at room temperature for 40 min, the reaction was terminated by adding 0.5% phosphoric acid. Then 10 µL of the reaction product was spotted on a P30 filter pad which was washed three times with 0.425% phosphoric acid and once with methanol within 4 min, dried, and subjected to scintillation counting.

### JAK3(h) enzyme reaction

JAK3(h) was incubated with 8 mM MOPS pH 7.0, 0.2 mM EDTA, 500 µM GGEEEEYFELVKKKK, 10 mM magnesium acetate, and [γ-³³P]-ATP (activity and concentration were determined as required). The reaction was started by adding the Mg/ATP mixture, and after incubation at room temperature for 40 min, the reaction was terminated by adding 0.5% phosphoric acid. Then 10 µL of the reaction product was spotted on a P30 filter pad which was washed three times with 0.425% phosphoric acid and once with methanol within 4 min, dried, and subjected to scintillation counting.

### TYK2(h) enzyme reaction

TYK2(h) was incubated with 8 mM MOPS pH 7.0, 0.2 mM EDTA, 250 µM GGMEDIYFEFMGGKKK, 10 mM magnesium acetate, and [γ-³³P]-ATP (activity and concentration were determined as required). The reaction was started by adding the Mg/ATP mixture, and after incubation at room temperature for 40 min, the reaction was terminated by adding 0.5% phosphoric acid. Then 10 µL of the reaction product was spotted on a P30 filter pad which was washed three times with 0.425% phosphoric acid and once with methanol within 4 min, dried, and subjected to scintillation counting.

### Data analysis

IC₅₀ results were analyzed by XI,FITS (205 formula) of IDBS Company. Details are shown in Table 7.

**Table 7. Results of in vitro screening tests of the compound of the present disclosure**

| Compound No. | **JAK1** (IC₅₀, nM) | **JAK2** (IC₅₀, nM) | **JAK3** (IC₅₀, nM) | **TYK2** (IC₅₀, nM) |
|---|---|---|---|---|
| **1** | 10 | 4 | 189 | 0.9 |
| **2** | 123 | 21 | 480 | 2 |
| **3** | 1166 | 387 | 10000 | 177 |
| **4** | 606 | 82 | 10000 | 20 |
| **5** | 78 | 40 | 2256 | 9 |
| **6** | 3130 | Not detected | Not detected | 177 |
| **7** | 2910 | Not detected | Not detected | 228 |
| **8** | 13.7 | 1.13 | 22.8 | 6.43 |
| **9** | 128 | 5.16 | 209 | 2.81 |
| **10** | 281 | 33 | 10000 | 30 |
| **12** | 122 | 138 | 7743 | 6 |
| **13** | 233 | 103 | 1076 | 10 |
| **14** | 51 | 6 | 296 | 0.8 |
| **15** | 124 | 9 | > 1000 | 2 |
| **16** | 42 | 6 | 67 | 2 |
| **17** | 229 | 28 | > 1000 | 2 |
| **18** | 12 | 3 | 93 | 2 |
| **19** | 42 | 6 | 670 | 2 |
| **20** | 14 | 5 | 480 | 1 |
| **21** | 5 | 3 | 99 | 0.7 |

**Conclusion:** The compound of the present disclosure shows good selective inhibition on TYK2 and/or JAK1 and/or JAK2 in the in vitro activity test of 4 kinase subtypes JAK1, JAK2, JAK3 and TYK2.

### Experimental example 2: Pharmacokinetic (PK) test

The clear solution obtained after dissolving the test compound was administered to male mice (balb/c) via tail vein injection and gavage respectively. After administration of the test compound, blood samples were collected from mandibular veins for the intravenous injection group (1 mg/kg) at 0.083, 0.25, 0.5, 1, 2, 4, 8 and 24 h and for the gavage group (3 mg/kg) was at 0.25, 0.5, 1, 2, 4, 6, 8 and 24 h, respectively, and centrifuged to obtain plasma. The LC-MS/MS method was used to determine the blood drug concentration, and the WinNonlin^{™} Version 6.3 pharmacokinetic software was used to calculate the relevant pharmacokinetic parameters by the non-compartmental model linear logarithmic trapezoidal method. The test results are shown in Table 8:

**Table 8 PK test results of compound 1 in mice**

| PK parameter | Result |
|---|---|
| Cl (mL/Kg/min) | 15.6 |
| Vd (L/kg) | 1.59 |
| T_{1/2} (hr) | 2.39 |
| Cₘₐₓ (nM) | 3040 |
| AUC_{0-inf} (nM.hr) | 7673 |
| Bioavailability (%)^{a} | 88.7 |

| | |
|---|---|
| Note: T_{1/2}: half-life; Cₘₐₓ: Peak concentration; Cl: Clearance rate; Vd: Distribution volume; AUC_{0-inf}: the area under the plasma concentration-time curve from time zero extrapolated to infinity; Bioavailability: the extent to which the drug is bioavailable. | |

**Conclusion:** The compounds of the present disclosure have good oral bioavailability and higher exposure in mice, which are beneficial to produce good in vivo efficacy.

### Experimental example 3: In vivo drug efficacy test in mouse psoriasis model

**Purpose:** The purpose of this experiment is to evaluate the preventive and therapeutic effect of the test substance on IL-23 induced psoriasis-like skin lesions in mice through the psoriasis-like skin lesions model induced by intradermal injection of IL-23 into the mouse auricle.

Method: After the adaptation period of experimental animals, 50 mice were divided into 5 groups according to the Excel completely random grouping method, with 10 mice in each group, as follows: vehicle (0.5% MC & 0.5% Tween 80) control group, modeling group, compound 1 (10, 20, 50 mg/kg) group. Except for the vehicle control group, the animals in the other groups were intradermally injected with IL-23 (3 µg/10 µL/mouse/day, QD) in their right ears for 8 consecutive days (D1-D8). The animals in the control group were intradermally injected with an equal volume of physiological saline solution (10 µL/mouse/day, QD) in the right ear for 8 consecutive days (D1-D8). For the modeling group, the vehicle and test drug were administered orally at the same time, twice a day (Bid), with an interval of 6 h, for 8 consecutive days (D1-D8). During the dosing period, the animal body weight was monitored every two days. On D1, D3, D5, D7 (all before IL-23 injection), and D9, the thickness of the mouse right ear was measured and the auricle appearance was observed and scored. At the end of the experiment (Day 10), the ear on the side where the model was created was taken, and the ear was punched along the edge of the auricle with an 8 mm punch to obtain a piece of ear which was weighed. The piece of ear from the right ear was fixed with formalin and then subjected to histopathological examination (H&E) staining.

The experimental results are shown in Table 9-12.

**Table 9. Effects of oral gavage administration of the test substance twice a day for 8 consecutive days on the body weight of IL-23 induced mouse auricular epidermal dysplasia model mice**

| Group and dosage | Weight (g)/day | | | | |
|---|---|---|---|---|---|
| | 1 | 3 | 5 | 7 | 9 |
| Vehicle control group | 19.78 ± 0.60 | 1988 ± 0.89 | 20.36 ± 0.64 | 20.06 ± 0.78 | 20.23 ± 0.57 |
| Modeling group | 1976 ± 0.56 | 19.76 ± 0.65 | 1973 ± 0.70 | 19.51 ± 0.87 | 19.90 ± 0.72 |
| Compound 1 10 mg/kg | 19.77 ± 0.59 | 1969 ± 0.57 | 20.02 ± 0.77 | 1969 ± 0.63 | 20.04 ± 0.72 |
| Compound 1 20 mg/kg | 19.77 ± 0.54 | 1985 ± 0.78 | 20.13 ± 0.78 | 19.80 ± 0.92 | 20.27 ± 0.90 |
| Compound 1 50 mg/kg | 19.78 ± 0.53 | 1985 ± 0.68 | 20.41 ± 0.70 | 20.15 ± 0.56 | 20.28 ± 0.76 |

**Table 10. Effects of oral gavage administration of the test substance twice a day for 8 consecutive days on the ear thickness of IL-23 induced mouse auricular epidermal dysplasia model mice**

| Group and dosage | Ear thickness (mm)/day | | | | |
|---|---|---|---|---|---|
| | 1 | 3 | 5 | 7 | 9 |
| Vehicle control group | 0.193 ± 0.005 | 0.218 ± 0.009 | 0.216 ± 0.012 | 0.230 ± 0.017 | 0.227 ± 0.015 |
| Modeling group | 0.188 ± 0.007 | 0.303 ± 0.029^{##} | 0.338 ± 0.027^{##} | 0.474 ± 0.068^{##} | 0.520 ± 0.078^{##} |
| Compound 1 10 mg/kg | 0.189 ± 0.006 | 0.287 ± 0.018 | 0.311 ± 0.013* | 0.373 ± 0.030** | 0.430 ± 0.048* |
| Compound 1 20 mg/kg | 0.190 ± 0.009 | 0.289 ± 0.028 | 0.306 ± 0.018** | 0.350 ± 0.025** | 0.381 ± 0.032** |
| Compound 1 50 mg/kg | 0.193 ± 0.006 | 0.279 ± 0.024* | 0.289 ± 0.025** | 0.338 ± 0.023** | 0.352 ± 0.032** |

| | | | | | |
|---|---|---|---|---|---|
| ^{##}*P* < 0.01 vs. Control **P* < 0.05, ***P* < 0.01 vs. Model | | | | | |

**Table 11. Effects of the test substance on the ear weight of IL-23 induced mouse auricular epidermal dysplasia model mice**

| Group and dosage | Ear weight (mg) |
|---|---|
| Vehicle control group | 12.94 ± 0.75 |
| Modeling group | 22.80 ± 3.01## |
| Compound 1 10 mg/kg | 19.16 ± 1.56** |
| Compound 1 20 mg/kg | 18.76 ± 1.62** |
| Compound 1 50 mg/kg | 17.63 ± 1.47** |

| | |
|---|---|
| ^{##}*P* < 0.01 vs. Control ***P* < 0.01 vs. Model | |

**Table 12. Effects of oral gavage administration of the test substance twice a day for 8 consecutive days on the total score of IL-23 induced mouse auricular epidermal dysplasia**

| Group and dosage | Ear comprehensive score/day | | | | |
|---|---|---|---|---|---|
| | 1 | 3 | 5 | 7 | 9 |
| Vehicle control group | 0.00 ± 0.00 | 0.00 ± 0.00 | 0.00 ± 0.00 | 0.00 ± 0.00 | 0.00 ± 0.00 |
| Modeling group | 0.00 ± 0.00 | 0.20 ± 0.42 | 1.70 ± 0.95## | 5.60 ± 1.43## | 6.40 ± 1.58## |
| Compound 1 10 mg/kg | 0.00 ± 0.00 | 0.00 ± 0.00 | 0.60 ± 0.52** | 1.70 ± 1.06** | 2.60 ± 1.43** |
| Compound 1 20 mg/kg | 0.00 ± 0.00 | 0.00 ± 0.00 | 0.60 ± 0.70** | 1.40 ± 0.52** | 1.50 ± 0.71** |
| Compound 1 50 mg/kg | 0.00 ± 0.00 | 0.00 ± 0.00 | 0.10 ± 0.32** | 1.40 ± 0.70** | 1.00 ± 0.47** |

| | | | | | |
|---|---|---|---|---|---|
| *^{##}P* < 0.01 vs. Control ***P* < 0.01 vs. Model Note: IL-23: Interleukin-23. | | | | | |

### Conclusion:

(i). In the IL-23 induced auricular epidermal dysplasia model of C57BL/6 mice, oral gavage administration of the compound of the present disclosure (10, 20, 50 mg/kg) twice a day for 8 consecutive days had no significant effect on the body weight of animals in the IL-23 induced auricle epidermal dysplasia model of C57BL/6 mice.
(ii). Effect on the ear thickness of the modeling side of the animal: Compound 1 at 10 and 20 mg/kg can significantly reduce the ear thickness of the modeling side of mice starting from day 5. The compound of the present disclosure can significantly reduce the ear thickness of the modeling side of animals starting from D3 at a dose of 50 mg/kg, and 3 dose groups show a good dose-effect relationship. It shows that each group of the compound of the present disclosure (10, 20, 50 mg/kg) can inhibit the increase in the ear thickness of the modeling side of mice to varying degrees.
(iii). Effect on the ear weight of the modeling side of the animal: Oral gavage administration of the compound of the present disclosure (10, 20, 50 mg/kg) twice a day for 8 consecutive days can significantly reduce the ear weight of the modeling side of the animal, and 3 dose groups show a good dose-effect relationship in terms of the effect on the ear weight of the modeling side of the animal.
(iv). Effect on the total score of the modeling side ear of the animal: Oral gavage administration of the compound of the present disclosure (10, 20, 50 mg/kg) twice a day orally for 8 consecutive days can significantly reduce the total score of the affected ear of mice from D5 to D9, showing good efficacy and a good dose-effect relationship.

## Claims

1. A compound represented by formula (IV) or a pharmaceutically acceptable salt thereof, 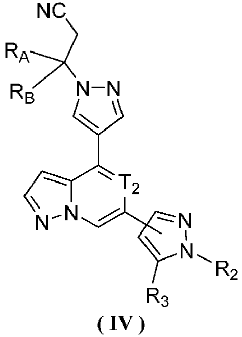 wherein
R_{A} is selected from H, and R_{B} is selected from 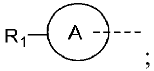 alternatively, R_{A} and R_{B} and the carbon atom to which they are both attached form 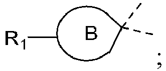
ring A is selected from C₅₋₈ cycloalkyl;
ring B is selected from C₅₋₁₀ cycloalkyl or 5- to 10-membered heterocycloalkyl;
R₁ is selected from H, C₁₋₃ alkyl-SO₂-, CN and -CH₂CN, and the C₁₋₃ alkyl is optionally substituted with 1, 2 or 3 halogens;
R₂ is selected from H and C₁₋₃ alkyl, and the C₁₋₃ alkyl is optionally substituted with 1, 2 or 3 halogens;
R₃ is selected from H, NH₂, halogen, OH, CN and C₁₋₃ alkyl, and the C₁₋₃ alkyl is optionally substituted with 1, 2 or 3 halogens;
T₂ is selected from CH and N.

2. The compound represented by formula (IV) or the pharmaceutically acceptable salt thereof of claim 1, wherein, ring A is selected from

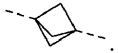

3. The compound represented by formula (IV) or the pharmaceutically acceptable salt thereof of claim 1, wherein, ring B is selected from 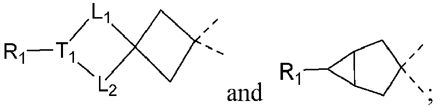
Li is selected from -(CH₂)ₘ-, and the -(CH₂)ₘ- is optionally substituted with 1, 2 or 3 halogens;
L₂ is selected from -(CH₂)ₙ-, and the -(CH₂)ₙ- is optionally substituted with 1, 2 or 3 halogens;
m and n are each independently selected from 1, 2 and 3;
T₁ is selected from CH and N.

4. The compound represented by formula (IV) or the pharmaceutically acceptable salt thereof of any one of claims 1-3, wherein, the compound is selected from a compound represented by formulas (IV-1) and (IV-2), 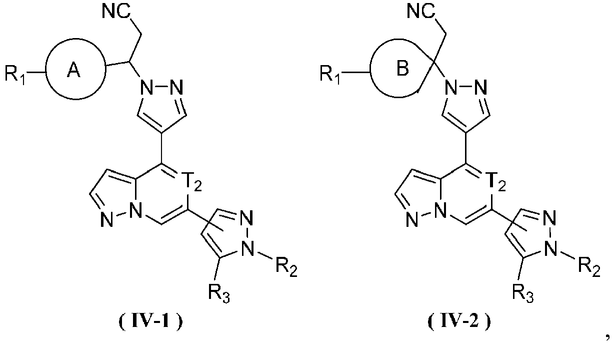 wherein, Ri, R₂, R₃, T₂, ring A and ring B are as defined in any one of claims 1-3.

5. The compound represented by formula (IV) or the pharmaceutically acceptable salt thereof of claim 1, wherein, the compound is selected from a compound represented by formula (I), 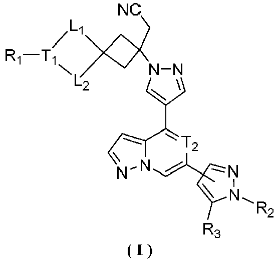 wherein
Ri is selected from H, C₁₋₃ alkyl-SO₂-, CN and -CH₂CN, and the C₁₋₃ alkyl is optionally substituted with 1, 2 or 3 halogens;
R₂ is selected from H and C₁₋₃ alkyl, and the C₁₋₃ alkyl is optionally substituted with 1, 2 or 3 halogens;
R₃ is selected from H, NH₂, halogen, OH, CN and C₁₋₃ alkyl, and the C₁₋₃ alkyl is optionally substituted with 1, 2 or 3 halogens;
Li is selected from -(CH₂)ₘ-, and the -(CH₂)ₘ- is optionally substituted with 1, 2 or 3 halogens;
L₂ is selected from -(CH₂)ₙ-, and the -(CH₂)ₙ- is optionally substituted with 1, 2 or 3 halogens;
m and n are each independently selected from 1, 2 and 3;
T₁ is selected from CH and N;
T₂ is selected from CH and N.

6. The compound or the pharmaceutically acceptable salt thereof of claim 5, wherein, Ri is selected from H, C₁₋₃ alkyl-SO₂-, CN and -CH₂CN, and the C₁₋₃ alkyl is optionally substituted with 1, 2 or 3 F.

7. The compound or the pharmaceutically acceptable salt thereof of claim 6, wherein, Ri is selected from H, CF₃SO₂-, CN and -CH₂CN.

8. The compound or the pharmaceutically acceptable salt thereof of claim 5, wherein, R₂ is selected from H and CH₃.

9. The compound or the pharmaceutically acceptable salt thereof of claim 5, wherein, R₃ is selected from H and NH₂.

10. The compound or the pharmaceutically acceptable salt thereof of claim 5, wherein, L₁ is selected from -CH₂- and -CH₂CH₂-, and the -CH₂- and - CH₂CH₂- are optionally substituted with 1, 2 or 3 F.

11. The compound or the pharmaceutically acceptable salt thereof of claim 10, wherein, Li is selected from -CH₂- and -CH₂CH₂-.

12. The compound or the pharmaceutically acceptable salt thereof of claim 5, wherein, L₂ is selected from -CH₂- and -CH₂CH₂-, and the -CH₂- and -CH₂CH₂- are optionally substituted with 1, 2 or 3 F.

13. The compound or the pharmaceutically acceptable salt thereof of claim 12, wherein, L₂ is selected from -CH₂- and -CH₂CH₂-.

14. The compound or the pharmaceutically acceptable salt thereof of claim 5, wherein, the moiety 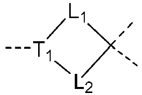 is selected from acridinyl, cyclobutyl, tetrahydropyrrolyl, piperidinyl and cyclohexyl.

15. The compound or the pharmaceutically acceptable salt thereof of claim 14, wherein, the moiety 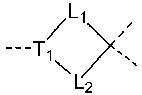 is selected from 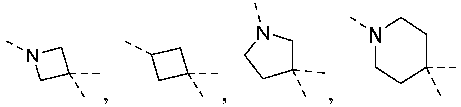 and

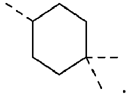

16. The compound or the pharmaceutically acceptable salt thereof of any one of claims 5-15, wherein, the compound is selected from a compound represented by formulas (I-1) and (I-2): 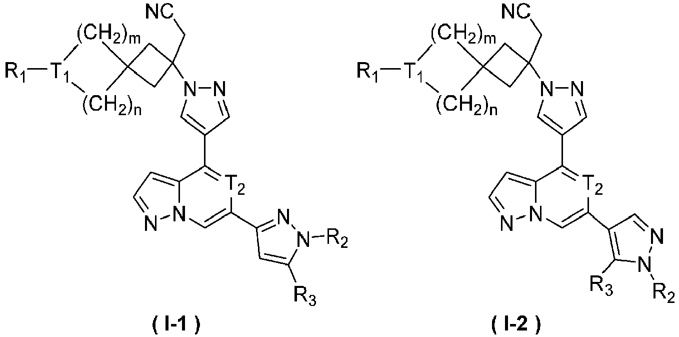 wherein Ri, R₂, R₃, T₁, T₂, m and n are as defined in any one of claims 5-15.

17. A compound or a pharmaceutically acceptable salt thereof, which is selected from:

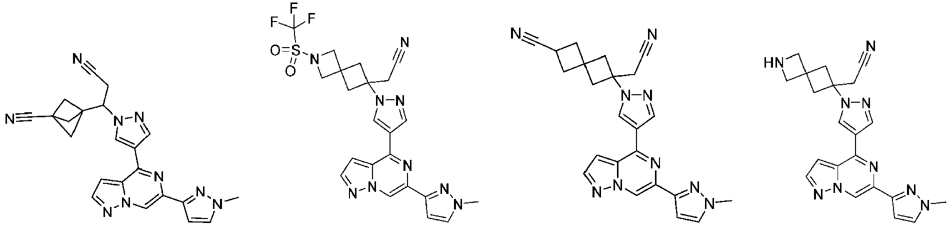

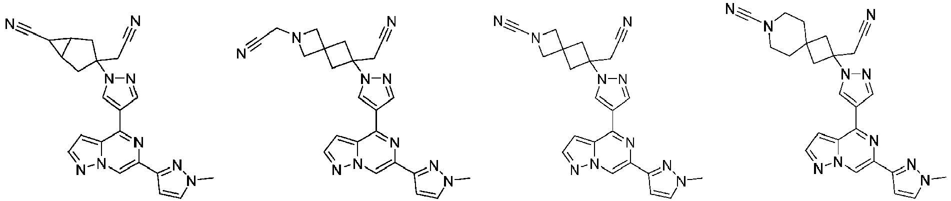

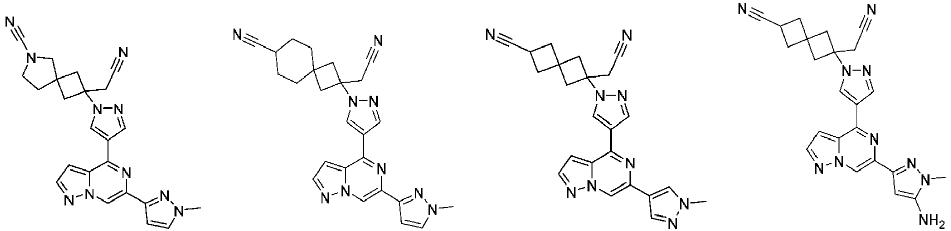

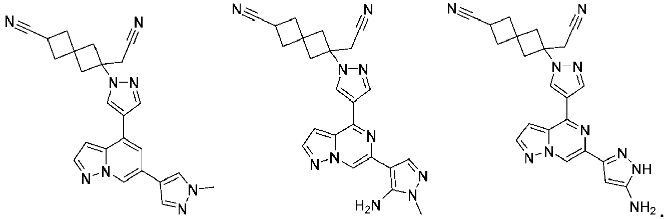

18. The compound or a pharmaceutically acceptable salt thereof of claim 17, wherein the compound is selected from:

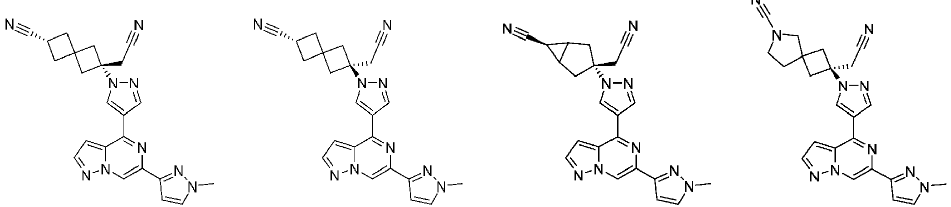

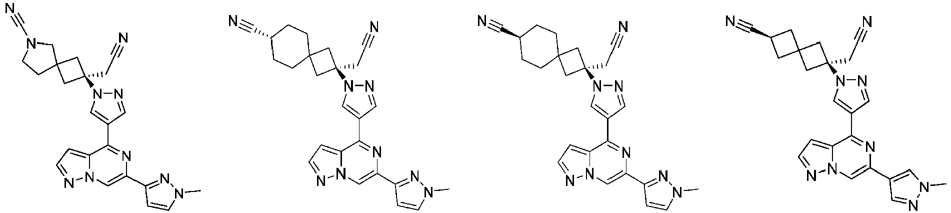

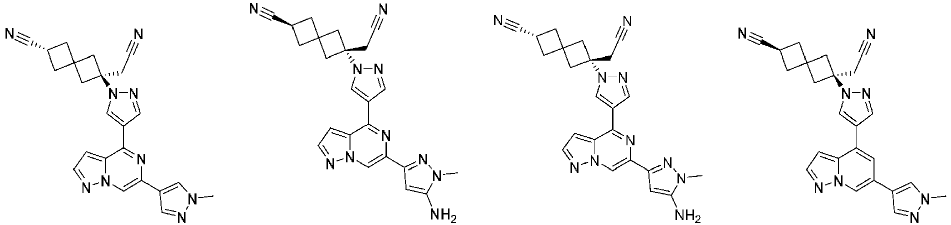

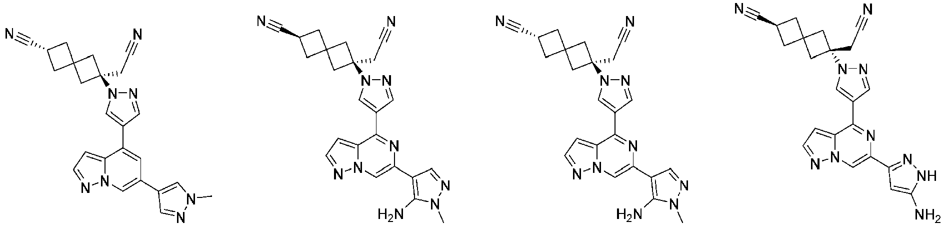

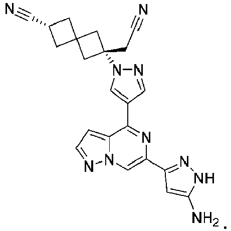

19. Use of the compound or the pharmaceutically acceptable salt thereof of any one of claims 1 to 18 in the preparation of a drug for treating psoriasis and/or inflammatory bowel disease.
